# FASCICULE DE BREVET EUROPEEN

(11) **EP 3 129 014 B1**
(45) Date de publication et mention de la délivrance du brevet: **12.05.2021**
(21) Numéro de dépôt: 15732022.7
(22) Date de dépôt: 07.04.2015
(51) Int. Cl.: A61K 31/16, A61K 31/19, A61K 31/22, A61K 31/235, A61K 31/265, A61P 35/00, C07C 391/00, C07F 11/00

(54) **NOUVEAUX COMPOSES ORGANO-SELENIES, PROCÉDÉ DE FABRICATION ET APPLICATIONS EN PHARMACIE COMME AGENTS ANTI-TUMORAUX**
NEUARTIGE ORGANOSELENVERBINDUNGEN, VERFAHREN ZUR HERSTELLUNG DAVON UND PHARMAZEUTISCHE VERWENDUNGEN ALS ANTITUMORMITTEL
NOVEL ORGANOSELENIUM COMPOUNDS, METHOD FOR PRODUCING SAME, AND PHARMACEUTICAL USES THEREOF AS ANTITUMOUR AGENTS

(30) Priorité: 08.04.2014 FR 1453136
(43) Date de publication de la demande: 15.02.2017
(73) Titulaire: Tetrahedron, 75001 Paris (FR)
(72) Inventeur: YADAN, Jean-Claude, 75001 Paris (FR); ERDELMEIER, Irène, 75001 Paris (FR); MOUTET, Marc, 75001 Paris (FR); LEBEL, Rémi, 75001 Paris (FR)
(74) Mandataire: Pereira da Cruz, Joao
(86) Numéro de dépôt international: PCT/FR2015/050886
(87) Numéro de publication internationale: WO 2015/155453

(56) Documents cités:
- SAAD SHAABAN ET AL: "Novel peptidomimetic compounds containing redox active chalcogens and quinones as potential anticancer agents", EUROPEAN JOURNAL OF MEDICINAL CHEMISTRY, vol. 58, 1 décembre 2012 (2012-12-01), pages 192-205, XP055153094, ISSN: 0223-5234, DOI: 10.1016/j.ejmech.2012.09.033
- MUHAMMAD ABBAS ET AL: "One pot synthesis of selenocysteine containing peptoid libraries by Ugi multicomponent reactions in water", CHEMICAL COMMUNICATIONS, no. 5, 1 janvier 2006 (2006-01-01), page 541, XP055153106, ISSN: 1359-7345, DOI: 10.1039/b514597j

## Description

La présente invention a pour objet :
- des nouveaux composés organo-séléniés;
- leurs procédés de préparation ;
- leur utilisation en tant que principe actif en pharmacie ;
- ainsi que des compositions pharmaceutiques les renfermant, notamment comme nouveaux agents anti-tumoraux.

### Etat de l'Art

Cette invention se rapporte à de nouveaux composés organo-séléniés, leur procédé de préparation, leur utilisation en tant que principe actif pharmaceutique, ainsi que des compositions pharmaceutiques les renfermant, notamment comme nouveaux agents anti-tumoraux. Plus particulièrement, cette invention concerne la préparation de composés organo-séléniés comprenant l'acide 2-hydroxy-3-méthylsélénopropanoïque, ses sels ainsi que les esters et les amides dérivés de l'acide 2-hydroxy-3-méthylsélénopropanoïque, doués d'activité pharmaceutique et notamment d'activité anti-tumorale ou anti-cancer, seuls ou en association avec d'autres agents pharmaceutiques et notamment des agents anti-tumoraux.

Le cancer reste l'une des principales causes de décès dans les pays industrialisés. En dépit des nombreux progrès dans le développement de nouvelles thérapies anticancéreuses, un traitement curatif reste encore un besoin réel pour la plupart des tumeurs solides. Le potentiel anti-tumoral du « sélénium » a été identifié, à la fin des années 60, suite à des études mettant en évidence des taux de mortalité due au cancer plus faibles dans certaines régions dont le sol était riche en sélénium inorganique. Depuis une vingtaine d'années, de nombreux travaux ont mis en évidence, dans le cadre d'expérimentations animales, l'activité anti-cancéreuse du « sélénium » dans un certain nombre d'organes [Papp LV et al., 2007, Antioxid Redox Signal, 9:775]. Sur un total de huit essais cliniques d'évaluation de l'effet du « sélénium » sur l'incidence de cancers, sept ont abouti à des résultats positifs [Whanger PD, 2004, Br. J. Nutr., 91(1):11]. Ceci confirme les nombreuses études réalisées chez l'animal.

L'activité anti-tumorale de la méthylsélénocystéine (MeSeCys) a été mise en évidence par Ip et coll. au début des années 2000 [Medina D et al., 2001, Nutrition and Cancer, 40(1): 12]. Pour être efficace, la MeSeCys doit être «activée» en méthylsélénol par la β-lyase [Ip C. et al., 2002 Cancer & Metast. Rev., 21(3-4):281]. Les cellules tumorales humaines de cancer du sein ou de la prostate présentent un faible niveau d'activité β-lyase, ce qui entraine une faible sensibilité de ces cellules tumorales à des concentrations physiologiquement acceptables de MeSeCys. Par contre des doses journalières de 3mg équivalent Sélénium/kg (eqSe/kg) de MeSeCys administrées pendant 18 à 26 semaines à des souris transgéniques portant un adénome prostatique retardent la progression des lésions tumorales, augmentent l'apoptose et réduisent la prolifération des cellules tumorales [Wang L et al., 2009, Cancer Prev. Res., 2:484].

La MeSeCys induit l'apoptose des cellules de carcinomes par activation de la voie des caspases [Suzuki M. et al., 2010, Cancer Chemother. Pharmacol., 66(3):475]. La MeSeCys inhibe la croissance de cellules mammaires, et induit l'apoptose selon un mécanisme caspase-dépendent et implique la libération du cytochrome C mitochondrial ainsi que la fragmentation de l'ADN nucléosomal [Hu H et al., 2005, Carcinogenesis, 26:1374]. Ce même composé inhibe la progression du cancer de la prostate dans un modèle murin et augmente la survie des souris [Wang L et al., 2009, Cancer Prev. Res., 2:484].

La MeSeCys, n'est pas facilement synthétisable et nécessite, de plus, une activation métabolique pour être efficace.

L'acide méthylséléninique (MSA) a été utilisé pour sa capacité à générer du méthylsélénol CH₃SeH après réduction par des réducteurs biologiques tel que le glutathion intracellulaire. Ceci lui confère une activité anti-cancéreuse dont il a été démontré qu'elle est supérieure à celle de la MeSeCys [Ip C et al., 2000, Cancer Res., 60:2882] ainsi qu'à celle de la sélénométhionine [Li GX et al., 2008, Carcinogenesis, 29:1005]. Le MSA est capable de moduler plusieurs bio-marqueurs spécifiques ce qui aboutit à une diminution de la prolifération cellulaire et une activation de l'apoptose [Dong Y. et al., 2002, Anticancer Res., 22:27]. Il induit un stress au niveau du réticulum endoplasmique par oxydation des thiols de protéines qui conduit à un repliement anormal de ces dernières. Si le système de réparation est « débordé », alors la cellule s'engage dans un processus d'apoptose [Wu Y et al., 2005, Cancer Res., 65(19):9073]. Par ailleurs, le MSA diminue, de façon dose-dépendante, le niveau du facteur inductible d'hypoxie HIF-1α dans des cellules d'adénome prostatique hormono-indépendantes [Sinha R et al., 2008, Cancer Prev. Res., 1, 7, Suppl., abstract n°B117]. Le MSA réduit les métastases spontanées de cellules cancéreuses de poumon chez la souris [Yan L et al., 2011, Int. J. Cancer, 131:1260]. Le MSA, bien que très actif, est difficilement utilisable en tant que principe actif de médicament pour des raisons évidentes liées à sa formulation et à sa stabilité.

Enfin, d'autres composés organo-séléniés ont été décrits comme ayant des propriétés anti-cancéreuses tels que le S,S'-1,4-phenylènebis(1,2-éthanediyl)-isoséléno-urée [SubbaRao VM et al., 2008, Mol. Cancer Ther., 7(5):1297], le 1,4-phenylènebis(méthylène)-sélénocyanate [Chen KM, 2007, Cancer Res., 67(21):10475] ainsi qu'une série d'isosélénocyanates [Sharma A, 2009, Clin. Cancer Res., DOI 10.1158/1078-0432]. Pour les mêmes raisons que le MSA, ces composés sont très difficilement utilisables comme médicaments.

Le document SHAABAN, dans European Journal of Médicinal Chemistry, 58,(2012), P192-205, décrit divers composés contenant des chalcogènes actifs redox et des quinones comme agents anti-cancer potentiels. A la page 195, SHAABAN décrit des composés dans lesquels R1 pourrait comprendre un noyau aromatique (pouvant être aryle) et R2= C(=O)R4 et R4O(C=O) qui ont une similitude avec R4 = aryle des composés de la formule (I) de la présente invention décrite ci-après.

Cependant, dans les composés de formule (I) de la présente invention décrite ci-après, un noyau aromatique n'est prévu qu'en second, c'est-à-dire sous forme alkyl-aryle avec alkyle attaché au Sélénium.

De ce fait, les composés de formule (I) sont nouveaux par rapport aux composés 4 à 18, page 195 de SHAABAN,.

Par ailleurs, pour les composés 4, 5, 7, 10 et 11 de SHAABAN qui ont un groupe NH-tertbutyle, correspondant à X de la formule (I) de la présente invention, les composés de formule (I) de la présente invention sont en outre différents car dans ce cas R2 est ≠ de C(=O) CH3 et aussi R2 est seulement alkyle ou aryle.

Egalement, les composés de formule (I) n'ont pas de groupe redox type quinone.

Par ailleurs, le document ABBAS , publié dans www.rsc.org/chemcomm, Royal Society of Chemistry, 2006, P541-543, décrit divers composés n'ayant pas d'activité anti-cancer parmi lesquels un composé 7t à la page 542 qui a une substitution méthyle sur Se correspondant à R1 de la formule (I) de la présente invention décrite ci-après.

Cependant, le substituant en haut de la formule de 7t correspond au substituant X de la formule (I) de la présente invention décrite ci-après mais la définition donnée prévoit pour X =**alpha**-aminoacide un radical de type - NHCH(Y)COOH (**alpha**-aminoacide) qui exclut le radical -NH-(CH2)5-COOBn (**epsilon**-aminoacide) du composé 7t de ABBAS.

En outre le substituant en bas de la formule de 7t= -O-C(=O)-CH2-NH-C(=O)-O-tertButyl correspond au substituant -O-R2 lorsque R2= R4(C=O) mais R4 = alkyle ou aryle dans le cadre de la présente invention.

Et, la définition interprétée correctement de « alkyle » de la présente invention décrite ci-après prévoit une substitution notamment par un groupe amine et/ou acide ou ester mais pas une fonction NH-C(=O)-O-R qui permettrait de correspondre à 7t décrit dans ABBAS.

Ainsi, la définition de alkyle dans la formule (I) de la présente invention ne couvre pas le composé 7t de ABBAS.

Pour les raisons exposées ci-dessus, les produits de l'art antérieur ayant des propriétés anti-cancéreuses ne sont pas satisfaisants en tant qu'agents anti-tumoraux.

### BUTS DE L'INVENTION

L'un des buts de la présente invention est de concevoir de nouveaux composés organo-séléniés facilement synthétisables et formulables.

Un autre des buts de la présente invention est de préparer ces nouveaux composés organo-séléniés pouvant être synthétisés en grande quantité par des procédés utilisables à l'échelle industrielle.

Un autre des buts de la présente invention est de préparer de nouveaux composés organo-séléniés facilement formulables en tant qu'agents pharmaceutiquement actifs et notamment en tant qu'agents anti-tumoraux ou anti-cancer.

Un autre des buts de la présente invention est de préparer de nouvelles compositions pharmaceutiques contenant au moins l'un de ces nouveaux composés organo-séléniés, seul ou en association avec un autre agent anti-tumoral ou anti-cancer.

Ces buts sont atteints grâce à la présente invention qui repose sur la conception et la préparation de nouveaux composés organo-séléniés et leurs esters et amides ainsi que leurs applications en pharmacie notamment en tant qu'agents anti-tumoraux. Ceci a été exemplifié dans la présente invention.

### Description de l'invention

La présente invention a donc pour but :
1) de résoudre le nouveau problème technique consistant en la fourniture de nouveaux composés organo-séléniés et leurs dérivés esters et amides constituant ainsi des principes actifs de compositions pharmaceutiques ;
2) de résoudre ce nouveau problème technique selon une solution qui englobe un procédé de préparation utilisable à l'échelle industrielle de ces nouveaux composés organo-séléniés et de leurs dérivés esters et amides ;
3) de proposer des compositions pharmaceutiques de ces composés organo-séléniés répondant à la formule générale (I) seuls ou en association avec d'autres agents pharmaceutiques, notamment d'autres agents anti-tumoraux.

Les problèmes techniques énoncés ci-dessus sont résolus pour la première fois d'une manière simultanée par la présente invention, de façon très aisée et économique, le procédé de préparation desdits nouveaux composés étant très simple à mettre en œuvre tout en fournissant de bons rendements.

Selon son premier aspect, la présente invention a pour objet de nouveaux agents organo-séléniés répondant à la formule générale (I) suivante : 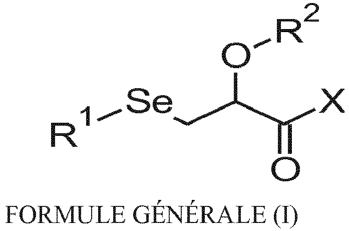 dans laquelle
**R¹** = alkyle constitué par- un radical carboné saturé de 1 à 26 atomes de carbone linéaire, ramifié ou cyclique;
**R²** = **H, R⁴**C(=O), **R⁴**OC(=O), α-amino-acyle constitué par -C(=O)CH(Y)NH2, Y correspondant à la chaine latérale de l'un des aminoacides protéogèniques, CH₃SeCH₂CH₂CH(NH₂)C(=O), CH₃SeCH₂CH₂CH(OH)C(=O),
**X** = OH, O**R**³, NH₂, N**R**⁴**R**⁵, α-amino-acide constitué par -NHCH(Y)COOH, Y correspondant à la chaine latérale de l'un des aminoacides protéogèniques, CH₃SeCH₂CH₂CH(COOH)NH-, CH₃SeCH₂CH₂CH(COOH)O-;
**R³** = alkyle constitué par un radical carboné saturé de 1 à 26 atomes de carbone linéaire, ramifié ou cyclique;
**R⁴** = alkyle constitué par un radical carboné saturé de 1 à 26 atomes de carbone linéaire, ramifié ou cyclique, aryle; pyridinyle; ou imidazole;
**R**⁵ = H, alkyle constitué par un radical carboné saturé de 1 à 26 atomes de carbone linéaire, ramifié ou cyclique, aryle ; pyridinyle; ou imidazole ;
**R**⁴ et **R**⁵ pouvant former ensemble un radical cyclo-alkyle à 5 ou 6 chainons pouvant comporter un hétéroatome;
étant entendu que lorsque **X** = NHterbutyle alors **R²** ≠ C(=O)CH₃
ainsi que- tous les stéréoisomères, diastéréoisomères et énantiomères notamment au niveau de l'atome de carbone portant le groupement **OR²**, ainsi qu'au niveau des radicaux **R¹** à **R⁵**, ainsi que les oligomères constitués par l'enchaînement de 2 à 15 monomères obtenus entre deux ou plusieurs molécules de dérivés de formule (I) par réaction d'estérification entre les fonctions alcool et acide carboxylique présentes le cas échéant, pris isolément ou en mélange ;

Elle englobe également tous les sels d'acides ou de bases pharmaceutiquement acceptables desdits composés de formule générale (I), ainsi que les sels de sodium, de calcium, de zinc et de magnésium.

Parmi les composés de formule générale (I), l'invention a notamment pour objet :
- ceux caractérisés en ce que **R¹** représente un groupe méthyle, éthyle ;
- ceux caractérisés en ce que **R²** est choisi dans le groupe constitué par H, α-amino-acyle constitué par-C(=O)CH(Y)NH2, Y correspondant à la chaine latérale de l'un des aminoacides protéogèniques, CH₃SeCH₂CH₂CH(OH)C(=O), R₄(C=O), R₄O(C=O);
- ceux caractérisés en ce que **X** est choisi dans le groupe OH, α-amino-acide constitué par-NHCH(Y)COOH, Y correspondant à la chaine latérale de l'un des aminoacides protéogèniques, CH3SeCH2CH2CH(COOH)NH-, CH₃SeCH₂CH₂CH(COOH)O-;
- ceux caractérisés en ce que R¹ représente un groupe méthyle, éthyle; R² représente R4(C=O), R4O(C=O) et X représente OH ou OR3.
- ceux préparés dans la partie expérimentale, notamment les composés 4, 10, 38, 46 et 48.

Parmi les acides pharmaceutiquement acceptables, on peut citer, à titre non limitatif, les acides minéraux tels que les acides chlorhydrique, bromhydrique, iodhydrique, sulfurique, tartrique, phosphorique ou avec les acides organiques tels que les acides formique, acétique, trifluoroacétique, propionique, benzoïque, maléique, fumarique, succinique, citrique, oxalique, glyoxylique, aspartique, alcanesulfoniques tels que les acides méthane-sulfonique, trifluorométhane-sulfonique, éthane-sulfonique, aryl-sulfoniques tels que les acides benzène- et paratoluène-sulfonique.

Parmi les bases pharmaceutiquement acceptables, on peut citer, à titre non limitatif, les bases minérales telles que les hydroxydes de sodium, de lithium, de calcium, de potassium, de magnésium, d'ammonium ou de zinc, les carbonates de métaux alcalins ou alcalino-terreux tels que les carbonates et bicarbonates de sodium, de lithium, de calcium, de potassium, de magnésium, d'ammonium ou de zinc ou des bases organiques comme la méthylamine, la propylamine, la triméthylamine, la diéthylamine, la triéthylamine, la N,N-diméthyléthanolamine, le tris(hydroxy-méthyl)aminométhane, l'éthanolamine, la pyridine, la picoline, la dicyclohexylamine, la morpholine, la proceïne, la lysine, l'arginine, l'histidine, la N-méthylglucamine ou encore les sels de phosphonium tels que les sels d'alkyl-phosphonium, les sels d'aryl-phosphonium, les sels d'alkyl-aryl-phosphonium, les alkènyl-aryl-phosphonium ou les sels d'ammonium quaternaires tels que les sels de tétra-n-butyl-ammonium.

Dans la formule (I) ci-dessus :
- par alkyle, il est entendu un radical carboné saturé de 1 à 26 atomes de carbone linéaire, ramifié ou cyclique.
- par aryle, il est entendu un radical phényle pouvant être substitué par un ou plusieurs atomes de fluor,
- par α-aminoacyle, il est entendu un radical de type -C(=O)CH(Y)NH2, Y correspondant à la chaine latérale de l'un des aminoacides protéogèniques c'est-à-dire des aminoacides constituant les protéines.
- par α-aminoacide, il est entendu un radical de type -NHCH(Y)COOH, Y correspondant à la chaine latérale de l'un des aminoacides protéogèniques c'est-à-dire des aminoacides constituant les protéines.
- par oligomère, il est entendu tout composé constitué par l'enchaînement de 2 à 15 monomères tels que décrits dans l'invention reliés entre eux par l'intermédiaire d'une liaison de type ester.
- par polymère, il est entendu tout composé constitué par l'enchaînement de plus de 15 monomères tels que décrits dans l'invention reliés entre eux par l'intermédiaire d'une liaison de type ester.
- par dimère cyclique, il est entendu le composé constitué par la formule suivante : 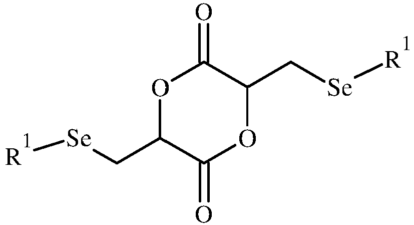 qui relie entre eux 2 monomères de composés de formule générale (I) tels que décrits dans l'invention par l'intermédiaire de 2 liaisons ester entre les 2 groupements hydroxy-acides.

Dans un autre mode de réalisation, le composé sélénié en particulier de formule (I) est choisi parmi les composés cités en exemples.

Ainsi, le composé sélénié en particulier de formule (I) peut être choisi parmi le groupe consistant de :
l'ester méthylique de l'acide 2-hydroxy-3-(méthylséléno)propanoïque ;
l'ester méthylique de l'acide (*R*)-2-hydroxy-3-(méthylséléno)propanoïque ;
l'ester méthylique de l'acide (*S*)-2-hydroxy-3-(méthylséléno)propanoïque ;
l'ester éthylique de l'acide 2-hydroxy-3-(méthylséléno)propanoïque ;
l'ester *tert*-butylique de l'acide 2-hydroxy-3-(méthylséléno)propanoïque ;
l'ester benzylique de l'acide (*S*)-(2-hydroxy-3-(méthylséléno)propanoïque ;
l'ester benzylique de l'acide (*S*)-(2-hydroxy-3-(méthylséléno)propanoïque ;
l'ester méthylique de l'acide 3-(éthylséléno)-2-hydroxypropanoïque ;
l'ester méthylique de l'acide 2-hydroxy-3-(isobutylséléno)propanoïque ;
l'ester isopropyle de l'acide 2-hydroxy-3-(méthylséléno)propanoïque ;
l'acide 2-hydroxy-3-(méthylséléno)propanoïque ;
l'acide (*R*)-2-hydroxy-3-(méthylséléno)propanoïque ;
l'acide (S)-2-hydroxy-3-(méthylséléno)propanoïque ;
l'acide 3-éthylséléno-2-hydroxypropanoïque ;
l'acide 2-hydroxy-3-(isobutylséléno)propanoïque ;
le sel dicyclohexylammonium 2-hydroxy-3-(méthylséléno)propanoate ;
le sel de sodium 2-hydroxy-3-(méthylséléno)propanoate ;
le sel de magnésium bis(2-hydroxy-3-(méthylséléno)propanoate ;
le sel du zinc bis(2-hydroxy-3-(méthylséléno)propanoate ;
le sel du calcium bis(2-hydroxy-3-(méthylséléno)propanoate ;
le 2-hydroxy-3-(méthylséléno)propanamide ;
le N-cyclopropyl-2-hydroxy-3-(méthylséléno)propanamide ;
le N-[2-(diméthylamino)éthyl]-2-hydroxy-3-(méthylséléno)propanamide ;
la 2-hydroxy-3-(méthylséléno)-1-(pyrrolidin-1-yl)propan-1-one ;
la 2-hydroxy-3-(méthylséléno)-1-(pipéridin-1-yl)propan-1-one ;
la 2-hydroxy-3-(méthylséléno)-1-(morpholin-4-yl)propan-1-one ;
le N,N-diéthyl-2-hydroxy-3-(méthylséléno)-propanamide ;
le 2-hydroxy-N-(2-hydroxyéthyl)-3-(méthylséléno)-propanamide ;
l'ester *tert*-butylique du [(2*RS*)-2-hydroxy-3-méthylsélénopropanoyl]-(*S*)-alanine ;
la [(*2RS*)-2-hydroxy-3-méthylsélénopropanoyl]-(*S*)-alanine ;
l'ester méthylique du [(*2RS*)-2-hydroxy-3-méthylsélénopropanoyl]-(*S*)-méthionine ;
la [(2*RS*)-2-hydroxy-3-méthylsélénopropanoyl]-(*S*)-méthionine ;
l'ester méthylique de la [(2*R*)-2-hydroxy-3-méthylsélénopropanoyl]-(*S*)-méthionine ;
la [(2*R*)-2-hydroxy-3-méthylsélénopropanoyl]-(*S*)-méthionine ;
l'ester méthylique de la [(2*S*)-2-hydroxy-3-méthylsélénopropanoyl]-(*S*)-méthionine ;
la [(2*S*)-2-hydroxy-3-méthylsélénopropanoyl]-(*S*)-méthionine;
l'ester méthylique de la N(α)-[(2*RS*)-2-hydroxy-3-méthylsélénopropanoyl]-N (ω)-*tert*-butoxycarbonyl-(*S*)-lysine ;
l'ester méthylique de la N(α)-[(2*RS*)-2-hydroxy-3-méthylsélénopropanoyl]-N (ω)-fluorenylméthyloxycarbonyl-(*S*)-lysine ;
l'ester méthylique de la N(α)-[(2*RS*)-2-hydroxy-3-méthylsélénopropanoyl]-N (ω)-benzyloxycarbonyl-(*S*)-lysine ;
l'ester méthylique de la [(2*RS*)-2-hydroxy-3-méthylsélénopropanoy1]-(*S*)-sélénométhionine ;
la [(2*RS*)-2-hydroxy-3-méthylsélénopropanoyl]-(*S*)-sélénométhionine ;
l'ester méthylique de la [(2*R*)-2-hydroxy-3-méthylsélénopropanoyl]-(*S*)-sélénométhionine ;
la [(2*R*)-2-hydroxy-3-méthylsélénopropanoyl]-(*S*)-sélénométhionine ;
l'ester méthylique de la [(2*S*)-2-hydroxy-3-méthylsélénopropanoyl]-(*S*)-sélénométhionine ;
la [(2*S*)-2-hydroxy-3-méthylsélénopropanoyl]-(*S*)-sélénométhionine ;
l'acide 2-(acétyloxy)-3-(méthylséléno)propanoïque ;
l'acide 2-(dodécanoyloxy)-3-(méthylséléno)propanoïque ;
l'ester méthylique de l'acide 2-(benzoyloxy)-3-(méthylséléno)propanoïque ;
l'acide 2-(benzoyloxy)-3-(méthylséléno)propanoïque ;
l'ester méthylique de l'acide 3-(méthylséléno)-2-[(3'-pyridine)oxycarbonyl]propanoïque ;
l'ester méthylique de l'acide 2-[(*tert*-butoxycarbonyl)oxy]-3-(méthylséléno)propanoïque ;
l'acide 2-[(*tert*-butoxycarbonyl)oxy]-3-(méthylséléno)propanoïque ;
l'ester méthylique de l'acide (2*RS*)-[N-(*tert*-butoxycarbonyl)-*S*-methionyl]-3-(méthylséléno)propanoïque ;
l'ester méthylique de l'acide 4-méthylséléno-2-(2'-acétyloxy-3'-méthylsélénopropanoyl)butyrique ;
l'acide 4-méthylséléno-2-(2'-acétyloxy-3'-méthylsélénopropanoyl)butyrique ;
l'ester méthylique de l'acide 3-méthylséléno-2-(2'-acétyloxy-4'-méthylsélénobutanoyl)propanoïque ;
l'acide 3-méthylséléno-2-(2'-hydroxy-4'-méthylsélénobutanoyl)-propanoïque ;
l'acide 2-(pentanoyloxy)-3-(méthylséléno)-propanoïque ;
l'acide 2-(nonanoyloxy)-3-(méthylséléno)-propanoïque ;
l'acide 2-(linoleoyloxy)-3-(méthylséléno)-propanoïque ;
l'ester méthylique de l'acide 2-(linoleoyloxy)-3-(méthylséléno)-propanoïque ;
l'acide 2-(pivaloyloxy)-3-(méthylséléno)-propanoïque ;
l'acide 2-(3-chloropropanoyloxy)-3-(méthylséléno)-propanoïque ;
l'acide 2- {(1H-imidazoyl-4-ylcarbonyl)oxy)}-3-(méthylséléno)-propanoïque ; l'ester méthylique de l'acide 2-(pivaloyloxy)-3-(méthylséléno)-propanoïque ;
ainsi que leurs mélanges en toutes proportions.

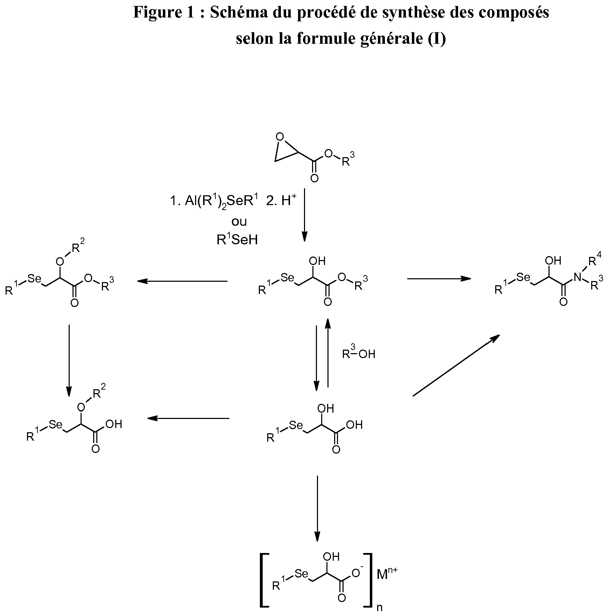

Selon un second aspect, l'invention concerne un procédé de préparation des nouveaux composés organo-séléniés et de leurs dérivés esters et amides en particulier de formule générale (I), explicité sur la Figure 1, et caractérisé en ce qu'il comprend les étapes suivantes :
1) la réaction d'un ester de l'acide oxirane-2-carboxylique racémique (DL) ou de l'un de ses énantiomères (D ou L) disponibles commercialement (par exemple chez SAF France), avec
   a/ soit avec un dérivé dialkyle aluminium alkylsélénolate de formule Al(R¹)₂SeR¹, lui-même généré *in situ* à partir du trialkyle aluminium A1(R¹)₃ correspondant, disponible commercialement (par exemple chez SAF, France) et du sélénium élémentaire Se° disponible commercialement (par exemple chez SAF, France) (selon Kozikowski AP and Ames A, 1978, J. Org. Chem., 43(13):2735).
   b soit un alkylsélénole R¹SeH, lui-même préparé *in situ* à partir d'un sel alcalin d'alkyle sélénolate de formule R¹-Se-M₁ obtenu lui-même par réduction du dialkyle disélénure correspondant disponible commercialement (par exemple chez SAF, France) ou par réaction entre du sélénium métal Se(0) et un sel d'alkyle lithium correspondant disponible commercialement (par exemple chez SAF, France) dans laquelle M₁ représente un atome de métal alcalin), mis en réaction avec du chlorure d'ammonium (en analogie avec l'ouverture des alkyles oxirane carboxylates avec un thiol tel que décrit dans Org. Letters, 2004, 6(4), 497) ;
2) le cas échéant une, ou plusieurs, des réactions ou séries de réactions suivantes bien connues de l'homme de l'art:
   - hydrolyse de la fonction ester, puis
   - acidification du milieu réactionnel pour obtenir les acides correspondants en particulier de formule (I) où X = OH; puis
   - estérification des acides en particulier de formule (I) ou de leurs sels alcalins de formule (la) avec un alcool ou un halogénure d'alkyle pour obtenir les esters correspondants de formule générale (I) dans laquelle X = OR³, avec R³ tel que défini précédemment;
   - amidification des acides en particulier de formule (I) avec une amine appropriée de formule R⁴R⁵NH, ou NH₃ dans laquelle R⁵ est tel que défini précédemment, pour obtenir le composé sélénié en particulier de formule générale (I) dans laquelle X = NH2, NR⁴R⁵ ou α-aminoacide, CH₃SeCH₂CH₂CH(COOH)NH-;
   - estérification, lorsque R² = H, de la fonction hydroxyle par un acide aproprié pour obtenir le composé sélénié en particulier de formule générale (I) dans laquelle OR² est différent du groupe OH ;
   - salification par un acide ou par une base.

Selon une mise en œuvre particulière du procédé selon l'invention :
le réactif sélénié est :
   * soit un dialkyle aluminium alkylsélénolate, et par exemple le diméthyle aluminium méthylsélénolate généré *in situ* à partir de sélénium métal Se° et du triméthyle aluminium Al(CH₃)₃ dans un solvant aprotique tel que par exemple le tétrahydrofurane (THF).
   * soit un alkylsélénole, généré *in situ* à partir de sélénium métal Se(0) et d'alkyle lithium, dans un solvant aprotique tel que par exemple le tétrahydrofurane (THF), puis mis en présence de chlorure d'ammonium

On opère au sein d'un solvant polaire aprotique tel que par exemple le THF. Les réactions subséquentes conduisant aux différents composés de formule (I), soit acidification, estérification, amidification, salification, sont effectuées dans des conditions bien connues de l'homme du métier.

Les oligomères (dimères, trimères) et polymères, linéaires ou ramifiés, acycliques ou cycliques, obtenus entre deux ou plusieurs molécules de dérivés de formule (I) décrits dans l'invention par réaction d'estérification entre les fonctions alcool et acide carboxylique présentes le cas échéant, pris isolément ou en mélange, sont obtenus par condensation et déhydratation en analogie avec les exemples décrits dans Acta Chemica Scandinavica B, 1980, 34, 633-636 ou encore selon EP2238124.

Dans le cadre de la préparation d'esters séléno-méthyliques (RCOSeMe), Kosikowski et Ames décrivent l'obtention, en proportion minoritaire, d'un sous-produit non désiré qui est le 3-méthylséléno-2-hydroxy-propanoate d'éthyle [Kosikowski, AP and Ames A, 1978, J. Org. Chem., 43(13):2735]. Bien que n'en faisant pas partie, ce composé est structuralement proche des composés de formule générale (I) décrits dans la présente invention.

Selon un troisième aspect l'invention a également pour objet un composé sélénié en particulier de formule générale (I) tel que précédemment défini, pour son utilisation comme agent pharmaceutique, en particulier comme agent anti-tumoral, seul ou associé à au moins un autre agent pharmaceutique et notamment à au moins un agent anti-tumoral, dans une méthode de traitement en particulier pour réaliser la prévention ou le le traitement de tumeurs ou cancers, soit seul soit en association avec un ou plusieurs autres agents anti-cancéreux ou cytotoxiques connus, et soit en pré-administration soit en co-administration, tels que ceux notamment de la prostate, du foie, des reins, du pancréas, des poumons, du colon et de la peau ;

Par agent anti-tumoral on entend tout agent ayant la propriété de traiter une tumeur ou communément appelée « cancer ».

Selon un quatrième aspect, l'invention concerne aussi une composition pharmaceutique comprenant au moins un principe pharmaceutiquement actif comprenant au moins un composé sélénié en particulier_de formule générale (I), tel que précédemment défini, seul ou associé avec au moins un autre principe pharmaceutiquement actif.

En particulier, le composé sélénié en particulier de formule générale (I), tel que précédemment défini selon l'invention, permet de réaliser le traitement de tumeurs ou cancers, soit seul soit en association avec un ou plusieurs autres agents anti-cancéreux ou cytotoxiques connus, et soit en pré-administration soit en co-administration, tels que ceux notamment de la prostate, du foie, des reins, du pancréas, des poumons, du colon et de la peau ;

Comme autres agents anti-cancéreux, il est entendu les composés suivants : les inhibiteurs de métalloprotéinase de matrice tels que l'aminoglutéthimide, l'estramustine, le medroxyprogesteroneacetate, le leuprolide, le flutamide, le torémifène, le Zoladex; les inhibiteurs du VEGF, tels que les anticorps anti-VEGF (Avastin (R)) et les petites molécules comme le ZD6474 et le SU6668; le vatalanib, le BAY-43-9006, le SU11248, le CP-547632, et le CEP-7055; les inhibiteurs de l'EGFR, tels que le gefitinib, l'erlotinib, le ABX-EGF, l'EMD72000, le 11F8, et le cetuximab ; les inhibiteurs d'Eg5, tels que le SB-715992, le SB-743921, et le MKI-833; les inhibiteurs de PAN, tels que le canertinib, le EKB-569, le CI-1033, l'AEE-788, et le XL-647; les inhibiteurs de kinase, terls que le 2C4, le GW-572016, le Gleevec (R) et le dasatinib (Sprycel (R); le Casodex (R) (bicalutamide, Astra Zeneca), le tamoxifène; les inhibiteurs de MAPK kinase, les inhibiteurs de PI3 kinase, les inhibiteurs de PDGF, tels que l'imatinib; les inhibiteurs de récepteurs tyrosine kinases, les inhibiteurs de la signalisation de l'intégrine; la tubuline ; les agents agissant comme la vinblastine, la vincristine, la vinorelbine, vinflunine, le paclitaxel, le docétaxel, le 7-O-methylthiomethylpaclitaxel, le 4-désacétyl-4-methylcarbonatepaclitaxel, le C-4 méthyl carbonate paclitaxel, l'epothilone A, l'epothilone B, l'épothilone C, l'epothilone D, la désoxyépothilone A, la désoxyépothilone B, l'oxabicyclo [14.1.0], l'heptadécane-5-9-dione (ixabepilone), et des dérivés de ceux-ci; les inhibiteurs de CDK, les inhibiteurs du cycle cellulaire anti-prolifératifs, l'epidophyllotoxin, l'étoposide, le VM-26; les inhibiteurs de topoisomérase I ou II, tels que la camptothécine, le topotécan, la SN-38, la procarbazine, la mitoxantrone ; les complexes de coordination du platine tels que le cisplatine, le carboplatine et l'oxaliplatine; les inhibiteurs de croissance, les agents thérapeutiques anti-hormonal; la leucovorine; le tégafur; les antimétabolites tels que les antagonistes de la purine (par exemple la 6-thioguanine et la 6-mercaptopurine); les antagonistes de la glutamine.

Comme autres agents cytotoxiques, il est entendu les composés suivants : le cyclophosphamide, la doxorubicine, la daunorubicine, la mitoxantrone, le melphalan, l'hexaméthyle mélamine, le thiotépa, la cytarabine, l'idatrexate, trimétrexate, la dacarbazine, la L-asparaginase, le bicalutamide, le leuprolide, les dérivés du pyridobenzoindole, les interférons, les interleukines.

Selon un cinquième aspect l'invention a pour objet les compositions pharmaceutiques renfermant les composés séléniés en particulier de formule générale (I) à titre de principe actif.

Selon ce cinquième aspect de la présente invention, les composés séléniés en particulier de formule générale (I) sont par exemple utilisés dans des quantités se situant entre 0,02% et 0,15% équivalents Sélénium (eq. Se) en poids de la préparation.

Selon ce cinquième aspect de la présente invention, les compositions pharmaceutiques comprennent un support pharmaceutiquement acceptable et une quantité thérapeutiquement efficace d'un ou plusieurs des composés de la présente invention ou d'un stéréoisomère, d'un tautomère, d'un sel pharmaceutiquement acceptable. Ces supports peuvent notamment consister en :
* une solution injectable ou buvable,
* un milieu solide composé d'un ou plusieurs excipients pouvant être sélectionnés parmi des vitamines, des antioxydants naturels tel que la L-ergothionéine, des sels minéraux, des mono-, di- ou polysaccharides, notamment l'acide folique, les vitamines B₆, E ou C, du lactose, de l'amidon. Ce milieu solide composé d'un ou plusieurs excipients tels que définis ci-dessus, et comprenant au moins l'un des composés de formule générale (I), peut être formulé sous la forme d'une gélule, d'un comprimé ou d'une poudre.

Selon ce cinquième aspect de la présente invention, les compositions pharmaceutiques renfermant au moins l'un des composés de formule générale (I) à titre de principe actif, peuvent selon le cas être administrées par voie orale, intraveineuse, par voie parentérale, par voie topique incluant la voie transdermique ou nasale ou oculaire, ou par inhalation. Les quantités des différents constituants de ces compositions, autres que les composés séléniés en particulier de formule générale (I), sont celles habituellement utilisées pour les applications citées.

La présente invention englobe toutes les combinaisons des aspects et / ou modes de réalisation de l'invention cités ci-dessus. Il est entendu que tout mode de réalisation de la présente invention peut être pris en conjonction avec un autre mode de réalisation pour décrire des modes de réalisation supplémentaires plus préférés. Il est également bien entendu que chaque élément individuel des modes de réalisation préférés est son propre mode de réalisation préféré indépendant.

### Description des figures :

**Figure 1** **: Schéma du procédé de synthèse des composés selon la formule générale (I)**
**Figure 2** **: Pourcentage de viabilité des cellules DU145 et LS174T en fonction de la concentration en composé 4, après 96 heures de traitement**
**Figure 3** **: Pourcentage de viabilité des cellules DU145, LS174T et HT-29 en fonction de la concentration en composé 10, après 96 heures de traitement**
**Figure 4** **: Pourcentage de viabilité des cellules PC3, DU145, PANC-1 et MIA PaCa-2 en fonction de la concentration en composé 38, après 96 heures de traitement**
**Figure 5** **: Pourcentage de viabilité des cellules HT-29, LS174T, HepG2 et MCF-7 en fonction de la concentration en composé 38, après 96 heures de traitement**

### Exemples

Les exemples ci-après, de même que le schéma du procédé de l'invention (Figure 1) et les autres figures, sont fournis simplement à titre d'illustration et ne sauraient, en aucune façon, limiter la portée de l'invention.

Dans les exemples, décrits ci-après, tous les pourcentages sont donnés en poids, la température est la température ambiante ou est donnée en degré Celsius, et la pression est la pression atmosphérique, sauf indication contraire.

Les réactifs utilisés sont disponibles commercialement chez des fournisseurs internationaux tel que SAF (France), Alfa Aesar , Fisher Scientific, TCI Europe, Bachem (Suisse) sauf les composés suivants, qui ont été préparés selon les protocoles cités : l'ester éthylique de l'oxirane-2-carboxylate (selon Org . Synth. 2006, 83, 162-169) ; l'ester tert-butylique de l'oxirane-2-carboxylate (selon J. Am. Chem. Soc. 2008, 130 (31), 10096-10102) l'ester benzylique du (*R*)-oxirane-2-carboxylate (J. Org. Chem. 1992, 57 (11), 3380-3387).

### I. Exemples de préparation des composés selon l'invention

### 1a-Préparation des composés A par introduction du sélénium: Esters de l'acide 3-(alkylséléno)-2-hydroxy-propanoïque

Les composés A sont préparés par réaction d'un alkylsélénole R¹SeH ou d'un dialkyle aluminium alkylsélénolate Al(R¹)₂SeR¹ (généré *in situ* à partir du trialkyle aluminium et du sélénium élémentaire Se° selon A. P. Kozikowski and A. Ames, J. Org. Chem. 1978, 43, 2735*),* avec un alkyle oxirane carboxylate.

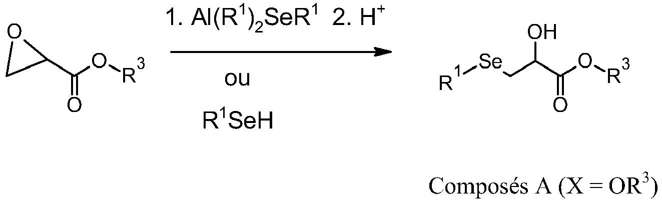

### Exemple A1 : Préparation de l'ester méthylique de l'acide 2-hydroxy-3-(méthylséléno)propanoïque (Composé 1) utilisant le diméthylaluminium méthylsélénolate

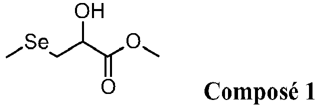

8,6 g (12,6 ml; 25,2 mmoles; 1,1 équiv.) d'une solution 2 M de triméthyle aluminium dans le toluène sont ajoutés goutte à goutte (temps d'addition 15 min) sous azote à 2,0 g (25,2 mmoles; 1,1 équiv.) de sélénium Se°. La suspension est agitée 15 min à température ambiante puis 2 h à reflux en milieu clos. Le milieu est laissé revenir à température ambiante puis refroidie à 0°C sous azote.
2,386 g (22,9 mmoles) de méthyle oxirane-2-carboxylate en solution dans 12 ml de dichlorométhane sont additionnés goutte à goutte au milieu réactionnel (temps d'addition 15 min). Le milieu est laissé sous agitation pendant 30 min à 0°C puis 16 h à température ambiant.

Le milieu réactionnel est refroidi à - 4°C pendant 15 min. 1,286 g (24,05 mmoles; 1,05 équiv.) d'ammonium chlorure dans 10 mL d'eau sont additionnés goutte à goutte très lentement au milieu réactionnel (temps d'addition 15 min) car un fort dégagement gazeux se produit *(Attention : génération et dégagement de méthane).* 20 mL de dichlorométhane sont additionnés goutte à goutte puis le milieu est laissé sous agitation pendant 10 min à froid. 20 mL d'une solution aqueuse saturée de NH₄Cl sont additionnés goutte à goutte puis le milieu est laissé sous agitation pendant 10 min à froid.

Le milieu réactionnel est filtré sur Célite qui est rincée avec du dichlorométhane (7x20 mL). La phase organique est décantée et la phase aqueuse est extraite avec du dichlorométhane (3x20 mL). Les phases organiques sont regroupées, séchées avec Na₂SO₄, filtrées et concentrées.

L'huile jaune obtenue est distillée sous pression réduite de 8 mbar (125°C). On obtient 1,998 g (42 %) du composé **1** sous forme d'une huile jaune.
¹H-RMN (CDCl₃, 400 MHz) : δ (ppm) = 2,09 (s, 3H) ; 2,91 (m, 1H) ; 3,01 (m, 1H) ; 3,18 (m, 1H) ; 3,83 (s, 3H) ; 4,51 (m, 1H).
¹³C-RMN (CDCl₃, 75 MHz) : δ (ppm) = 5,9; 29,9; 53,1; 70,7; 174,1 UPLC-MS (AP+) : 220,8 (M+Na)⁺

### Exemple A2 : Préparation de l'ester méthylique de l'acide 2-hydroxy-3-(méthylséléno)propanoïque (Composé 1) utilisant le méthylsélénol

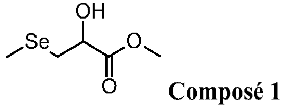

992 mg (12,5 mmoles; 1 éq.) de sélénium Se° sont mis en suspension sous azote dans 44 ml de THF. La suspension est refroidie à -3°C puis 6,2 ml (18,6 mmoles; 1,49 éq.) d'une solution 3 M de méthyllithium dans le diéthoxyméthane sont ajoutés goutte à goutte (temps d'addition 7 min). Le milieu complètement décoloré est agité 20 min à froid puis 802 mg (15 mmoles; 1,2 éq.) d'ammonium chlorure en solution dans 44 ml de méthanol sont ajoutés goutte à goutte. Le milieu est agité 20 min à froid puis 2,11 ml (15 mmoles; 1,2 éq.) de triéthylamine sont additionnés. Le milieu est agité 20 min à froid puis 1,71 g (16,25 mmoles; 1,3 éq.) de méthyl oxirane-2-carboxylate sont additionnés. Le milieu est laissé sous agitation pendant 1 h à 0°C puis 22 h à température ambiant.

Le milieu réactionnel est refroidi à 0°C pendant 15 min. 90 mL de dichlorométhane sont additionnés goutte à goutte puis le milieu est laissé sous agitation pendant 10 min à froid. 90 mL d'une solution aqueuse saturée de NH₄Cl sont additionnés goutte à goutte puis le milieu est laissé sous agitation pendant 10 min à froid. Le milieu est dilué avec 90 mL d'eau. La phase organique est récupérée et la phase aqueuse est extraite avec du dichlorométhane (2x90 mL). Les phases organiques sont regroupées, séchées avec Na₂SO₄, filtrées et concentrées. L'huile jaune obtenue est purifiée sur colonne de silice (cyclohexane/acétate d'éthyle).

On obtient 330 mg (14 %) du composé **1.**

### Exemple A3 : Préparation de l'ester méthylique de l'acide (R)-2-hydroxy-3-(méthylséléno)propanoïque (Composé 2)

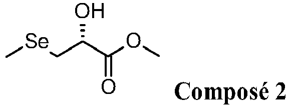

Le composé **2** est obtenu en utilisant les conditions de l'exemple A1 en partant de 4,959 g de méthyle (S)-oxirane-2-carboxylate. Après rinçage de la Célite et évaporation des solvants, l'huile obtenue est purifiée sur colonne de silice (cyclohexane/acétate d'éthyle).

On obtient 3,257 g (33 %) du composé **2** sous forme d'une huile jaune.

Le spectre ¹H RMN est identique à celui obtenu dans l'exemple A1. ${\left[α\right]}_{D} : + 11,5 \left(c=1,0;MeOH\right)$

### Exemple A4 : Préparation de l'ester méthylique de l'acide (S)-2-hydroxy-3-(méthylséléno)propanoïque (Composé 3)

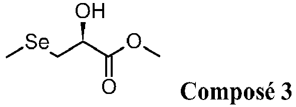

Le composé **3** est obtenu en utilisant les conditions de l'exemple A1 en partant de 7 g de méthyle (*R*)-oxirane-2-carboxylate. Après rinçage de la Célite et évaporation des solvants, l'huile obtenue est purifiée sur colonne de silice (cyclohexane/acétate d'éthyle).

On obtient 4,04 g (29 %) du composé **3** sous forme d'une huile orange.

Le spectre ¹H RMN est identique à celui obtenu dans l'exemple A1. ${\left[α\right]}_{D} : - 11,1 \left(c=1,0;MeOH\right)$

### Exemple A5 : Préparation de l'ester éthylique de l'acide 2-hydroxy-3-(méthylséléno)propanoïque (Composé 4)

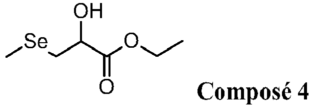

Le composé 4 est obtenu en utilisant les conditions de l'exemple A1 en partant de 1,56 g d'éthyle oxirane-2-carboxylate. Après rinçage de la Célite et évaporation des solvants, l'huile obtenue est purifiée sur colonne de silice (cyclohexane/acétate d'éthyle).

On obtient 1,188 g (38 %) du composé 4 sous forme d'une huile jaune.
¹H-RMN (CDCl₃, 400 MHz) : δ (ppm) = 1,35 (t, J=7,0 Hz, 3H) ; 2,10 (s, 3H) ; 2,91 (dd, J=13,0 Hz, J=5,5 Hz, 1H) ; 3,01 (dd, J=13,0 Hz, J=4,0 Hz, 1H) ; 3,16 (d, J=6,0 Hz, 1H) ; 4,3 (q, J=7,0 Hz, 2H) ; 4,5 (td, J=5,5 Hz, J=4,0 Hz, 1H).
¹³C-RMN (CDCl₃, 75 MHz) : δ (ppm) = 6,0; 14,6; 29,9; 62,4; 70,8; 173,7 UPLC-MS (AP+) : 234,8 (M+Na)⁺

### Exemple A6 : Préparation de l'ester tert-butylique de l'acide 2-hydroxy-3-(méthylséléno)propanoïque (Composé 5)

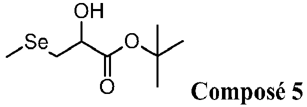

Le composé **5** est obtenu en utilisant les conditions de l'exemple A1 en partant de 1,0 g de *ter*-butyle oxirane-2-carboxylate. Après rinçage de la Célite et évaporation des solvants, l'huile obtenue est purifiée sur colonne de silice (cyclohexane/acétate d'éthyle).

On obtient 889 mg (54 %) du composé **5** sous forme d'une huile légèrement jaune.
¹H-RMN (CDCl₃, 400 MHz) : δ (ppm) = 1,53 (s, 9H) ; 2,13 (s, 3H) ; 2,87 (dd, J=13,0 Hz, J=5,5 Hz, 1H) ; 2,97 (dd, J=13,0 Hz, J=4,0 Hz, 1H) ; 3,19 (d, J=5,5 Hz, 1H) ; 4,39 (td, J=5,5 Hz, J=4,0 Hz, 1H).

### Exemple A7 : Préparation de l'ester benzylique de l'acide (S)-(2-hydroxy-3-(méthylséléno)propanoïque (Composé 6)

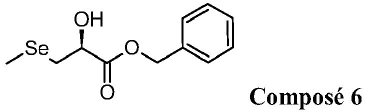

Le composé **6** est obtenu en utilisant les conditions de l'exemple A1 en partant de 622 mg de benzyle (*R*)-oxirane-2-carboxylate. Après rinçage de la Célite et évaporation des solvants, l'huile obtenue est purifiée sur colonne de silice (cyclohexane/acétate d'éthyle).

On obtient 325 mg (33 %) du composé **6** sous forme d'une huile légèrement jaune.
¹H-RMN (CDCl₃, 400 MHz) : δ (ppm) = 2,05 (s, 3H) ; 2,91 (dd, J=13,0 Hz, J=5,5 Hz, 1H) ; 3,02 (dd, J=13,0 Hz, J=4,5 Hz, 1H) ; 3,18 (d, J=5,5 Hz, 1H) ; 4,55 (td, J=5,5 Hz, J=4,5 Hz, 1H), 5,26 (s, 2H) ; 7,41 (m, 5H).

### Exemple A8 : Préparation de l'ester méthylique de l'acide 3-(éthylséléno)-2-hydroxypropanoïque (Composé 7)

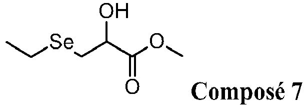

Le composé **7** est obtenu en utilisant les conditions de l'exemple A1 en partant de 2 g de sélénium Se°, 10,98 g d'une solution 25 % de triéthyle aluminium dans le toluène, et 2,386 g de méthyle oxirane-2-carboxylate. Après rinçage de la Célite et évaporation des solvants, l'huile obtenue est purifiée sur colonne de silice (cyclohexane/acétate d'éthyle).

On obtient 1,977 g (40 %) du composé **7** sous forme d'une huile jaune.
¹H-RMN (CDCl₃, 400 MHz) : δ (ppm) = 1,41 (t, J=7,5 Hz, 3H) ; 2,67 (q, J=7,5 Hz, 2H) ; 2,92 (dd, J=13,0 Hz, J=5,5 Hz, 1H) ; 3,02 (dd, J=13,0 Hz, J=4,5 Hz, 1H) ; 3,18 (d, J=4,5 Hz, 1H) ; 3,82 (s, 3H) , 4,5 (m, 1H).
¹³C-RMN (CDCl₃, 75 MHz) : (ppm) = 16,2; 18,9; 27,8; 53,1; 70,8; 174,1

### Exemple A9 : Préparation de l'ester méthylique de l'acide 2-hydroxy-3-(isobutylséléno)propanoïque (Composé 8)

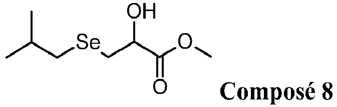

Le composé **8** est obtenu en utilisant les conditions de l'exemple A1 en partant de 2,0 g de sélénium Se°, 19,08 g d'une solution 25 % de triisobutyle aluminium dans le toluène et 2,386 g de méthyle oxirane-2-carboxylate. Après rinçage de la Célite et évaporation des solvants, l'huile obtenue est purifiée sur colonne de silice (cyclohexane/acétate d'éthyle).

On obtient 2,291 g (42 %) du composé **8** sous forme d'une huile jaune.
¹H-RMN (CDCl₃, 300 MHz) : δ (ppm) = 0,77 (d, J=6,5 Hz, 6H) ; 1,61 (m, 1H) ; 2,39 (d, J=7,0 Hz, 2H) ; 2,66 (dd, J=13,0 Hz, J=5,5 Hz, 1H) ; 2,76 (dd, J=13,0 Hz, J=4,5 Hz, 1H) ; 3,58 (s, 3H) ; 4,25 (m, 1H).
¹³C-RMN (CDCl₃, 75 MHz) : (ppm) = 22,9; 29,0; 29,7; 35,7; 53,1; 70,7; 174,0

### lb- Préparation des composés A par estérification

Les composés A peuvent être obtenus par introduction des réactifs séléniés, comme décrit dans le paragraphe la ci-dessus, mais également par estérification des composés B (pour leur préparation voir paragraphe 2 ci-après):

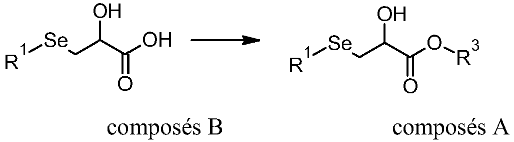

### Exemple A10: Préparation de l'ester éthylique de l'acide 2-hydroxy-3-(méthylséléno)propanoïque (Composé 4)

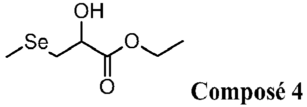

500 mg (2,68 mmoles; 1 éq.) de l'acide 2-hydroxy-3-(méthylséléno)propanoïque **(10,** paragraphe 2) sont mis en solution dans 11 mL d'éthanol absolu sous azote. 66 mg (1,07 mmoles; 0,4 éq.) d'acide orthoborique sont ajoutés au milieu. Le milieu est laissé au reflux sous agitation et sous azote pendant 48 h.

33 mg (535 µmoles; 0,2 éq.) d'acide orthoborique sont ajoutés de nouveau au milieu. Le milieu est laissé au reflux pendant 24 h.

Le milieu est concentré à sec puis le concentrât est repris avec une solution aqueuse de NH4Cl demi saturée (40 mL). Le milieu est extrait par acétate d'éthyle (3x40 mL). Les phases organiques sont regroupées, séchées avec Na₂SO₄, filtrées et concentrées.

On obtient 446 mg (77 %) du composé **4** sous forme d'une huile jaune.

Le spectre ¹H RMN est identique à celui obtenu dans l'exemple A5.

### Exemple A11: Préparation de l'ester isopropyle de l'acide 2-hydroxy-3-(méthylséléno)propanoïque (Composé 9)

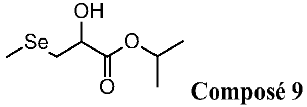

374 mg (2 mmoles; 1 éq.) de l'acide 2-hydroxy-3-(méthylséléno)propanoïque **(10)** sont mis en solution dans 8 mL de propanol-2 sous azote. 49 mg (800 µmmoles; 0,4 éq.) d'acide orthoborique sont ajoutés au milieu. Le milieu est laissé au reflux sous agitation et sous azote pendant 48 h.

25 mg (400 µmoles; 0,2 éq.) d'acide orthoborique sont ajoutés de nouveau au milieu. Le milieu est laissé au reflux pendant 24 h.

Le milieu est concentré à sec puis le concentrât est repris avec une solution aqueuse de NaHCO₃ demi saturée (40 mL). Le milieu est extrait par acétate d'éthyle (3x40 mL). Les phases organiques sont regroupées, séchées avec Na₂SO₄, filtrées et concentrées. L'huile obtenue est purifiée sur colonne de silice (cyclohexane/acétate d'éthyle).

On obtient 285 mg (62 %) du composé **9** sous forme d'une huile jaune.
¹H-RMN (CDCl₃, 400 MHz) : δ (ppm) = 1,32 (d, J=6,0 Hz, 6H) ; 2,11 (s, 3H) ; 2,89 (dd, J=13,0 Hz, J=5,5 Hz, 1H) ; 2,99 (m, 1H) ; 3,20 (s, 1H) ; 4,45 (m, 1H) ; 5,14 (m, 1H).
¹³C-RMN (CDCl₃, 75 MHz) : δ (ppm) = 6,0; 22,2; 29,8; 70,4; 70,9; 173,2

UPLC-MS (AP+) : 248,9 (M+Na)⁺

### 2- Préparation des composés B et C: les acides 3-(alkylséléno)-2-hydroxypropanoique et les sels correspondants

Les composés B sont préparés par hydrolyse de la fonction ester des composés A, et les sels correspondants C sont obtenus par réaction des composés B avec des oxydes ou hydroxydes :

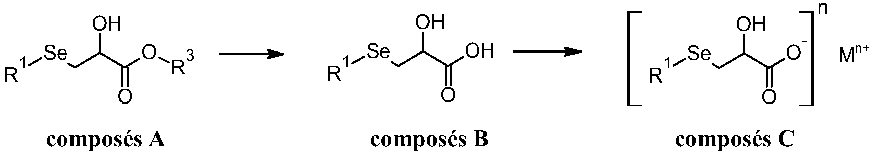

### Exemple B1 : Préparation de l'acide 2-hydroxy-3-(méthylséléno)propanoïque (Composé 10) à partir de l'ester de méthyle

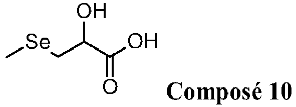

4,908 g (23,53 mmoles) de composé **1** sont solubilisés dans 14 ml de THF, 5 ml de méthanol et 5 ml d'eau déminéralisée. 47,1 ml (47,07 mmoles; 2 équiv.) d'une solution aqueuse d'hydroxyde de lithium à 1M sont ajoutés, la solution est agitée à température ambiante pendant 16 h.

Le pH du milieu est ajusté à 1 par l'ajout d'une solution d'acide chlorhydrique 2 M (14 mL). Le milieu est extrait à l'acétate d'éthyle (4x100 mL). Les phases organiques sont regroupées, séchées sur Na₂SO₄, filtrées et concentrées.

On obtient 4,177 g (95 %) du composé **10** sous forme d'un solide jaune pâle.
¹H-RMN (D₂O, 400 MHz) : δ (ppm) = 2,08 (s, 3H) ; 2,92 (dd, J=13,5 Hz, J=6,5 Hz, 1H) ; 3,03 (dd, J=13,5 Hz, J=4,5 Hz, 1H) ; 4,57 (dd, J=6,5 Hz, J=4,5 Hz, 1H). ¹H-RMN (DMSO, 400 MHz) : δ (ppm) = 1,99 (s, 3H) ; 2,70 (dd, J=12,5 Hz, J=6,5 Hz, 1H) ; 2,79 (dd, J=12,5 Hz, J=5,0 Hz, 1H) ; 4,18 (dd, J=6,5 Hz, J=5,0 Hz, 1H).
¹³C-RMN (CDCl₃, 75 MHz) : δ (ppm) = 6,0; 29,6; 70,1; 178,0
UPLC-MS (AP-) : 182,6 (M-H⁺)

Analyse élémentaire : C₄H₈O₃Se ; Théorique : C (26,24 %) ; H (4,4 %) ; Trouvé : C (26,9 %) ; H (4,42 %)

### Exemple B2 : Préparation de l'acide 2-hydroxy-3-(méthylséléno)propanoïque (Composé 10) à partir de l'ester tert-butylique

Le composé **10** est obtenu en utilisant les conditions de l'exemple B1 en partant de 100 mg de *ter-*butyle 2-hydroxy-3-(méthylséléno)propanoate **(5).**

On obtient 64 mg (82 %) du produit désiré sous forme d'un solide.

Le spectre ¹H RMN est identique à celui obtenu dans l'exemple B1.

### Exemple B3 : Préparation de l'acide (R)-2-hydroxy-3-(méthylséléno)propanoïque (Composé 11)

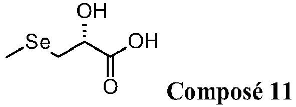

Le composé **11** est obtenu en utilisant les conditions de l'exemple B1 en partant de 2,117 g de méthyle (*R*)-2-hydroxy-3-(méthylséléno)propanoate **(2).**

On obtient 1,875 g (96 %) du produit désiré sous forme d'un solide jaune.

Le spectre ¹H RMN est identique à celui obtenu dans l'exemple B1. ${\left[α\right]}_{D} : + 1,07 \left(c=6,0;EtOH\right)$

### Exemple B4 : Préparation de l'acide (S)-2-hydroxy-3-(méthylséléno)propanoïque (Composé 12) à partir de l'ester de méthyle

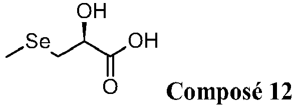

Le composé **12** est obtenu en utilisant les conditions de l'exemple B1 en partant de 2,95 g de méthyle (S)-2-hydroxy-3-(méthylséléno)propanoate **(3).**

On obtient 2,61 g (95 %) du produit désiré sous forme d'un solide jaune.

Le spectre ¹H RMN est identique à celui obtenu dans l'exemple B1. ${\left[α\right]}_{D} : - 1,03 \left(c=6,0;EtOH\right)$

### Exemple B5: Préparation de l'acide (S)-2-hydroxy-3-(méthylséléno)propanoïque (Composé 12) à partir de l'ester benzylique

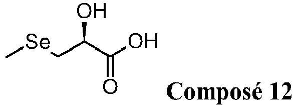

Le composé **12** est obtenu en utilisant les conditions de l'exemple B1 en partant de 494 mg de benzyle (*S*)-2-hydroxy-3-(méthylséléno)propanoate **(6).**

Après 16 h d'agitation à température ambiante, le milieu réactionnel est extrait à l'acétate d'éthyle (2x30 mL). Le pH du milieu est ajusté à 1 par l'ajout d'une solution aqueuse d'acide chlorhydrique à 2 M. Le milieu est extrait à l'acétate d'éthyle (2x30 mL). Les phases organiques de cette deuxième extraction sont regroupées, séchées sur Na₂SO₄, filtrées et concentrées.

On obtient 280 mg (84 %) du composé **12** sous forme d'un solide jaune pâle.

Le spectre ¹H RMN est identique à celui obtenu dans l'exemple B1.

### Exemple B6 : Préparation de l'acide 3-éthylséléno-2-hydroxypropanoïque (Composé 13) à partir de l'ester de méthyle

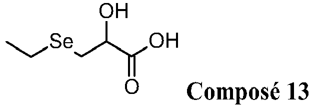

Le composé **13** est obtenu en utilisant les conditions de l'exemple B1 en partant de 500 mg de l'ester méthylique de l'acide 3-(éthylséléno)-2-hydroxypropanoïque **(7).** On obtient 471 mg (100 %) du composé **13** sous forme d'un solide jaune pâle.
¹H-RMN (CDCl₃, 400 MHz) : δ (ppm) = 1,44 (t, J=7,5 Hz, 3H) ; 2,71 (q, J=7,5 Hz, 2H) ; 3,00 (dd, J=13,5 Hz, J=6,0 Hz, 1H) ; 3,11 (dd, J=13,5 Hz, J=4,5 Hz, 1H) ; 4,55 (dd, J=6,0 Hz, J=4,5 Hz, 1H).
¹³C-RMN (CDCl₃, 75 MHz) : (ppm) = 16,1; 19,1; 27,6; 70,3; 177,9
UPLC-MS (AP-) : 197,2 (M-H⁺)

### Exemple B7 : Préparation de l'acide 2-hydroxy-3-(isobutylséléno)propanoïque (Composé 14) à partir de l'ester de méthyle

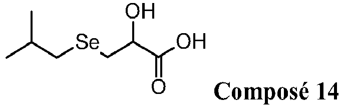

Le composé **14** est obtenu en utilisant les conditions de l'exemple B1 en partant de 500 mg de l'ester méthylique de l'acide 2-hydroxy-3-(isobutylséléno)propanoïque **(8).**

On obtient 423 mg (90 %) du composé **14** sous forme d'un solide jaune pâle.
¹H-RMN (CDCl₃, 400 MHz) : δ (ppm) = 1,03 (d, J=6,5 Hz, 6H) ; 1,88 (m, 1H) ; 1,95 (dd, J=7,0 Hz, 2H) ; 2,97 (dd, J=13,0 Hz, J=6,0 Hz, 1H) ; 3,08 (dd, J=13,0 Hz, J=4,5 Hz, 1H) ; 4,53 (m, 1H).
¹³C-RMN (CDCl₃, 75 MHz) : (ppm) = 22,9; 28,7; 29,7; 35,8; 70,2; 177,9
UPLC-MS (AP-) : 225,3 (M-H⁺)

### Exemple C1 : Préparation du sel dicyclohexylammonium 2-hydroxy-3-(méthylséléno) propanoate (Composé 15)

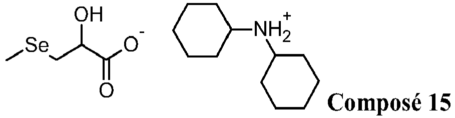

373 mg (2 mmoles) de l'acide 2-hydroxy-3-(méthylséléno)propanoïque sont solubilisés dans 2 ml d'acétone. 733 mg (805 µl; 4 mmoles; 2 éq.) de dicyclohexylamine sont ajoutés au milieu

Un précipité apparaît instantanément et est filtré, rincé avec de l'acétone (2x10 ml) puis avec un mélange acétate d'éthyle/cyclohexane 1/1 (2x10 ml).

On obtient 524 mg (70 %) du composé **15** sous forme d'un solide blanc.
¹H-RMN (CDCl₃, 400 MHz) : δ (ppm) = 1,26 (m, 7H) ; 1,50 (m, 4H) ; 1,68 (m, 2H) ; 1,84 (m, 4H) ; 2,06 (m, 4H) ; 2,12 (s, 3H) ; 2,91 (dd, J=12,5 Hz, J=6 Hz, 1H) ; 3,02 (m, 3H) ; 4,22 (m, 1H).
¹³C-RMN (CDCl₃, 75 MHz) : δ (ppm) = 5,7; 25,2; 25,5; 29,4; 31,6; 53,2; 72,2; 177,7

### Exemple C2 : Préparation du sel de sodium 2-hydroxy-3-(méthylséléno)propanoate (Composé 16)

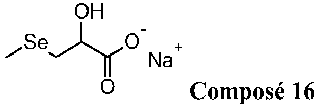

80 mg (2 mmoles) d'hydrure de sodium à 60 % dans l'huile minérale sont mis en suspension sous azote dans 2 ml de THF. 373 mg (2 mmoles) de l'acide 2-hydroxy-3-(méthylséléno)propanoïque solubilisés dans 2 ml de THF sont ajoutés goutte à goutte en 5 min.

Le milieu est refroidi dans un bain d'eau-glace puis du cyclohexane (3 ml) est ajouté. Un précipité se forme. Le milieu est filtré puis le solide est lavé avec du cyclohexane (3 ml) puis avec du TBME (3x3 ml).

On obtient 315 mg (75 %) du composé **16** sous forme d'un solide blanc cassé.
¹H-RMN (D₂O, 400 MHz) : δ (ppm) = 2,08 (s, 3H) ; 2,88 (dd, J=13,0 Hz, J=6,5 Hz, 1H) ; 2,99 (dd, J=13,0 Hz, J=4,0 Hz, 1H) ; 4,32 (dd, J=6,5 Hz, J=4,0 Hz, 1H).
¹³C-RMN (D₂O, 75 MHz) : δ (ppm) = 4,8; 30,0; 71,7; 179,4

UPLC-MS (AP-) : 182,7 (M-Na⁺)

### Exemple C3 : Préparation du sel de magnésium bis(2-hydroxy-3-(méthylséléno)propanoate (Composé 17)

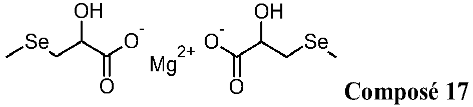

495 mg (2,65 mmoles) de l'acide 2-hydroxy-3-(méthylséléno)propanoïque sont solubilisés dans 1 ml d'eau déminéralisée puis 50 mg (1,25 mmoles) d'oxyde de magnésium sont ajoutés. Le milieu est agité à température ambiante pendant 16 h.

Le milieu est dilué avec de l'acétone (2 ml) puis le milieu est filtré sur verre fritté. Le solide est rincé avec de l'eau (0,5 mL) puis avec du TBME (2 ml). Le solide est séché sous vide.

On obtient 565 mg du composé **17** sous forme d'un solide blanc (quantitatif).
¹H-RMN (D₂O, 400 MHz) : δ (ppm) = 2,04 (s, 3H) ; 2,83 (dd, J=13,0 Hz, J=6,5 Hz, 1H) ; 2,95 (dd, J=13,0 Hz, J=4,0 Hz, 1H) ; 4,25 (dd, J=6,5 Hz, J=4,0 Hz, 1H).

### Exemple C4 : Préparation du sel du zinc bis(2-hydroxy-3-(méthylséléno)propanoate (Composé 18)

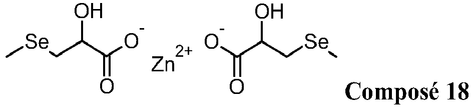

500 mg (2,65 mmoles) de l'acide 2-hydroxy-3-(méthylséléno)propanoïque sont solubilisés dans 1 ml d'eau déminéralisée puis 145 mg de carbonate de zinc basique sont ajoutés. 1 ml d'eau déminéralisée sont ajoutés puis le milieu est agité à température ambiante pendant 16 h.

Le milieu est dilué avec de l'acétone (2 ml) puis le milieu est filtré sur verre fritté. Le solide est rincé avec de l'eau (1 mL) puis avec de l'acétone (3x2 ml). Le solide est séché sous vide.

On obtient 496 mg (79 %) du composé **18** sous forme d'un solide blanc.
¹H-RMN (D₂O, 400 MHz) : δ (ppm) = 2,05 (s, 3H) ; 2,87 (dd, J=13,0 Hz, J=6,5 Hz, 1H) ; 2,98 (dd, J=13,0 Hz, J=4,0 Hz, 1H) ; 4,32 (dd, J=6,5 Hz, J=4,0 Hz, 1H).

### Exemple C5 : Préparation du sel du calcium bis(2-hydroxy-3-(méthylséléno)propanoate (Composé 19)

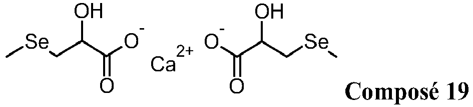

374 mg (2 mmoles) de l'acide 2-hydroxy-3-(méthylséléno)propanoïque sont solubilisés dans 0,9 ml d'eau déminéralisée puis 70 mg (945 µmoles) d'hydroxyde de calcium sont ajoutés. 1,1 ml d'eau déminéralisée sont ajoutés puis le milieu est agité à température ambiante pendant 16 h..

Le milieu est filtré sur verre fritté. Le solide est rincé avec de l'eau (2x2 mL) puis avec d'éther terbutylméthylique (TBME) (2x2 ml). Le solide est séché sous vide. On obtient 74 mg (19 %) du produit désiré sous forme d'un solide blanc cassé.

Le filtrat est concentré à sec. Le résidu est trituré avec du TBME (5 ml). Le solide est séché sous vide.

On obtient 205 mg (53 %) du composé **19** sous forme d'un solide blanc cassé. ¹H-RMN (D₂O, 400 MHz) : δ (ppm) = 2,07 (s, 3H) ; 2,88 (dd, J=13,0 Hz, J=6,5 Hz, 1H) ; 2,99 (dd, J=13,0 Hz, J=4,0 Hz, 1H) ; 4,30 (dd, J=6,5 Hz, J=4,0 Hz, 1H). ¹³C-RMN (D₂O, 300 MHz) : δ (ppm) = 4,8; 30,4; 72,1; 180,1

### 3- Préparation des composés D : Amides dérivés de l'acide 2-hydroxy-3-(alkylséléno)propanoique

Les composés D sont préparés soit par aminolyse des composés A, ou synthétisés à partir des composés B par couplage peptidique.

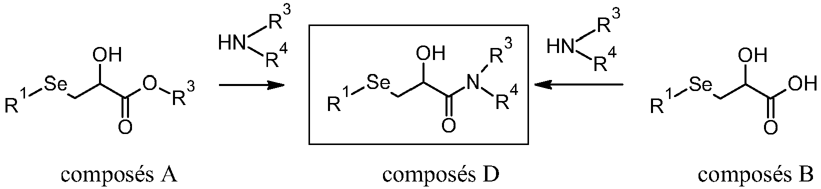

### Exemple D1: Préparation du 2-hydroxy-3-(méthylséléno)propanamide (Composé 20)

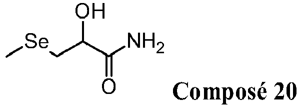

295 mg (1,5 mmoles) de composé **1** sont solubilisés dans 4,04 g (5,25 ml; 36,76 mmoles; 24 équiv.) d'une solution aqueuse d'ammoniaque à 20 %. Le milieu est agité à température ambiante pendant 22h.

Le milieu est évaporé à sec puis co-évaporé avec de l'acétate d'éthyle.

On obtient 284 mg (100 %) du composé **20** sous forme d'un solide blanc cassé.
¹H-RMN (MeOD, 400 MHz) : δ (ppm) = 2,11 (s, 3H) ; 2,85 (dd, J=13,0 Hz, J=7,0 Hz, 1H) ; 2,98 (dd, J=13,0 Hz, J=4,0 Hz, 1H) ; 4,3 (dd, J=7,0 Hz, J=4,0 Hz, 1H). ¹H-RMN (DMSO, 400 MHz) : δ (ppm) = 1,97 (s, 3H) ; 2,68 (dd, J=12,5 Hz, J=7,0 Hz, 1H) ; 2,80 (dd, J=12,5 Hz, J=4,0 Hz, 1H) ; 4,04 (m, 1H) ; 5,58 (d, J=5,5 Hz, 1H) ; 7,19 (d, J=15,5 Hz, 2H)
¹³C-RMN (MeOD, 75 MHz) : δ (ppm) = 5,2; 31,0; 73,3; 179,2
UPLC-MS (AP+) : 183,8 (M+H)⁺

UPLC-MS (AP+) : 206,0 (M+Na)⁺

### Exemple D2 : Préparation du N-cyclopropyl-2-hydroxy-3-(méthylséléno)propanamide (Composé 21)

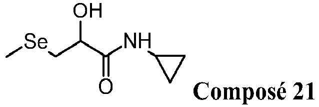

513 mg (2,5 mmoles) de composé **1** sont solubilisés dans 428 mg (519 µl; 7,5 mmoles; 3 équiv.) de cyclopropylamine. Le milieu est agité à 85°C pendant 72 h.

Le milieu est laissé revenir à température ambiante, évaporé à sec puis purifiée sur colonne de silice (dichlorométhane/méthanol).

On obtient 452 mg (79 %) du composé **21** sous forme d'un solide jaune pâle.
¹H-RMN (CDCl₃, 400 MHz) : δ (ppm) = 0,56 (m, 2H) ; 0,82 (m, 2H) ; 2,03 (s, 3H) ; 2,76 (m, 1H) ; 2,89 (dd, J=13,0 Hz, J=7,5 Hz, 1H) ; 3,08 (dd, J=13,0 Hz, J=4,5 Hz, 1H) ; 3,52 (d, J=3,5 Hz, 1H) ; 4,17 (m, 1H) ; 6,88 (s, 1H).
¹³C-RMN (CDCl₃, 75 MHz) : δ (ppm) = 4,9; 6,8; 6,9; 22,7; 31,6; 69,9; 173,8 UPLC-MS (AP+) : 245,9 (M+Na)⁺

### Exemple D3 : Préparation du N-[2-(diméthylamino)éthyl]-2-hydroxy-3-(méthylséléno)propanamide (Composé 22)

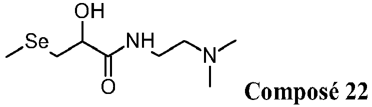

417 mg (2 mmoles) de composé **1** sont solubilisé dans 539 mg (674 µl; 6 mmoles; 3 équiv.) de N,N-diméthyléthylènediamine. Le milieu est agité à 85°C pendant 72 h. Le milieu est laissé revenir à température ambiante, évaporé à sec puis purifiée sur colonne de silice (dichlorométhane/méthanol).

On obtient 460 mg (89 %) du composé **22** sous forme d'une huile orange.
¹H-RMN (CDCl₃, 400 MHz) : δ (ppm) = 2,05 (s, 3H) ; 2,28 (s, 6H) ; 2,48 (t, J=6,0 Hz, 2H) ; 2,91 (dd, J=13,0 Hz, J=7,0 Hz, 1H) ; 3,06 (dd, J=13,0 Hz, J=4,0 Hz, 1H) ; 3,40 (m, 2H) ; 4,26 (dd, J=7,0 Hz, J=4,0 Hz, 1H) ; 7,34 (m, 1H).
¹³C-RMN (CDCl₃, 75 MHz) : δ (ppm) = 5,2; 31,6; 36,7; 45,6; 58,6; 71,0; 172,9
UPLC-MS (AP+) : 254,9 (M+H)⁺

### Exemple D4 : Préparation du 2-hydroxy-3-(méthylséléno)-1-(pyrrolidin-1-yl)propan-1-one (Composé 23)

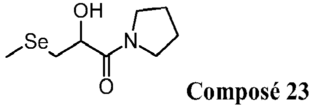

513 mg (2,5 mmoles) de composé **1** sont solubilisé dans 533 mg (616 µl; 7,5 mmoles; 3 équiv.) de pyrrolidine. Le milieu est agité à 85°C pendant 48 h.

Le milieu est laissé revenir à température ambiante, évaporé à sec puis purifiée sur colonne de silice (cyclohexane/acétate d'éthyle).

On obtient 402 mg (66 %) du composé **23** sous forme d'une huile orange.
¹H-RMN (CDCl₃, 400 MHz) : δ (ppm) = 1,85-2,07 (m, 4H) ; 2,11 (s, 3H) ; 2,77 (dd, J=13,0 Hz, J=7,0 Hz, 1H) ; 2,85 (dd, J=13,0 Hz, J=4,5 Hz, 1H) ; 3,45-3,65 (m, 4H) ; 3,78 (d, J=8,0 Hz, 1H) ; 4,45 (m, 1H).
¹³C-RMN (CDCl₃, 75 MHz) : δ (ppm) = 5,8; 24,3; 26,5; 29,7; 46,7; 46,8; 70,2; 171,6 UPLC-MS (AP+) : 237,9 (M+H)⁺
UPLC-MS (AP+) : 259,9 (M+Na)⁺

### Exemple D5 : Préparation du 2-hydroxy-3-(méthylséléno)-1-(pipéridin-1-yl)propan-1-one (Composé 24)

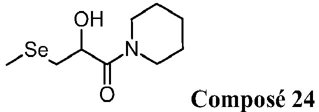

417 mg (2 mmoles) de composé **1** sont solubilisé dans 517 mg (600 µl; 6 mmoles; 3 équiv.) de pipéridine. Le milieu est agité à 85°C pendant 72 h.

Le milieu est laissé revenir à température ambiante, évaporé à sec puis purifiée sur colonne de silice (dichlorométhane/acétone).

On obtient 151 mg (29 %) du composé **24** sous forme d'une huile jaune.
¹H-RMN (CDCl₃, 400 MHz) : δ (ppm) = 1,56-1,74 (m, 6H) ; 2,13 (s, 3H) ; 2,72 (dd, J=13,0 Hz, J=7,0 Hz, 1H) ; 2,82 (dd, J=13,0 Hz, J=4,0 Hz, 1H) ; 3,40 (d, J=4,0 Hz, 2H) ; 3,62 (m, 2H) ; 4,03 (d, J=7,0 Hz, 1H) ; 4,61 (m, 1H).
¹³C-RMN (CDCl₃, 75 MHz) : δ (ppm) = 6,0; 24,8; 25,8; 26,6; 30,4; 44,2; 46,5; 68,7; 171,3
UPLC-MS (AP+) : 251,9 (M+H)⁺
UPLC-MS (AP+) : 273,9 (M+Na)⁺

### Exemple D6 : Préparation du 2-hydroxy-3-(méthylséléno)-1-(morpholin-4-yl)propan-1-one (Composé 25)

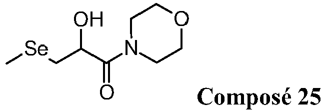

486 mg (2,4 mmoles) de composé 1 sont solubilisé dans 641 µl (7,2 mmoles; 3 équiv.) de morpholine. Le milieu est agité à 85°C pendant 72 h.

Le milieu est laissé revenir à température ambiante, évaporé à sec puis purifiée sur colonne de silice (dichlorométhane/acétone).

On obtient 262 mg (42 %) du composé **25** sous forme d'une huile jaune pâle.
¹H-RMN (CDCl₃, 400 MHz) : δ (ppm) = 2,12 (s, 3H) ; 2,79 (m, 2H) ; 3,52 (m, 2H) ; 3,65 (m, 1H) ; 3,74 (m, 5H) ; 3,82 (d, J=8,0 Hz, 1H) ; 4,59 (m, 1H).
¹³C-RMN (CDCl₃, 75 MHz) : δ (ppm) = 6,1; 30,1; 43,3; 46,2; 66,8; 67,1; 68,6; 171,8 UPLC-MS (AP+) : 253,9 (M+H)⁺
UPLC-MS (AP+) : 276,0 (M+Na)⁺

### Exemple D7 : Préparation du N,N-diéthyl-2-hydroxy-3-(méthylséléno)-propanamide (Composé 26)

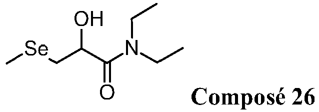

417 mg (2 mmoles) de composé **1** sont solubilisé dans 443 mg (633 µl;6 mmoles; 3 équiv.) de diéthylamine. Le milieu est agité à 85°C pendant 72 h.

Le milieu est laissé revenir à température ambiante, évaporé à sec puis purifiée sur colonne de silice (dichlorométhane/méthanol).

On obtient 105 mg (19 %) du composé **26** sous forme d'une huile orange.
¹H-RMN (CDCl₃, 400 MHz) : δ (ppm) = 1,38 (t, J=7,0 Hz, 6H) ; 2,10 (s, 3H) ; 2,91 (dd, J=12,5 Hz, J=6,0 Hz, 1H) ; 3,02 (m, 5H) ; 4,33 (m, 1H).

### Exemple D8 : Préparation du 2-hydroxy-N-(2-hydroxyéthyl)-3-(méthylséléno)-propanamide (Composé 27)

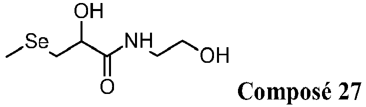

250 mg (1,22 mmoles) de composé **1** sont solubilisé dans 228 mg (225 µl; 3,66 mmoles; 3 équiv.) d'éthanolamine. Le milieu est agité à 85°C pendant 72 h.

Le milieu est laissé revenir à température ambiante, évaporé à sec puis purifiée sur colonne de silice (dichlorométhane/méthanol).

On obtient 203 mg (72 %) du composé **27** sous forme d'une huile verdâtre.
¹H-RMN (CDCl₃, 400 MHz) : δ (ppm) = 2,06 (s, 3H) ; 2,92 (dd, J=13,0 Hz, J=7,5 Hz, 1H) ; 2,95 (s, 1H) ; 3,11 (dd, J=13,0 Hz, J=4,0 Hz, 1H) ; 3,49 (m, 2H) ; 3,65 (d, J=4,5 Hz, 1H) ; 3,78 (m, 2H) ; 4,26 (m, 1H) ; 7,26 (s, 1H).
¹³C-RMN (CDCl₃, 75 MHz) : δ (ppm) = 5,2; 31,2; 42,5; 62,2; 70,6; 173,9 UPLC-MS (AP+) : 227,9 (M+H)⁺
UPLC-MS (AP+) : 249,8 (M+Na)⁺

### Exemple D9 : Préparation du [(2RS)-2-hydroxy-3-méthylsélénopropanoyl]-(S)-alanine (Composé 29)

### D9.1. Préparation de l'ester tert-butylique du [(2RS)-2-hydroxy-3-méthylsélénopropanoyl]-(S)-alanine (Composé 28)

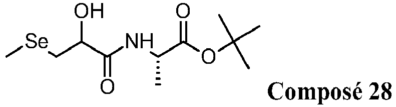

100 mg (546 µmoles) de l'acide 2-hydroxy-3-(méthylséléno)propanoïque **(10)** sont solubilisés sous azote dans 5 ml de dichlorométhane. Le milieu est refroidi à 0°C puis 183 mg (600 µmoles; 1,1 éq.) de 3-(diethoxyphosphoryloxy)-1,2,3-benzotriazin-4(3H)-one sont ajoutés. Le milieu est agité pendant 30 min à 0°C puis lh30 à température ambiante. Le milieu est refroidi à 0°C puis 99 mg (546 µmoles; 1 éq.) du chlorhydrate de l'ester *tert*-butylique de la (*S*)-alanine et 156 mg (200 µl; 1,20 mmoles; 2,2 éq.) de N,N-diisopropyléthylamine sont ajoutés. Le milieu est agité pendant 30 min à 0°C puis 16h à température ambiante.

Le milieu réactionnel est dilué avec du dichlorométhane (25 ml) puis le milieu est lavé avec une solution aqueuse d'acide chlorhydrique 1N (2x10 mL) puis avec une solution aqueuse de NaHCO₃ (1N, 2x10 mL) puis avec une solution aqueuse de NaCl saturée (10 mL). La phase organique est séchée sur Na₂SO₄, filtrée et concentrée. Le résidu est purifié sur colonne de silice (cyclohexane/acétate d'éthyle).

On obtient 116 mg (67 %) du composé **28** sous forme d'une huile incolore.
¹H-RMN (CDCl₃, 400 MHz) mélange 50/50 de 2 diastéréoisomères **28a** (*R*,S) et **28b** (*S*,*S*) : δ (ppm) = 1,42 et 1,43 (2d, J=3,5 Hz, 3H) ; 1,49 et 1,50 (2s, 9H) ; 2,04 et 2,05 (2s, 3H) ; 2,92 (dd, J=13,0 Hz, J=7,5 Hz, 1H) ; 3,09 (m, 1H) ; 3,43 (d, J=3,5 Hz, 1H) ; 4,21 (m, 1H) ; 4,48 (m, 1H) ; 7,27 et 7,33 (2d, J=6,5 Hz, 1H).
UPLC-MS (AP-) : 309,8 (M-H+)
UPLC-MS (AP+) : 333,9 (M+Na)⁺

### D9.2 : Préparation du [(2RS)-2-hydroxy-3-méthylsélénopropanoyl]-(S)-alanine (Composé 29)

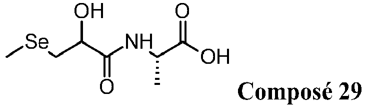

Le composé **29** est obtenu en utilisant les conditions de l'exemple B1 en partant de 58 mg de l'ester **28.**

On obtient 40 mg (81 %) du composé **29** sous forme d'une huile incolore.
¹H-RMN (CDCl₃, 400 MHz) mélange 50/50 de 2 diastéréoisomères **29a** (*R*,*S*) et **29b** (*S,S*) : δ (ppm) = 1,51 et 1,53 (2d, J=3,0 Hz, 3H) ; 2,05 et 2,06 (2s, 3H) ; 2,90 (m, 1H) ; 3,10 (m, 1H) ; 3,52 (s, 1H) ; 4,24 et 4,31 (2dd, J=8,0 Hz, J=4,0 Hz, 1H) ; 4,61 (m, 1H) ; 7,36 et 7,43 (2d, J=7,5 Hz, 1H).

### Exemple D10 : Préparation du [(2RS)-2-hydroxy-3-méthylsélénopropanoyl]-(S)-méthionine (Composé 31)

### D10.1. Préparation de l'ester méthylique du [(2RS)-2-hydroxy-3-méthylsélénopropanoyl]-(S)-méthionine (Composé 30)

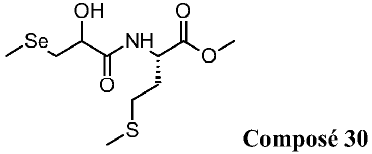

Le composé **30** est obtenu en utilisant les conditions de l'exemple D9 en partant de 500 mg (2,65 mmoles; 1 éq.) de l'acide 2-hydroxy-3-(méthylséléno)propanoïque **(10)** et 557 mg (2,65 mmoles; 1 éq.) de l'ester méthylique du chlorhydrate de la (*S*)-méthionine. On obtient 549 mg (60 %) du produit désiré sous forme d'une huile légèrement jaune.
¹H-RMN (CDCl₃, 400 MHz) mélange 50/50 de 2 diastéréoisomères **30a** *(R,S)* et **30b** (*S,S*) : δ (ppm) = 2,05 (m, 4H) ; 2,13 (m, 3H) ; 2,23 (m, 1H) ; 2,56 (t, J=7,5 Hz, 2H) ; 2,92 (m, 1H) ; 3,09 (m, 1H ) ; 3,45 (m, 1H) ; 3,79 et 3,80 (2s, 3H) ; 4,25 (m, 1H) ; 4,75 (m, 1H) ; 7,39 (m, 1H).

### D10.2. : Préparation du [(2RS)-2-hydroxy-3-méthylsélénopropanoyl]-(S)-méthionine (Composé 31)

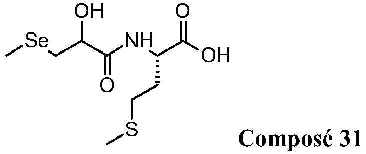

Le composé **31** est obtenu en utilisant les conditions de l'exemple B1 en partant de 445 mg du composé **30.**

Après 16 h d'agitation à température ambiante, le milieu réactionnel est dilué dans l'eau (40 ml) puis extrait à l'acétate d'éthyle (2x25 mL). Le pH du milieu est ajusté à 1 par l'ajout d'une solution aqueuse d'acide chlorhydrique 2 M. Le milieu est extrait à l'acétate d'éthyle (4x25 mL). Les phases organiques de cette deuxième extraction sont regroupées, séchées sur Na₂SO₄, filtrées et concentrées.

On obtient 400 mg (92 %) du produit désiré sous forme d'une huile jaune pâle.
¹H-RMN (CDCl₃, 400 MHz) mélange 50/50 de 2 diastéréoisomères **31a** *(R,S)* et **31b** (*S,S*) : δ (ppm) = 2,05 et 2,08 (2s, 3H) ; 2,11 (m, 1H) ; 2,15 (s, 3H) ; 2,27 (m, 1H) ; 2,62 (t, J=7,5 Hz, 2H) ; 2,91 (m, 1H) ; 3,10 (m, 1H) ; 4,28 et 4,38 (2dd, J=8,0 Hz, J=4,5 Hz, 1H) ; 4,75 (m, 1H) ; 7,51 et 7,57 (2d, J=8,0 Hz, 1H).

### Exemple D11 : Préparation de la [(2R)-2-hydroxy-3-méthylsélénopropanoyl]-(S)-méthionine (Composé 31a)

### D11.1 Préparation de l'ester méthylique de la [(2R)-2-hydroxy-3-méthylsélénopropanoyl]-(S)-méthionine (Composé 30a)

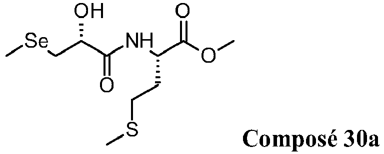

Le composé **30a** est obtenu en utilisant les conditions de l'exemple D9 en partant de 100 mg (513 µmoles; 1 éq.) de l'acide (*R*)-2-hydroxy-3-(méthylséléno)propanoïque **(11)** et 108 mg (513 µmoles; 1 éq.) de l'ester méthylique du chlorhydrate de la (*S*)-méthionine. On obtient 89 mg (50 %) du produit désiré sous forme d'une huile incolore.
¹H-RMN (CDCl₃, 400 MHz) : δ (ppm) = 2,06 (s, 3H) ; 2,07 (m, 1H) ; 2,13 (s, 3H) ; 2,22 (m, 1H) ; 2,57 (t, J=7,5 Hz, 2H) ; 2,93 (dd, J=13,0 Hz, J=7,5 Hz, 1H) ; 3,09 (dd, J=13,0 Hz, J=4,5 Hz, 1H) ; 3,40 (d, J=4,0 Hz, 1H) ; 3,80 (s, 3H) ; 4,28 (m, 1H) ; 4,75 (m, 1H) ; 7,40 (d, J=8,0 Hz, 1H).

### D11.2. Préparation de la [(2R)-2-hydroxy-3-méthylsélénopropanoyl]-(S)-méthionine (Composé 31a)

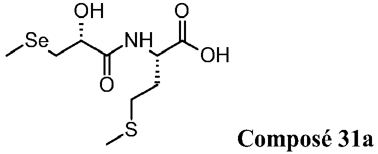

Le composé **31a** est obtenu en utilisant les conditions de l'exemple D10.2 en partant de 89 mg du composé **30a.**

On obtient 82 mg (99 %) du produit désiré sous forme d'une huile incolore.
¹H-RMN (CDCl₃, 400 MHz) : δ (ppm) = 2,08 (s, 3H) ; 2,11 (m, 1H) ; 2,15 (s, 3H) ; 2,28 (m, 1H) ; 2,63 (m, 2H) ; 2,91 (dd, J=13,0 Hz, J=7,0 Hz, 1H) ; 3,08 (dd, J=13,0 Hz, J=4,0 Hz, 1H) ; 4,39 (dd, J=7,0 Hz, J=4,0 Hz, 1H) ; 4,74 (m, 1H) ; 7,59 (d, J=8,0 Hz, 1H).

### Exemple D12 : Préparation de la [(2S)-2-hydroxy-3-méthylsélénopropanoyl]-(S)-méthionine (Composé 31b)

### D12.1. Préparation de l'ester méthylique de la [(2S)-2-hydroxy-3-méthylsélénopropanoyl]-(S)-méthionine (Composé 30b)

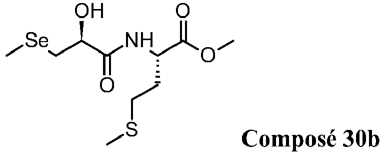

Le composé **30b** est obtenu en utilisant les conditions de l'exemple D9 en partant de 100 mg (535 µmoles; 1 éq.) de l'acide (*S*)-2-hydroxy-3-(méthylséléno)propanoïque **(12)** et 112 mg (535 µmoles; 1 éq.) de l'ester méthylique du chlorhydrate de la (*S*)-méthionine. On obtient 115 mg (57 %) du produit désiré sous forme d'une huile incolore.
¹H-RMN (CDCl₃, 400 MHz) : δ (ppm) = 2,04 (s, 3H) ; 2,07 (m, 1H) ; 2,13 (s, 3H) ; 2,23 (m, 1H) ; 2,57 (t, J=7,5 Hz, 2H) ; 2,92 (dd, J=13,0 Hz, J=8,0 Hz, 1H) ; 3,10 (dd, J=13,0 Hz, J=4,5 Hz, 1H) ; 3,42 (d, J=4,5 Hz, 1H) ; 3,79 (s, 3H) ; 4,22 (m, 1H) ; 4,75 (m, 1H) ; 7,35 (d, J=8,0 Hz, 1H).

### D12.2. Préparation de la [(2S)-2-hydroxy-3-méthylsélénopropanoyl]-(S)-méthionine (Composé 31b)

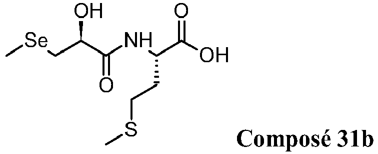

Le composé **31b** est obtenu en utilisant les conditions de l'exemple D10.2 en partant de 115 mg du composé **30b.**

On obtient 98 mg (98 %) du produit désiré sous forme d'une huile jaune.
¹H-RMN (CDCl₃, 400 MHz) : δ (ppm) = 2,05 (s, 3H) ; 2,10 (m, 1H) ; 2,15 (s, 3H) ; 2,28 (m, 1H) ; 2,62 (t, J=7,5 Hz, 2H) ; 2,91 (dd, J=13,0 Hz, J=8,0 Hz, 1H) ; 3,11 (dd, J=13,5 Hz, J=4,5 Hz, 1H) ; 4,27 (dd, J=8,0 Hz, J=4,5 Hz, 1H) ; 4,75 (m, 1H) ; 7,48 (d, J=8,0 Hz, 1H).

### Exemple D13 : Préparation de l'ester méthylique de la N(α)-[(2RS)-2-hydroxy-3-méthylsélénopropanoyl]-N(ω)-tert-butoxycarbonyl-(S)-lysine (Composé 32)

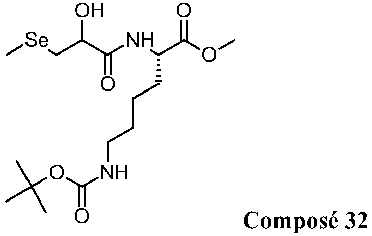

Le composé **32** est obtenu en utilisant les conditions de l'exemple D9 en partant de 100 mg (535 µmoles; 1 éq.) de l'acide 2-hydroxy-3-(méthylséléno)propanoïque **(10)** et 162 mg (546 µmoles; 1 éq.) de l'ester méthylique du chlorhydrate de la N(ω)*-tert-*butoxycarbonyl-(*S*)-lysine.

On obtient 171 mg (73 %) du produit désiré sous forme d'une huile incolore.
¹H-RMN (CDCl₃, 400 MHz) mélange 50/50 de 2 diastéréoisomères (*R,S*) et (*S,S*) : δ (ppm) = 1,37 (m, 2H) ; 1,44 (s, 9H) ; 1,48 (m, 2H) ; 1,73 (m, 1H) ; 1,89 (m, 1H) ; 2,02 et 2,04 (2s, 3H) ; 2,89 (m, 1H) ; 3,07 (m, 3H) ; 3,74 et 3,75 (s, 3H) ; 3,85 (m, 1H) ; 4,26 (m, 1H) ; 4,59 (m, 1H) ; 4,69 (m, 1H) ; 7,30 (m, 1H).
UPLC-MS (AP+) : 449,0 (M+Na)⁺

### Exemple D14 : Préparation de l'ester méthylique de la N(α)-[(2RS)-2-hydroxy-3-méthylsélénopropanoyl]-N(ω)-fluorenylméthyloxycarbonyl-(S)-lysine (Composé 33)

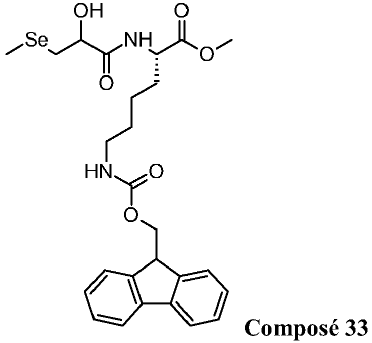

Le composé **33** est obtenu en utilisant les conditions de l'exemple D9 en partant de 200 mg (1,059 mmoles; 1 éq.) de l'acide 2-hydroxy-3-(méthylséléno)propanoïque **(10)** et 448 mg (1,059 mmoles; 1 éq.) de l'ester méthylique du chlorhydrate de la N(ω)-fluorenylméthyloxycarbonyl-(*S*)-lysine.

On obtient 430 mg (59 %) du produit désiré sous forme d'une huile incolore.
¹H-RMN (CDCl₃, 400 MHz) : δ (ppm) = 1,42 (m, 2H) ; 1,58 (m, 2H) ; 1,76 (m, 2H) ; 1,91 (m, 1H) ; 2,03 (s, 3H) ; 2,92 (m, 1H) ; 3,10 (m, 1H) ; 3,21 (m, 2H) ; 3,78 (s, 3H) ; 4,25 (m, 2H) ; 4,43 (m, 2H) ; 4,64 (m, 1H) ; 4,90 (m, 1H) ; 7,25 (m, 1H) ; 7,35 (m, 2H) ; 7,42 (m, 2H) ; 7,63 (m, 2H) ; 7,80 (m, 2H).
UPLC-MS (AP+) : 571,3 (M+Na)⁺

### Exemple D15 : Préparation de l'ester benzylique de la N(α)-[(2RS)-2-hydroxy-3-méthylsélénopropanoyl]-N(ω)-benzyloxycarbonyl-(S)-lysine (Composé 34)

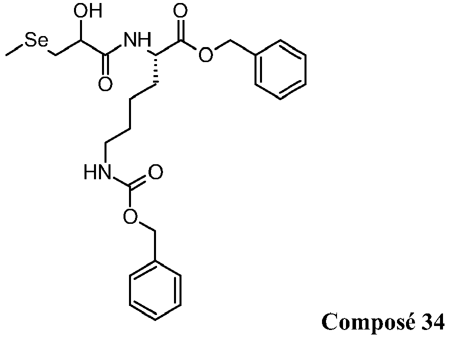

Le composé **34** est obtenu en utilisant les conditions de l'exemple D9 en partant de 400 mg (2,12 mmoles; 1 éq.) de l'acide 2-hydroxy-3-(méthylséléno)propanoïque (**10**) et 862 mg (2,12 mmoles; 1 éq.) de l'ester benzylique du chlorhydrate de la N(ω)-benzyloxycarbonyl-(*S*)-lysine.

On obtient 944 mg (76 %) du produit désiré sous forme d'une huile jaune.
¹H-RMN (CDCl₃, 400 MHz) mélange 50/50 de 2 diastéréoisomères **34a** (*R,S*) et **34b** (*S,S*) : δ (ppm) = 1,35 (m, 2H) ; 1,51 (m, 2H) ; 1,75 (m, 2H) ; 1,91 (m, 1H) ; 2,00 et 2,03 (2s, 3H) ; 2,89 (m, 1H) ; 3,07 (m, 1H) ; 3,17 (m, 2H) ; 3,37 et 3,49 (2s, 1H) ; 4,23 (m, 1H) ; 4,67 (m, 1H) ; 4,81 (m, 1H) ; 5,12-5,26 (m, 4H) ; 7,33-7,43 (m, 10H).

### Exemple D16 : Préparation de la [(2RS)-2-hydroxy-3-méthylsélénopropanoyl]-(S)-(Composé 36)

### D16.1. Préparation de l'ester méthylique de la [(2RS)-2-hydroxy-3-méthylsélénopropanoyl]-(S)-sélénométhionine (Composé 35)

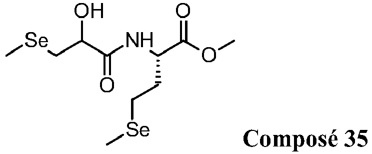

Le composé 35 est obtenu en utilisant les conditions de l'exemple D9 en partant de 136 mg (720 µmoles; 1 éq.) de l'acide 2-hydroxy-3-(méthylséléno)propanoïque **(10)** et 187 mg (720 µmoles; 1 éq.) de l'ester méthylique du chlorhydrate de la (*S*)-sélénométhionine.

On obtient 180 mg (63 %) du produit désiré sous forme d'une huile incolore. ¹H-RMN (CDCl₃, 400 MHz) mélange 50/50 de 2 diastéréoisomères **35a** (*R,S*) et **35b** (*S,S*) : δ (ppm) = 2,03 (s, 3H) ; 2,04 et 2,07 (2s, 3H) ; 2,13 (m, 1H) ; 2,28 (m, 1H) ; 2,57 (t, J=7,5 Hz, 2H) ; 2,92 (m, 1H) ; 3,10 (m, 1H) ; 3,38 (m, 1H) ; 3,79 et 3,80 (2s, 3H) ; 4,25 (m, 1H) ; 4,75 (m, 1H) ; 7,33 et 7,37 (2d, J=8,5 Hz, 1H).

### D16.2. Préparation de la [(2RS)-2-hydroxy-3-méthylsélénopropanoyl]-(S)-sélénométhionine (Composé 36)

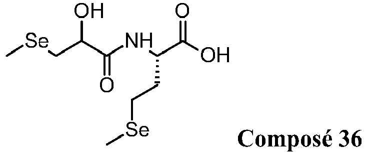

Le composé 36 est obtenu en utilisant les conditions de l'exemple B1 en partant de 130 mg du composé **35.**

Après 16 h d'agitation à température ambiante, le milieu réactionnel est dilué dans l'eau (20 ml) puis extrait à l'acétate d'éthyle (2x10 mL). Le pH du milieu est ajusté à 1 par l'ajout d'une solution aqueuse d'acide chlorhydrique 2 M. Le milieu est extrait à l'acétate d'éthyle (3x10 mL). Les phases organiques de cette deuxième extraction sont regroupées, séchées sur Na₂SO₄, filtrées et concentrées.

On obtient 112 mg (89 %) du produit désiré sous forme d'une huile jaune.
¹H-RMN (CDCl₃, 400 MHz) mélange 50/50 de 2 diastéréoisomères **36a** (*R,S*) et **36b** (*S,S*): δ (ppm) = 2,05 (s, 3H) ; 2,08 (s, 3H) ; 2,17 (m, 1H) ; 2,33 (m, 1H) ; 2,63 (m, 2H) ; 2,92 (m, 1H) ; 3,10 (m, 1H) ; 4,26 et 4,35 (2dd, J=8,0 Hz, J=4,5 Hz, 1H) ; 4,75 (m, 1H) ; 7,43 et 7,49 (2d, J=8,0 Hz, 1H).

### Exemple D17 : Préparation de la [(2R)-2-hydroxy-3-méthylsélénopropanoyl]-(S)-sélénométhionine (Composé 36a)

### D17.1. Préparation de l'ester méthylique de la [(2R)-2-hydroxy-3-méthylsélénopropanoyl]-(S)-sélénométhionine (Composé 35a)

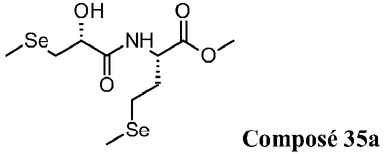

Le composé **35a** est obtenu en utilisant les conditions de l'exemple D9 en partant de 185 mg (950 µmoles; 1 éq.) de l'acide (*R*)-2-hydroxy-3-(méthylséléno)propanoïque **(11)** et 260 mg (950 µmoles; 1 éq.) de l'ester méthylique du chlorhydrate de la (*S*)-sélénométhionine.

On obtient 233 mg (62 %) du produit désiré sous forme d'une huile jaune pâle.
¹H-RMN (CDCl₃, 400 MHz) : δ (ppm) = 2,03 (s, 3H) ; 2,07 (s, 3H) ; 2,12 (m, 1H) ; 2,29 (m, 1H) ; 2,57 (m, 2H) ; 2,93 (dd, J=13,0 Hz, J=7,5 Hz, 1H) ; 3,09 (dd, J=13,0 Hz, J=4,5 Hz, 1H) ; 3,32 (m, 1H) ; 3,80 (s, 3H) ; 4,27 (m, 1H) ; 4,75 (m, 1H) ; 7,37 (d, J=8,0 Hz, 1H).

### D17.2. Préparation de la [(2R)-2-hydroxy-3-méthylsélénopropanoyl]-(S)-sélénométhionine (Composé 36a)

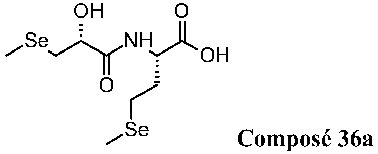

Le composé **36a** est obtenu en utilisant les conditions de l'exemple B1 en partant de 183 mg du composé **35a.**

Le milieu réactionnel est dilué dans l'eau (20 ml) puis extrait à l'acétate d'éthyle (2x10 mL). Le pH du milieu est ajusté à 1 par l'ajout d'une solution aqueuse d'acide chlorhydrique 2 M. Le milieu est extrait à l'acétate d'éthyle (3x10 mL). Les phases organiques sont regroupées, séchées sur Na₂SO₄, filtrées et concentrées.

On obtient 178 mg (100 %) du produit désiré sous forme d'un solide crème.
¹H-RMN (CDCl₃, 400 MHz) : δ (ppm) = 2,05 (s, 3H) ; 2,08 (s, 3H) ; 2,18 (m, 1H) ; 2,35 (m, 1H) ; 2,64 (m, 2H) ; 2,91 (dd, J=13,0 Hz, J=7,0 Hz, 1H) ; 3,09 (dd, J=13,0 Hz, J=4,0 Hz, 1H) ; 4,38 (dd, J=7,0 Hz, J=4,0 Hz, 1H) ; 4,74 (m, 1H) ; 7,53 (d, J=8,0 Hz, 1H).

### Exemple D18 : Préparation de l'ester méthylique de la [(2S)-2-hydroxy-3-méthylsélénopropanoyl]-(S)-sélénométhionine (Composé 36b)

### D18.1. Préparation de l'ester méthylique de la [(2S)-2-hydroxy-3-méthylsélénopropanoyl]-(S)-sélénométhionine (Composé 35b)

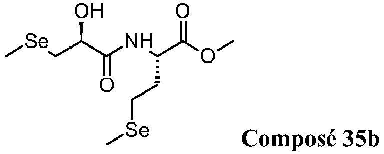

Le composé **35b** est obtenu en utilisant les conditions de l'exemple D9 en partant de 177 mg (949 µmoles; 1 éq.) de l'acide (*S*)-2-hydroxy-3-(méthylséléno)propanoïque **(12)** et 260 mg (950 µmoles; 1 éq.) de l'ester méthylique du chlorhydrate de la (*S*)-sélénométhionine.

On obtient 236 mg (58 %) du produit désiré sous forme d'une huile jaune pâle.
¹H-RMN (CDCl₃, 400 MHz) : δ (ppm) = 2,03 (s, 3H) ; 2,04 (s, 3H) ; 2,12 (m, 1H) ; 2,29 (m, 1H) ; 2,57 (t, J=7,5 Hz, 2H) ; 2,92 (dd, J=13,0 Hz, J=8,0 Hz, 1H) ; 3,10 (dd, J=13,0 Hz, J=4,5 Hz, 1H) ; 3,79 (s, 3H) ; 4,22 (dd, J=8,0 Hz, J=4,5 Hz, 1H) ; 4,75 (m, 1H) ; 7,33 (d, J=8,5 Hz, 1H).

### D18.2. Préparation de la [(2S)-2-hydroxy-3-méthylsélénopropanoyl]-(S)-sélénométhionine (Composé 36b)

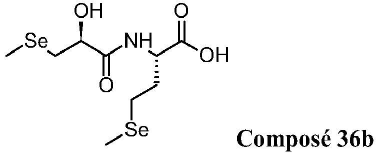

Le composé 36b est obtenu en utilisant les conditions de l'exemple B1 en partant de 186 mg du composé **35b.**

Après 16 h d'agitation à température ambiante, le milieu réactionnel est dilué dans l'eau (20 ml) puis extrait à l'acétate d'éthyle (2x10 mL). Le pH du milieu est ajusté à 1 par l'ajout d'une solution aqueuse d'acide chlorhydrique 2 M. Le milieu est extrait à l'acétate d'éthyle (3x10 mL). Les phases organiques sont regroupées, séchées sur Na₂SO₄, filtrées et concentrées. Le résidu est purifié sur colonne de silice (cyclohexane/acétate d'éthyle puis dichlorométhane/méthanol). L'huile obtenue est triturée avec du cyclohexane puis du pentane, solubilisée dans TBME et dichlorométhane puis concentré à sec.

On obtient 90 mg (50 %) du produit désiré sous forme d'une huile jaune.
¹H-RMN (CDCl₃, 400 MHz) : δ (ppm) = 2,05 (s, 6H) ; 2,19 (m, 1H) ; 2,34 (m, 1H) ; 2,63 (m, 2H) ; 2,91 (dd, J=13,5 Hz, J=8,0 Hz, 1H) ; 3,12 (dd, J=13,5 Hz, J=4,5 Hz, 1H) ; 4,26 (dd, J=8,0 Hz, J=4,5 Hz, 1H) ; 4,75 (m, 1H) ; 7,41 (d, J=8,0 Hz, 1H).

### 4- Préparation des composés E : les acides 2-acyloxy-3-(alkylséléno)propanoique et esters correspondants

Les composés E sont préparés soit en une étape par réaction des composés B avec des anhydrides carboxyliques, soit en deux étapes à partir de composés A par réactions avec des anhydrides carboxyliques suivi par hydrolyse.

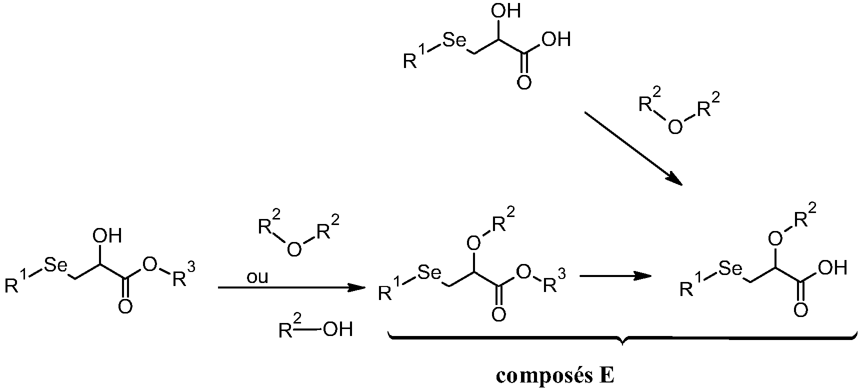

### Exemple E1 : Préparation de l'acide 2-(acétyloxy)-3-(méthylséléno)propanoïque (Composé 37)

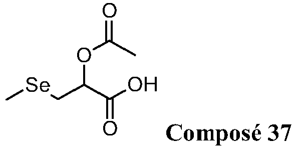

374 mg (2 mmoles) de l'acide 2-hydroxy-3-(méthylséléno)propanoïque **(10)** sont solubilisés sous azote dans 33 ml de dichlorométhane. 817 mg (756 µl; 8 mmoles; **4** éq.) d'anhydride acétique puis 2,5 mg (20 µmoles; 0,01 éq.) de 4-diméthylaminopyridine sont ajoutés au milieu. Le milieu est laissé sous agitation sous azote et à température ambiante pendant 6 h. 817 mg (756 µl; 8 mmoles; 4 éq.) d'anhydride acétique sont de nouveau ajoutés au milieu. Le milieu est laissé sous agitation sous azote et à température ambiante pendant 16 h. 10 mL d'eau sont additionnés puis le dichlorométhane du milieu est éliminé par évaporation. 40 mL d'une solution aqueuse de NH₄Cl saturée sont additionnés puis le milieu est extrait par acétate d'éthyle (3x40 mL). Les phases organiques sont regroupées, séchées avec Na₂SO₄, filtrées et concentrées. L'huile obtenue est purifiée sur colonne de silice (cyclohexane/acétate d'éthyle avec 1% TFA). On obtient 292 mg (63 %) du composé **37** sous forme d'une huile jaune.
¹H-RMN (CDCl₃, 400 MHz) : δ (ppm) = 2,15 (s, 3H) ; 2,20 (s, 3H) ; 2,97 (dd, J=13,5 Hz, J=7,5 Hz, 1H) ; 3,04 (dd, J=13,5 Hz, J=4,5 Hz, 1H) ; 5,37 (dd, J=7,5 Hz, J=4,5 Hz, 1H) ; 9,90 (s, 1H).
¹³C-RMN (CDCl₃, 75 MHz) : δ (ppm) = 6,2; 21,0; 25,0; 72,6; 170,7
UPLC-MS (AP-) : 224,7 (M-H⁺)

### Exemple E2 : Préparation de l'acide 2-(dodécanoyloxy)-3-(méthylséléno)propanoïque (Composé 38)

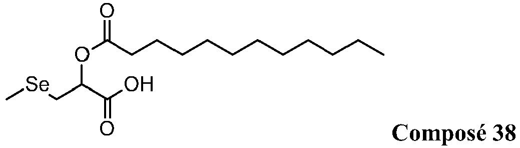

400 mg (2,14 mmoles) de l'acide 2-hydroxy-3-(méthylséléno)propanoïque **(10)** sont solubilisés sous azote dans 35 ml de dichlorométhane. 3,344 g (8,56 mmoles; 4 éq.) de l'anhydride laurique puis 2,6 mg (21 µmoles; 0,01 éq.) de 4-diméthylaminopyridine sont ajoutés au milieu. Le milieu est laissé sous agitation sous azote et à température ambiant pendant 6 h.

3,344 g (8,56 mmoles; 4 éq.) de l'anhydride laurique sont ajoutés de nouveau au milieu. Le milieu est laissé sous agitation sous azote et à température ambiant pendant 16 h.

10 mL d'eau sont additionnés puis le milieu est concentré à sec. Le résidu est purifié sur colonne de silice (cyclohexane/acétate d'éthyle puis dichlorométhane/méthanol). On obtient 394 mg (47 %) du composé **38** sous forme d'une huile jaune.
¹H-RMN (CDCl₃, 400 MHz) : δ (ppm) = 0,91 (m, 3H) ; 1,29 (m, 16H) ; 1,68 (m, 2H) ; 2,13 (s, 3H) ; 2,44 (t, J=7,5 Hz, 2H) ; 3,00 (m, 2H) ; 5,30 (m, 1H).

### Exemple E3 : Préparation de l'acide 2-(benzoyloxy)-3-(méthylséléno)-propanoïque (Composé 40)

### E3.1: Préparation de l'ester méthylique de l'acide 2-(benzoyloxy)-3-(méthylséléno)propanoïque (Composé 39)

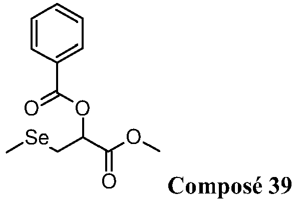

500 mg (2,46 mmoles) de composé **1** sont solubilisé sous azote dans 40 ml de dichlorométhane. 1,136 g (4,92 mmoles; 2 éq.) de l'anhydride benzoïque puis 30 mg (246 µmoles; 0,1 éq.) de 4-diméthylaminopyridine sont ajoutés au milieu. Le milieu est laissé sous agitation sous azote et à température ambiant pendant 25 h.

Le milieu est concentré à sec. Le résidu est purifié sur colonne de silice (cyclohexane/acétate d'éthyle). On obtient 633 mg (81 %) du composé 39 sous forme d'une huile incolore.
¹H-RMN (CDCl₃, 400 MHz) : δ (ppm) = 2,17 (s, 3H) ; 3,13 (m, 2H) ; 3,83 (s, 3H) ; 5,60 (t, J=6,0 Hz, 1H) ; 7,50 (t, J=7,5 Hz, 2H) ; 7,63 (t, J=7,5 Hz, 1H) ; 8,13 (d, J=7,5 Hz, 2H).

### E3.2: Préparation de l'acide 2-(benzoyloxy)-3-(méthylséléno)propanoïque (Composé 40)

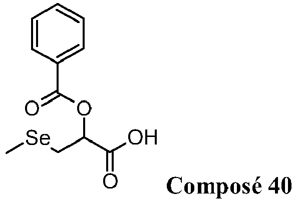

Le composé **40** est obtenu en utilisant les conditions de l'exemple B1 en partant du 372 mg du composé **39.**

Après 16 h d'agitation à température ambiante, le milieu réactionnel est dilué dans l'eau (10 ml) puis est extrait à l'acétate d'éthyle (2x10 mL). Le pH du milieu est ajusté à 1 par l'ajout d'une solution aqueuse d'acide chlorhydrique 2 M. Le milieu est extrait à l'acétate d'éthyle (3x10 mL). Les phases organiques de cette deuxième extraction sont regroupées, séchées sur Na₂SO₄, filtrées et concentrées.

On obtient 283 mg (81 %) du produit désiré sous forme d'un solide blanc.
¹H-RMN (CDCl₃, 400 MHz) : δ (ppm) = 2,13 (s, 3H) ; 2,99 (dd, J=13,5 Hz, J=6,0 Hz, 1H) ; 3,09 (dd, J=13,5 Hz, J=4,5 Hz, 1H) ; 4,58 (dd, J=6,0 Hz, J=4,5 Hz, 1H) ; 7,52 (t, J=7,5 Hz, 2H) ; 7,66 (t, J=7,5 Hz, 1H) ; 8,15 (d, J=7,5 Hz, 2H).

### Exemple E4: Préparation de l'ester méthylique de l'acide 3-(méthylséléno)-2-[(3'-pyridine)oxycarbonyl]propanoïque (Composé 41)

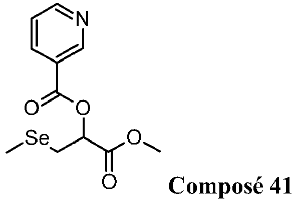

384 mg (1,85 mmoles) de composé 1 sont solubilisé sous azote dans 30 ml de dichlorométhane. 872 mg (3,7 mmoles; 2 éq.) de l'anhydride 3-pyridinecarboxylique puis 23 mg (185 µmoles; 0,1 éq.) de 4-diméthylaminopyridine sont ajoutés au milieu. Le milieu est laissé sous agitation sous azote et à température ambiant pendant 18 h. Le milieu est filtré sur verre fritté puis le filtrat est concentré à sec. Le résidu est purifié sur colonne de silice (cyclohexane/acétate d'éthyle).

On obtient 490 mg (83 %) du composé **41** sous forme d'une huile incolore.
¹H-RMN (CDCl₃, 400 MHz) : δ (ppm) = 2,16 (s, 3H) ; 3,13 (m, 2H) ; 3,84 (s, 3H) ; 5,60 (dd, J=7,0 Hz, J=5,0 Hz, 1H) ; 7,47 (dd, J=8,0 Hz, J=5,0 Hz, 1H) ; 8,39 (d, J=8,0 Hz, 1H) ; 8,85 (d, J=5,0 Hz, 1H) ; 9,31 (s, 1H).

### Exemple E5: Préparation de l'acide 2-[(tert-butoxycarbonyl)oxy]-3-(méthylséléno)propanoïque (Composé 43)

### E5.1 : Préparation de l'ester méthylique de l'acide 2-[(tert-butoxycarbonyl)oxy]-3-(méthylséléno)propanoïque (Composé 42)

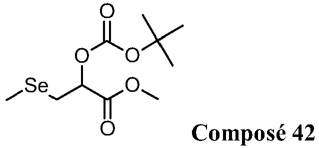

100 mg (507 µmoles) de composé **1** sont mis en solution dans 5 ml d'acétate d'éthyle. Le milieu est refroidi à 10°C puis 103 mg (143 µl; 1,01 mmoles; 2 éq.) de triéthylamine sont ajoutés goutte à goutte. 120 mg (533 µmoles; 1,05 éq.) de *di-tert-*butyl dicarbonate en solution dans 1 ml d'acétate d'éthyle sont ajoutés goutte à goutte rapidement. Le milieu est chauffé à 90°C pendant 48h.

Le milieu réactionnel est dilué avec de l'acétate d'éthyle (25 ml) puis le milieu est lavé avec une solution aqueuse d'acide citrique à 5 % (2x10 mL) puis avec une solution aqueuse saturée en NaCl (10 mL). La phase organique est séchée sur Na₂SO₄, filtrée et concentrée. Le résidu est purifié sur colonne de silice (cyclohexane/acétate d'éthyle).

On obtient 101 mg (63 %) du composé **42** sous forme d'une huile incolore.
¹H-RMN (CDCl₃, 400 MHz) : δ (ppm) = 1,55 (s, 9H) ; 2,13 (s, 3H) ; 2,94 (dd, J=13,5 Hz, J=7,5 Hz, 1H) ; 3,00 (dd, J=13,5 Hz, J=5,0 Hz, 1H) ; 3,82 (s, 3H) ; 5,17 (dd, J=7,5 Hz, J=5,0 Hz, 1H).

### E5.2: Préparation de l'acide 2-[(tert-butoxycarbonyl)oxy]-3-(méthylséléno)propanoïque (Composé 43)

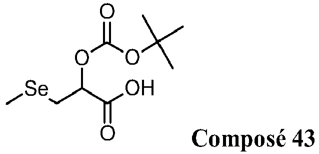

Le composé **43** est obtenu en utilisant les conditions de l'exemple B1 en partant de 363 mg du composé **42.**

Après 16 h d'agitation à température ambiante, le milieu réactionnel est dilué dans l'eau (25 ml) puis est extrait à l'acétate d'éthyle (2x20 mL). Le pH du milieu est ajusté à 4 par l'ajout d'une solution aqueuse d'acide citrique à 5 % (2x10 mL). Le milieu est extrait à l'acétate d'éthyle (5x25 mL). Les phases organiques de cette deuxième extraction sont regroupées, séchées sur Na₂SO₄, filtrées et concentrées.

Le résidu est purifié sur colonne de silice (dichlorométhane/méthanol) puis repurifié sur colonne de silice (cyclohexane/acétate d'éthyle).

On obtient 39 mg (11 %) du produit désiré sous forme d'une huile incolore.
¹H-RMN (CDCl₃, 400 MHz) : δ (ppm) = 1,52 (s, 9H) ; 2,14 (s, 3H) ; 2,96 (dd, J=13,5 Hz, J=7,5 Hz, 1H) ; 3,03 (dd, J=13,5 Hz, J=4,5 Hz, 1H) ; 5,20 (dd, J=7,5 Hz, J=4,5 Hz, 1H) ; 10,04 (s, 1H).

### Exemple E6: Préparation de l'ester méthylique de l'acide (2RS)-[N-(tert-butoxycarbonyl)-S-methionyl]-3-méthylséléno-propanoïque (Composé 44)

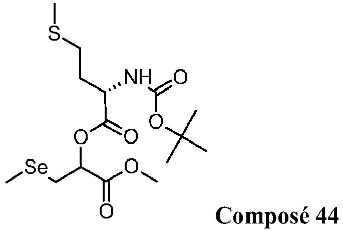

500 mg (2,0 mmoles) de la BOC-(*S*)-méthionine sont mis en solution dans 25 ml de dichlorométhane. 417 mg (2,0 mmoles; 1 éq.) de N,N'-dicyclohexylcarbodiimide, sont ajoutés. Le milieu est agité pendant 10 min à température ambiante puis 416 mg (2,0 mmoles; 1 éq.) de composé **1** et 25 mg (200 µmoles; 0,1 éq.) de 4-Diméthylaminopyridine sont ajoutés. Le milieu est agité à température ambiante pendant 16 h.

Le milieu est concentré à sec. Le résidu est purifié sur colonne de silice (cyclohexane/acétate d'éthyle).

On obtient 709 mg (78 %) du composé **44** sous forme d'une huile incolore.
¹H-RMN (CDCl₃, 400 MHz) mélange 50/50 de 2 diastéréoisomères **44a** (*R,S*) et **44b** (*S,S*) : δ (ppm) = 1,48 (s, 9H) ; 2,04 (m, 1H) ; 2,12 et 2,14 (2s, 3H) ; 2,15 et 2,16 (2s, 3H) ; 2,26 (m, 1H) ; 2,65 (m, 2H) ; 2,99 (m, 2H) ; 3,80 et 3,81 (2s, 3H) ; 4,58 (m, 1H) ; 5,17 (m, 1H) ; 5,39 (m, 1H).
UPLC-MS (AP+) : 451,9 (M+Na)⁺

### Exemple E7 : Préparation de l'ester méthylique de l'acide 4-méthylséléno-2-(2'-acétyloxy-3'-méthylsélénopropanoyl)butyrique (Composé 45) et de l'acide 4-méthylséléno-2-(2'-acétyloxy-3'-méthylsélénopropanoyl)butyrique (Composé 46)

### E7.1. Préparation de l'ester méthylique de l'acide 4-méthylséléno-2-(2'-acétyloxy-3'-méthylsélénopropanoyl)butyrique (Composé 45)

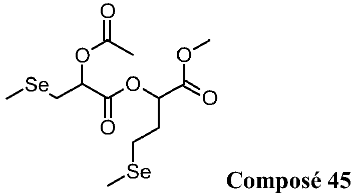

128 mg (522 µmoles) de l'acide 2-(acétyloxy)-3-(méthylséléno)propanoïque (37) sont solubilisé sous azote dans 5 ml de dichlorométhane. 108,7 mg (522 µmoles) de N,N'-Dicyclohexylcarbodiimide sont ajoutés au milieu. Le milieu est laissé sous agitation sous azote et à température ambiant pendant 10 min. 116 mg (522 µmoles) de méthyle 2-hydroxy-4-(méthylséléno)butanoate (EP1778706*,* préparé en analogie avec l'exemple 9 en utilisant méthanol au lieu d'éthanol) en solution dans 5 ml de dichlorométhane puis 6,4 mg (52 µmoles; 0,01 éq.) de 4-diméthylaminopyridine sont ajoutés. Le milieu est agité à température ambiante pendant 16 h.

Le milieu est concentré à sec. Le résidu est purifié sur colonne de silice (cyclohexane/acétate d'éthyle).

On obtient 189 mg (77 %) du composé **45** sous forme d'une huile incolore.
¹H-RMN (CDCl₃, 400 MHz) mélange de 2 diastéréoisomères : δ (ppm) = 2,03 et 2,04 (2s, 3H) ; 2,15 et 2,16 (2s, 3H) ; 2,20 (s, 3H) ; 2,23-2,3 (m, 2H) ; 2,54-2,7 (m, 2H) ; 2,95-3,02 (m, 1H) ; 3,11 (dd, J=13,5 Hz, J=4,0 Hz, 1H) ; 3,79 et 3,80 (2s, 3H) ; 5,15 à 5,45 (m, 2H).
UPLC-MS (AP+) : 442,9 (M+Na)⁺

### E7.2. Préparation de l'acide 4-méthylséléno-2-(2'-acétyloxy-3'-méthylsélénopropanoyl)butyrique (Composé 46) par hydrolyse de l'ester de méthyle

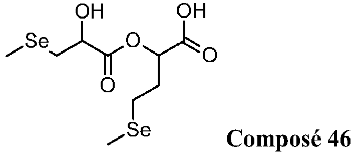

Le composé **46** est obtenu en utilisant les conditions de l'exemple B1 en partant de 180 mg du composé **45.**

Après 16 h d'agitation à température ambiante, le milieu réactionnel est dilué dans l'eau (20 ml) puis extrait à l'acétate d'éthyle (2x5 mL). La phase aqueuse est lyophilisée. Le lyophilisat est repris avec une solution à 4 N d'acide chlorhydrique dans le dioxane. Le milieu est agité 10 min puis concentré à sec. Le concentrât est repris dans 10 mL d'eau puis la solution est lyophilisée.

On obtient 138 mg (71 %) du produit désiré contenant 2 LiCl sous forme d'un solide collant jaune.
¹H-RMN (D₂O, 400 MHz) : δ (ppm) = 2,00 (s, 3H) ; 2,05 (s, 3H) ; 2,07-2,24 (m, 2H) ; 2,65 (m, 2H) ; 2,89 (dd, J=13,0 Hz, J=6,5 Hz, 1H) ; 3,00 (dd, J=13,0 Hz, J=4,5 Hz, 1H) ; 4,36 (dd, J=8,0 Hz, J=4,0 Hz, 1H) ; 4,50 (dd, J=6,5 Hz, J=4,5 Hz, 1H).

### Exemple E8 : Préparation de l'acide 3-méthylséléno-2-(2'-hydroxy-4'-méthylsélénobutanoyl)propanoïque (Composé 48)

### E8.1: Préparation de l'ester méthylique de l'acide 3-méthylséléno-2-(2'-acétyloxy-4'-méthylsélénobutanoyl)propanoïque (Composé 47)

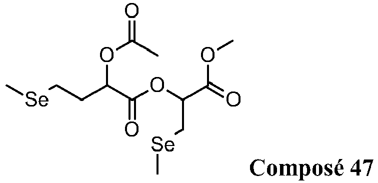

Le composé **47** est obtenu en utilisant les conditions de l'exemple E7 en partant de 89 mg de l'acide 2-acétyloxy-méthylsélénobutyrique (EP1778706*,* exemple 11) et 72 mg du composé **1.**

Le milieu est filtré sur verre fritté puis le filtrat est concentré à sec. Le résidu est purifié sur colonne de silice (cyclohexane/acétate d'éthyle).

On obtient 97 mg (60 %) du produit désiré sous forme d'une huile incolore.
¹H-RMN (CDCl₃, 400 MHz) mélange des diastéréoisomères : δ (ppm) = 2,05 et 2,06 (2s, 3H) ; 2,11 et 2,14 (2s, 3H) ; 2,18 et 2,19 (2s, 3H) ; 2,27-2,37 (m, 2H) ; 2,60-2,80 (m, 2H) ; 2,92-3,07 (m, 2H) ; 3,80 et 3,81 (2s, 3H) ; 5,24 (dd, J=12,5 Hz, J=6,0 Hz, 1H) ; 5,34 et 5,45 (2dd, J=8,0 Hz, J=4,5 Hz, 1H).
UPLC-MS (AP+) : 443,0 (M+Na)⁺

### E8.2 : Préparation de l'acide 3-méthylséléno-2-(2'-hydroxy-4'-méthylsélénobutanoyl)-propanoïque (Composé 48)

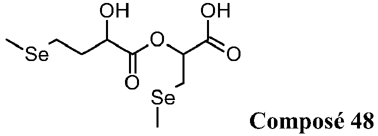

Le composé 48 est obtenu en utilisant les conditions de l'exemple E7.2 en partant de 79 mg du composé **47.**

On obtient 69 mg (84 %) du produit désiré contenant 2 LiCl sous forme d'un solide collant incolore.
¹H-RMN (D₂O, 400 MHz) : δ (ppm) = 2,04 (m, 3H) ; 2,09-2,14 (m, 3H) ; 2,20-2,27 (m, 2H) ; 2,64-2,74 (m, 2H) ; 2,93 (dd, J=13,0 Hz, J=6,5 Hz, 1H) ; 3,04 (dd, J=13,5 Hz, J=4,5 Hz, 1H) ; 4,55 (dd, J=6,5 Hz, J=4,5 Hz, 1H), 5,05 (m, 1H).

### Exemple E9 : Préparation de l'acide 2-(pentanoyloxy)-3-(méthylséléno)propanoïque (Composé 49)

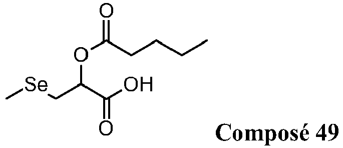

407 mg (1,98 mmoles) de l'acide 2-hydroxy-3-(méthylséléno)propanoïque **(10)** sont solubilisés sous azote dans 33 ml de dichlorométhane. 752 mg (817 µl; 3,96 mmoles; 2 éq.) d'anhydride valérique puis 2,4 mg (19,8 µmoles; 0,01 éq.) de 4-diméthylaminopyridine sont ajoutés au milieu. Le milieu est laissé sous agitation sous azote et à température ambiante pendant 24 h.

752 mg (817 µl; 3,96 mmoles; 2 éq.) d'anhydride valérique puis 2,4 mg (19,8 µmoles; 0,01 éq.) de 4-diméthylaminopyridine sont de nouveau ajoutés au milieu. Le milieu est laissé sous agitation sous azote et à température ambiante pendant 48 h. 10 mL d'eau sont additionnés puis le dichlorométhane du milieu est éliminé par évaporation. 25 mL d'une solution aqueuse de NH₄Cl saturée sont additionnés puis le milieu est extrait par acétate d'éthyle (3x10 mL). Les phases organiques sont regroupées, séchées avec Na₂SO₄, filtrées et concentrées. L'huile obtenue est purifiée sur colonne de silice (cyclohexane/acétate d'éthyle). On obtient 260 mg (48 %) du composé **49** sous forme d'une huile incolore.
¹H-RMN (CDCl₃, 400 MHz) : δ (ppm) = 0,96 (t, J=7,4 Hz, 3H) ; 1,42 (m, 2H) ; 1,69 (m, 2H) ; 2,15 (s, 3H) ; 2,46 (td, J=1,9 Hz, J=7,4 Hz, 2H) ; 3,01 (m, 2H) ; 5,36 (dd, J=4,3 Hz, J=7,6 Hz, 1H).
UPLC-MS (AP-) : 267,3 (M-H⁺)

### Exemple E10 : Préparation de l'acide 2-(nonanoyloxy)-3-(méthylséléno)propanoïque (Composé 50)

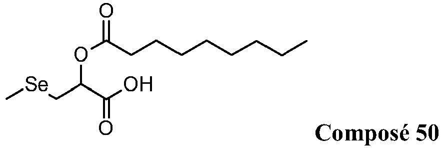

260 mg (1,39 mmoles) de l'acide 2-hydroxy-3-(méthylséléno)propanoïque (10) sont solubilisés sous azote dans 23 ml de dichlorométhane. 874 mg (963 µl; 2,78 mmoles; 2 éq.) d'anhydride nonanoique puis 1,7 mg (13,9 µmoles; 0,01 éq.) de 4-diméthylaminopyridine sont ajoutés au milieu. Le milieu est laissé sous agitation sous azote et à température ambiante pendant 24 h.

874 mg (963 µl; 2,78 mmoles; 2 éq.) d'anhydride nonanoique puis 1,7 mg (13,9 µmoles; 0,01 éq.) de 4-diméthylaminopyridine sont ajoutés au milieu. Le milieu est laissé sous agitation sous azote et à température ambiante pendant 24 h.

10 mL d'eau sont additionnés puis le dichlorométhane du milieu est éliminé par évaporation. 25 mL d'une solution aqueuse de NH₄Cl saturée sont additionnés puis le milieu est extrait par acétate d'éthyle (3x10 mL). Les phases organiques sont regroupées, séchées avec Na₂SO₄, filtrées et concentrées. L'huile obtenue est purifiée sur colonne de silice (cyclohexane/acétate d'éthyle). On obtient 116 mg (25 %) du composé **50** sous forme d'une huile incolore.
¹H-RMN (CDCl₃, 400 MHz) : δ (ppm) = 0,91 (t, J=6,9 Hz, 3H) ; 1,34 (m, 10H) ; 1,70 (m, 2H) ; 2,14 (s, 3H) ; 2,45 (td, J=1,7 Hz, J=7,4 Hz, 2H) ; 3,01 (m, 2H) ; 5,37 (dd, J=4,3 Hz, J=7,6 Hz, 1H).
UPLC-MS (AP-) : 322,9 (M-H⁺)

### Exemple E11.1. : Préparation de l'ester méthylique de l'acide 2-(linoleoyloxy)-3-(méthylséléno)propanoïque (Composé 51)

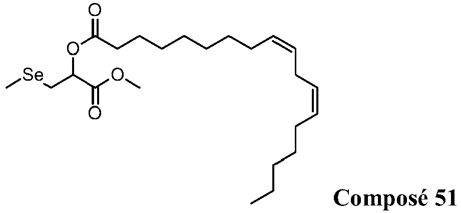

818 mg (2,83 mmoles) d'acide linoleique sont mis en solution dans 35 mL de dichlorométhane. 589 mg (2,83 mmoles; 1 éq.) de N,N'-dicyclohexylcarbodiimide, sont ajoutés. Le milieu est agité pendant 10 min à température ambiante puis 575 mg (2,83 mmoles; 1 éq.) de composé **1** et 35 mg (283 µmoles; 0,1 éq.) de 4-diméthylaminopyridine sont ajoutés. Le milieu est agité à température ambiante pendant 48 h.

Le milieu est filtré sur verre fritté puis le filtrat est concentré à sec. Le résidu est purifié sur colonne de silice (cyclohexane/acétate d'éthyle). On obtient 801 mg (60%) du composé **51** sous forme d'une huile incolore.
¹H-RMN (CDCl₃, 400 MHz) : δ (ppm) = 0,92 (t, J=6,9 Hz, 3H) ; 1,34 (m, 14H) ; 1,70 (m, 2H) ; 2,08 (m, 4H) ; 2,12 (s, 3H) ; 2,45 (td, J=2,8 Hz, J=7,4 Hz, 2H) ; 2,8 (t, J=6,6 Hz, 2H) ; 2,97 (m, 2H) ; 3,8 (s, 3H) ; 5,38 (m, 5H).
UPLC-MS (AP+) : 461,2 (M+H⁺)

### Exemple E11.2 : Préparation de l'acide 2-(linoleoyloxy)-3-(méthylséléno)propanoïque (Composé 52)

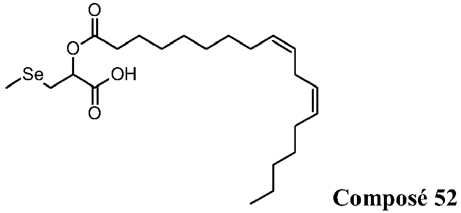

459 mg (1,59 mmoles) d'acide linoleique sont mis en solution dans 19,8 mL de dichlorométhane. 331 mg (2,83 mmoles; 1 éq.) de N,N'-dicyclohexylcarbodiimide, sont ajoutés. Le milieu est agité pendant 30 min à température ambiante puis 300 mg (1,59 mmoles; 1 éq.) d'acide 2-hydroxy-3-(méthylséléno)propanoïque et 20 mg (159 µmoles; 0,1 éq.) de 4-Diméthylaminopyridine sont ajoutés. Le milieu est agité à température ambiante pendant 24 h.

Le milieu est filtré sur verre fritté puis le filtrat est concentré à sec. Le résidu est purifié sur colonne de silice (cyclohexane/acétate d'éthyle puis dichlorométhane/méthanol). On obtient 198 mg (26 %) du composé **52** sous forme d'une huile incolore.
¹H-RMN (CDCl₃, 300 MHz) : δ (ppm) = 0,93 (t, J=6,8 Hz, 3H) ; 1,35 (m, 14H) ; 1,70 (m, 2H) ; 2,1 (m, 4H) ; 2,15 (s, 3H) ; 2,45 (m, 2H) ; 2,8 (t, J=5,9 Hz, 2H) ; 3,0 (m, 2H) ; 5,4 (m, 5H).
UPLC-MS (AP-) : 444,7 (M-H⁺)

### Exemple E12 : Préparation de l'ester méthylique de l'acide 2-[(1H-imidazol-4-ylcarbonyl)oxy]-3-(méthylséléno)propanoïque (Composé 53)

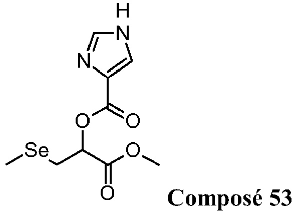

438 mg (3,83 mmoles) d'acide 4-imidazolecarboxylique sont mis en suspension dans 20 mL de dichlorométhane et 30 µl de N,N-diméthylformamide (383 µmoles; 0,1 éq.). Le milieu est refroidie à 0°C puis 503 µL (5,74 mmoles; 1,5 éq.) de chlorure d'oxalyle sont ajoutés. Le milieu est refroidi à 0°C pendant 15 min puis agité 2 h à température ambiante. Le milieu est concentré à sec.

Le concentrât est repris dans 20 ml de dichlorométhane. Le milieu est refroidie à 0°C puis 1,26 mL (7,66 mmoles; 2 éq.) de N,N-diisopropylethylamine sont ajoutés. 794 mg (3,83 mmoles; 1 éq.) de composé 1 sont ajoutés goutte à goutte. Le milieu est agité à température ambiante pendant 48 h.

Le milieu est dilué avec 50 mL d'acétate d'éthyle puis lavé avec 20 mL d'eau. La phase aqueuse est extraite avec 20 mL d' acétate d'éthyle. Les phases organiques sont regroupées, lavées avec 20 mL d'une solution aqueuse de NaCl saturée. La phase organique est séchée avec Na₂SO₄, filtrée et concentrée. Le résidu est purifié sur colonne de silice (cyclohexane/acétate d'éthyl puis dichlorométhane/méthanol). On obtient 806 mg (70 %) du composé **53** sous forme d'un solide jaune pâle.
¹H-RMN (DMSO, 400 MHz) : δ (ppm) = 2,12 (s, 3H) ; 3,03 (m, 2H) ; 3,70 (s, 3H) ; 5,42 (dd, J=4,6 Hz, J=7,3 Hz, 1H) ; 7,84 (s, 1H) ; 7,88 (s, 1H) ; 12,74 (sl, 1H).
UPLC-MS (AP+) : 314,7 (M+Na⁺)

### Exemple E13.1 : Préparation de l'ester méthylique de l'acide 2-(pivaloyloxy)-3-(méthylséléno)propanoïque (Composé 54)

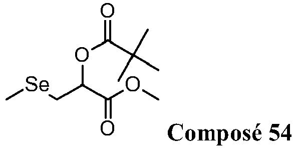

400 mg (1,99 mmoles) de composé **1** sont solubilisés sous azote dans 50 mL de dichlorométhane. 1,5 g (7,95 mmoles; 4 éq.) d'anhydride triméthylacétique puis 24 mg (199 µmoles; 0,1 éq.) de 4-diméthylaminopyridine sont ajoutés au milieu. Le milieu est laissé sous agitation sous azote et à température ambiante pendant 48 h.

748 mg (3,98 mmoles; 2 éq.) d'anhydride triméthylacetique sont de nouveau ajoutés au milieu. Le milieu est laissé sous agitation sous azote et à température ambiante pendant 96 h.

Le milieu est concentré à sec. Le résidu est purifié sur colonne de silice (cyclohexane/acétate d'éthyle). On obtient 484 mg (83 %) du composé 54 sous forme d'une huile incolore.
¹H-RMN (CDCl₃, 400 MHz) : δ (ppm) = 1,29 (s, 9H) ; 2,13 (s, 3H) ; 2,99 (m, 2H) ; 3,79 (s, 3H) ; 5,31 (dd, J=4,6 Hz, J=7,5 Hz, 1H).
UPLC-MS (AP+) : 281,9 (M+H⁺)

### Exemple E13.2 : Préparation de l'acide 2-(pivaloyloxy)-3-(méthylséléno)propanoïque (Composé 55)

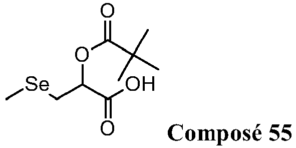

240 mg (0,83 mmoles) de composé **54** sont solubilisés dans 12 mL de THF. 830 µl (0,83 mmoles; 1 équiv.) d'une solution aqueuse d'hydroxyde de lithium à 1M sont ajoutés, la solution est agitée à température ambiante pendant 16 h. 41 µL (41µmoles; 0,05 équiv.) d'une solution aqueuse d'hydroxyde de lithium à 1M sont ajoutés, la solution est agitée à température ambiante pendant 24 h.

Le milieu réactionnel est dilué dans l'eau (20 mL) puis est extrait à l'acétate d'éthyle (3x20 mL). Le pH du milieu est ajusté à 4 par l'ajout d'une solution aqueuse d'acide chlorhydrique à 1 M. Le milieu est extrait à l'acétate d'éthyle (3x20 mL). Les phases organiques de cette deuxième extraction sont regroupées, séchées sur Na₂SO₄, filtrées et concentrées. On obtient 151 mg (66 %) du produit désiré **55** sous forme d'une huile incolore.
¹H-RMN (CDCl₃, 400 MHz) : δ (ppm) = 1,29 (s, 9H) ; 2,15 (s, 3H) ; 3,03 (m, 2H) ; 5,32 (dd, J=7,7 Hz, J=4,3 Hz, 1H).
UPLC-MS (AP-) : 266,6 (M-H⁺)

### Exemple E14 : Préparation de l'ester méthylique de l'acide 2-(3-chloropropanoyloxy)-3-(méthylséléno)propanoïque (Composé 56)

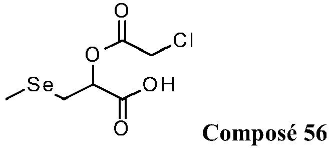

400 mg (1,99 mmoles) de composé **1** sont mis en suspension sous azote dans 6 mL d'acétone. 324 mg (3,98 mmoles; 2 éq.) de pyridine sont ajoutés au milieu. Le milieu est refroidie à - 10°C puis 390 µl (3,98 mmoles; 2 éq.) de 3-chloropropionyl chloride sont ajoutés au milieu à - 10°C. Le milieu est laissé sous agitation et à température ambiante pendant 64 h.

Le milieu réactionnel est dilué dans l'eau (20 mL) puis est extrait à l'acétate d'éthyle (2x20 mL). Les phases organiques sont regroupées, lavées avec 2x20 mL d'une solution aqueuse de NaHCO₃ à 1M et 10 mL d'une solution aqueuse de NaCl saturée. La phase organique est séchée avec Na₂SO₄, filtrée et concentrée. Le résidu est purifié sur colonne de silice (cyclohexane/acétate d'éthyl). On obtient 301 mg (51 %) du composé **56** sous forme d'une huile incolore.
¹H-RMN (CDCl₃, 400 MHz) : δ (ppm) = 2,13 (s, 3H) ; 2,97 (m, 4H) ; 3,81 (s, 3H) ; 3,82 (t, J=6,6 Hz, 2H) ; 5,4 (dd, J=4,6 Hz, J=7,5 Hz, 1H).

**Tableau récapitulatif des exemples de préparation des nouveaux composés**

| **Ex**. | **Composé** | **Structure** | **R1** | **R2** | **X** |
|---|---|---|---|---|---|
| **A1** | **1** | 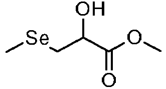 | CH₃ | H | OCH₃ |
| **A2** | **1** | 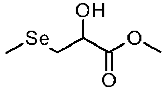 | CH₃ | H | OCH₃ |
| **A3** | **2** | 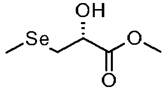 | CH₃ | H | OCH₃ |
| **A4** | **3** | 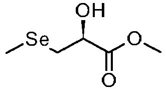 | CH₃ | H | OCH₃ |
| **A5** | **4** | 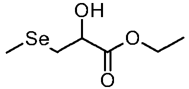 | CH₃ | H | OCH₂CH₃ |
| **A6** | **5** | 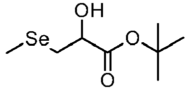 | CH₃ | H | OC(CH₃)₃ |
| **A7** | **6** | 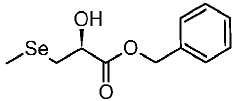 | CH₃ | H | OCH₂C₆H₅ |
| **A8** | **7** | 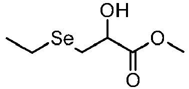 | CH₂CH₃ | H | OCH₃ |
| **A9** | **8** | 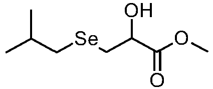 | CH₂CH(CH₃)₂ | H | OCH₃ |
| **A10** | **4** | 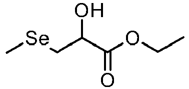 | CH₃ | H | OCH₂CH₃ |
| **A11** | **9** | 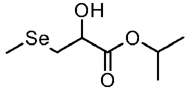 | CH₃ | H | OCH(CH₃)₂ |
| **B1** | **10** | 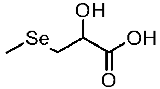 | CH₃ | H | OH |
| **B2** | **10** | 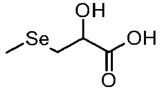 | CH₃ | H | OH |
| **B3** | **11** | 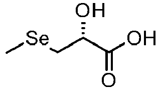 | CH₃ | H | OH |
| **B4** | **12** | 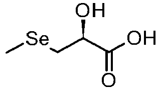 | CH₃ | H | OH |
| **B5** | **12** | 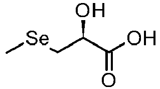 | CH₃ | H | OH |
| **B6** | **13** | 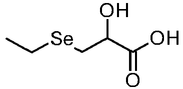 | CH₂CH₃ | H | OH |
| **B7** | **14** | 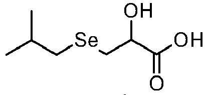 | CH₂CH(CH₃)₂ | H | OH |
| **C1** | **15** | 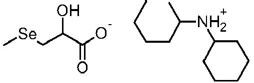 | CH₃ | H | ONH₂(C₆H₁₁)₂ |
| **C2** | **16** | 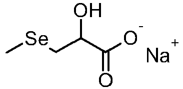 | CH₃ | H | Sel de Na |
| **C3** | **17** | 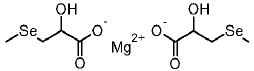 | CH₃ | H | Sel de Mg |
| **C4** | **18** | 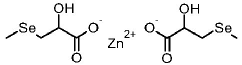 | CH₃ | H | Sel de Zn |
| **C5** | **19** | 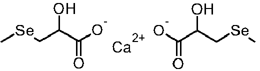 | CH₃ | H | Sel de Ca |
| **D1** | **20** | 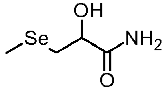 | CH₃ | H | NH₂ |
| **D2** | **21** | 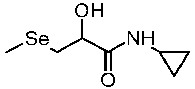 | CH₃ | H | NHC₃H₅ |
| **D3** | **22** | 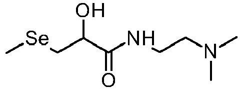 | CH₃ | H | NH(CH₂)₂N(CH₃) ₂ |
| **D4** | **23** | 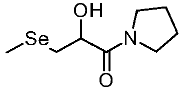 | CH₃ | H | N(CH₂)₄ |
| **D5** | **24** | 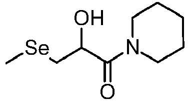 | CH₃ | H | N(CH₂)₅ |
| **D6** | **25** | 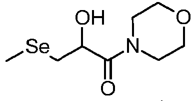 | CH₃ | H | N(CH₂)₄O |
| **D7** | **26** | 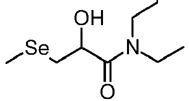 | CH₃ | H | N(C₂H₅)₂ |
| **D8** | **27** | 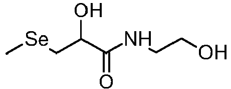 | CH₃ | H | NH(CH₂)₂OH |
| **D9.1** | **28** | 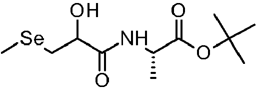 | CH₃ | H | NHAlaOC(CH₃)₃ |
| **D9.2** | **29** | 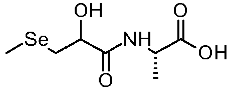 | CH₃ | H | NHAlaOH |
| **D10.1** | **30** | 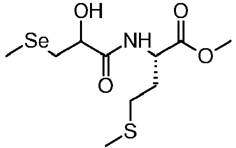 | CH₃ | H | NHMetOCH₃ |
| **D10.2** | **31** | 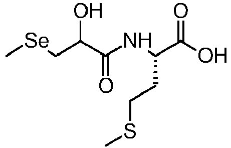 | CH₃ | H | NHMetOH |
| **D11.1** | **30a** | 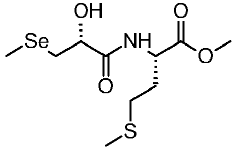 | CH₃ | H | NHMetOCH₃ |
| **D11.2** | **31a** | 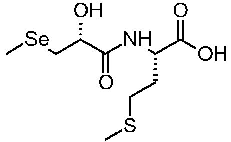 | CH₃ | H | NHMetOH |
| **D12.1** | **30b** | 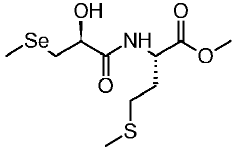 | CH₃ | H | NHMetOCH₃ |
| **D12.2** | **31b** | 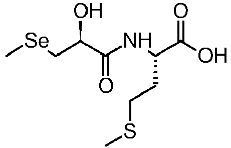 | CH₃ | H | NHMetOH |
| **D13** | **32** | 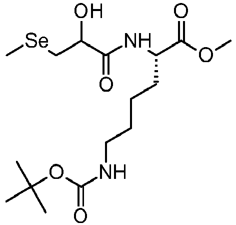 | CH₃ | H | NHLys(NHBoc)O CH₃ |
| **D14** | **33** | 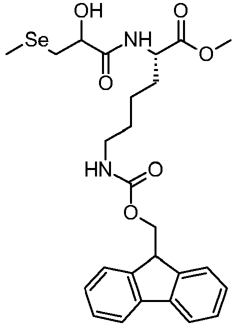 | CH₃ | H | NHLys(NHFmoc) OCH₃ |
| **D15** | **34** | 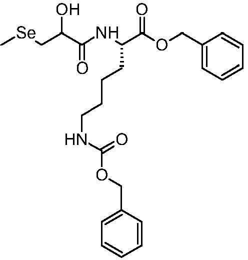 | CH₃ | H | NHLys(NHCbz)O CH₂C₆H₅ |
| **D16.1** | **35** | 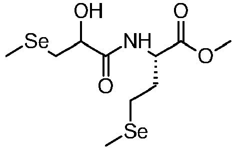 | CH₃ | H | NHSeMetOCH₃ |
| **D16.2** | **36** | 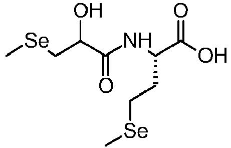 | CH₃ | H | NHSeMetOH |
| **D17.1** | **35a** | 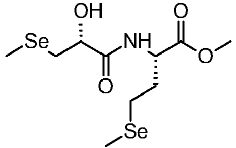 | CH₃ | H | NHSeMetOCH₃ |
| **D17.2** | **36a** | 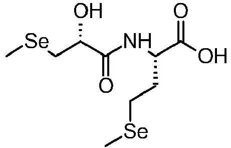 | CH₃ | H | NHSeMetOH |
| **D18.1** | **35b** | 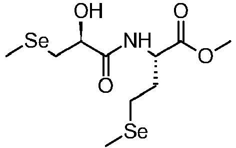 | CH₃ | H | NHSeMetOCH₃ |
| **D18.2** | **36b** | 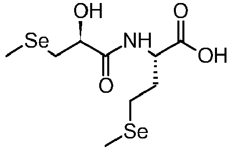 | CH₃ | H | NHSeMetOH |
| **E1** | **37** | 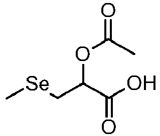 | CH₃ | C(=O)CH₃ | OH |
| **E2** | **38** | 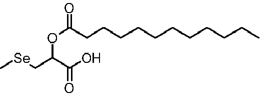 | CH₃ | C(=O)(CH₂)₁₀₋CH₃ | OH |
| **E3.1** | **39** | 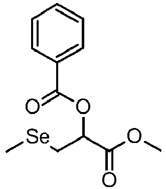 | CH₃ | C(=O)C₆H₅ | OCH₃ |
| **E3.2** | **40** | 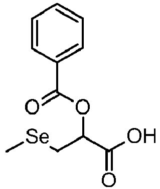 | CH₃ | C(=O)C₆H₅ | OH |
| **E4** | **41** | 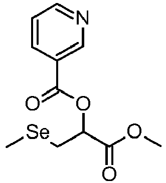 | CH₃ | C(=O)C₅H₄N | OCH₃ |
| **E5.1** | **42** | 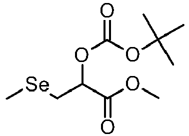 | CH₃ | Q=O)OC(CH₃)₃ | OCH₃ |
| **E5.2** | **43** | 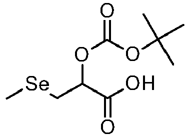 | CH₃ | C(=O)OC(CH₃)₃ | OH |
| **E6** | **44** | 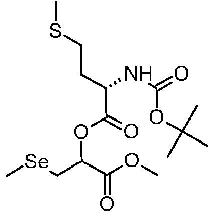 | CH₃ | MetNHBoc | OCH₃ |
| **E7.1** | **45** | 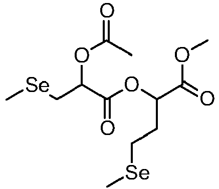 | CH₃ | C(=O)CH₃ | OSeMetOCH₃ |
| **E7.2** | **46** | 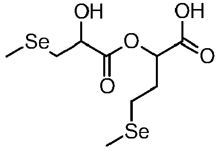 | CH₃ | H | OSeMetOH |
| **E8.1** | **47** | 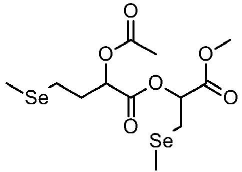 | CH₃ | C(=O)CH₃ | OSeMetOCH₃ |
| **E8.2** | **48** | 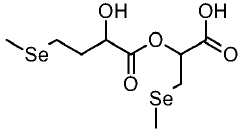 | CH₃ | H | OSeMetOH |
| **E9** | **49** | 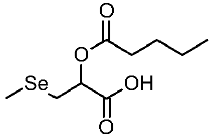 | CH₃ | C(=O)C₄H₉ | OH |
| **E10** | **50** | 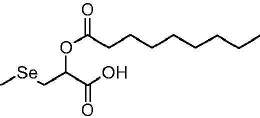 | CH₃ | C(=O)C₄H₉ | OH |
| **E11.1** | **51** | 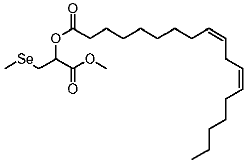 | CH₃ | C(=O)C₁₇H₃₁ | OCH₃ |
| **E11.2** | **52** | 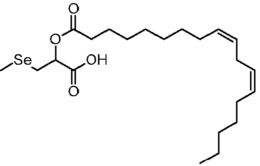 | CH₃ | C(=O)C₁₇H₃₁ | OH |
| **E12** | **53** | 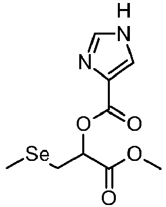 | CH₃ | C(=O)C₃H₃N₂ | OCH₃ |
| **E13.1** | **54** | 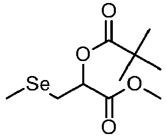 | CH₃ | C(=O)C(CH₃)₃ | OCH₃ |
| **E13.2** | **55** | 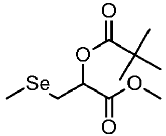 | CH₃ | C(=O)C(CH₃)₃ | OH |
| **E14** | **56** | 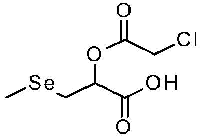 | CH₃ | C(=O)(CH₂)₂Cl | OCH₃ |

### II. Exemples décrivant l'activité anti-tumorale des composés selon l'invention

### II.1. Lignées cellulaires

Huit lignées cellulaires issues de différents types de cancers ont été utilsées: PC3 et DU145 (prostate), HT-29 et LS-174T (colon), Hep G2 (foie), MCF-7 (sein), MIA PaCa-2 et PANC-1 (pancréas).

Les caractéristiques de chaque lignée cellulaire sont résumées dans le **Tableau I.**

**Tableau I :Caractéristiques des lignées cellulaires utilisées**

| **Organe** | **Lignée cellulaire** | **Description** |
|---|---|---|
| Prostate | PC3 | Adhérente, adénocarcinome prostate humain grade IV dérivée de métastase osseuse |
| | DU145 | Adhérente, carcinome prostate humain |
| Colon | HT-29 | Adhérente, adénocarcinome humain (homme 44 ans Caucasien) |
| | LS174T | Adhérente, adénocarcinome humain (femme 58 ans Caucasien ) |
| Liver | Hep G2 | Adhérente, carcinome hépatocellulaire (homme 15 ans Caucasien) |
| Breast | MCF-7 | Adhérente, adénocarcinome humain (femme 69 ans Caucasien ) |
| Pancreas | PANC-1 | Adhérente, carcinome (homme 56 ans Caucasien) |
| | MIA PaCa-2 | Adhérente, carcinome (homme 65 ans Caucasien) |

### II.2. Milieux de culture

Les cellules sont cultivées dans le milieu de culture spécifique décrit dans le Tableau **II,** à 37°C, 5% CO₂, selon les procédures opératoires bien connues de l'Homme de l'Art.

**Tableau II: Composition des milieux de culture**

| **Organe** | **Lignée cellulaire** | **Milieu de Culture** |
|---|---|---|
| Prostate | PC3 | RPMI 1640 + 10% FBS |
| | DU145 | RPMI 1640 + 10% FBS |
| Colon | HT-29 | Mc Coy's 5a + 10% FBS + 0,5 mM Ultraglutamine |
| | LS174T | EMEM + 10% FBS + 2 mM Ultraglutamine + 1 mM pyruvate de sodium + 0,1 mM amino-acides non essentiels |
| Sein | MCF-7 | EMEM + 10% FBS + 2 mM Ultraglutamine + 1 mM pyruvate de sodium + 0,1 mM amino-acides non essentiels + 10 nM β-estradiol |
| Pancréas | PANC-1 | RPMI + 10% FBS |
| | MIA PaCa-2 | DMEM + 10% FBS |
| Foie | Hep G2 | EMEM + 10% de FBS complémenté + 0,1 mM amino-acides non essentiels + 2 mM ultraglutamine + Penicilline/Streptomycine |

### II.3. Evaluation de la cytotoxicité des composés selon l'invention

Après décongélation et amplification des cellules cancéreuses dans le milieu de culture approprié (décrit en 1.2.), des plaques 96 puits sont ensemencées avec ces cellules et incubées ou non (contrôles), dans leur milieu de culture respectif, en présence des composés à tester à 10, 50, 100, 250 et 500 µM.

Après 96h d'incubation, chaque plaque 96-puits est analysée afin de mesurer la viabilité des cellules par un test colorimétrique au WST-1.

### II.4 Exemples

### F1. Activité anti-tumorale du composé 4 (voir exemple A5)

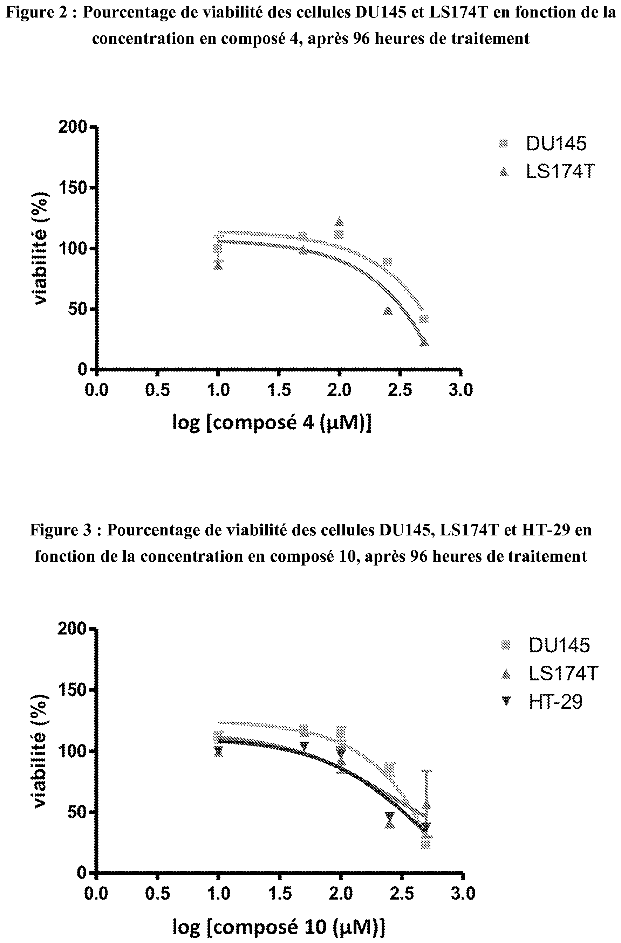

Les résultats de cytotoxicité obtenus avec le composé 4 sur deux lignées cellulaires sont présentés sur la Figure 2. Ces résultats montrent que la viabilité des cellules diminue d'autant plus que la concentration en composé 4 augmente, la concentration en composé 4 qui diminue de 50% la viabilité cellulaire étant égale à 481 et 335 µM, respectivement pour les cellules DU145 et LS174T.

### F2. Activité anti-tumorale du composé 10 (voir exemple B1)

Les résultats de cytotoxicité obtenus avec le composé 10 sur trois lignées cellulaires sont présentés sur la Figure 3. Ces résultats montrent que la viabilité des cellules diminue d'autant plus que la concentration en composé 10 augmente, la concentration en composé 10 qui diminue de 50% la viabilité cellulaire étant égale à 392, 430 et 328 µM, respectivement pour les cellules DU145, LS174T et HT-29.

### F3. Activité anti-tumorale du composé 38 (voir exemple E2)

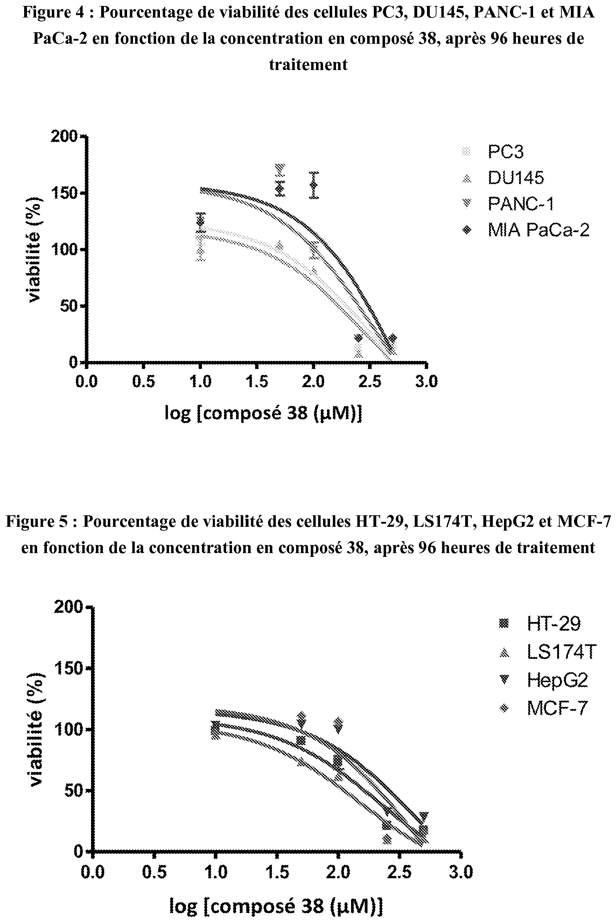

Les résultats de cytotoxicité obtenus avec le composé 38 sur huit lignées cellulaires sont présentés sur les Figures 4a et 4b. Ces résultats montrent que la viabilité des cellules diminue d'autant plus que la concentration en composé 38 augmente. La concentration en composé 38 qui diminue de 50% la viabilité cellulaire est donnée dans le tableau III pour chaque lignée cellulaire.

**Tableau III : Concentration en composé 38 diminuant de 50% la viabilité cellulaire après 96 heures de traitement**

| **Lignée cellulaire** | **Composé 38 (µM)** |
|---|---|
| PC3 | 198 |
| DU145 | 169 |
| PANC-1 | 253 |
| MIA PaCa-2 | 308 |
| HT-29 | 166 |
| LS174T | 113 |
| Hep G2 | 263 |
| MCF-7 | 215 |

### III. Exemples décrivant les compositions des composés selon l'invention

### Exemple G1 : Compositions contenant le composé 10

On a préparé des gélules de composition suivante :

| | |
|---|---|
| Composé 10 Acide 2-hydroxy-3-méthylsélénopropanoïque | 0,40mg (en eq. Se) |
| Excipients* et enveloppe** | une gélule de 1000 mg |

(* amidon de maïs, lactose, stéarate de magnésium, arôme,
** gélatine, dioxyde de titane, colorants)

### Exemple G2 : Compositions contenant le composé 38

On a préparé des gélules de composition suivante :

| | |
|---|---|
| Composé 38 Acide 2-(dodécanoyloxy)-3-(méthylséléno) | 0,40mg (en eq. Se) |
| propanoïque | |
| Excipients* et enveloppe** | une gélule de 1000 mg |

(* amidon de maïs, lactose, stéarate de magnésium, arôme,
** gélatine, dioxyde de titane, colorants)

### Exemple G3 : Compositions contenant le composé 10 et le composé 38

On a préparé des gélules de composition suivante :

| | |
|---|---|
| Composé 10 Acide 2-hydroxy-3-méthylsélénopropanoïque | 0,10mg (en eq. Se) |
| Composé 38 Acide 2-(dodécanoyloxy)-3-(méthylséléno) | 0,40mg (en eq. Se) |
| propanoïque | |
| Excipients* et enveloppe** | une gélule de 1000 mg |

(* amidon de maïs, lactose, stéarate de magnésium, arôme,
** gélatine, dioxyde de titane, colorants)

## Revendications

1. Composé sélénié répondant à la formule générale (I) suivante : 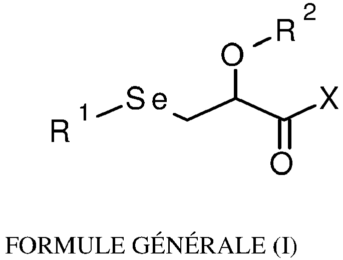 dans laquelle
**R¹** = alkyle constitué par un radical carboné saturé de 1 à 26 atomes de carbone linéaire, ramifié ou cyclique;
**R²** = H, **R⁴**C(=O), **R⁴**OC(=O), α-amino-acyle constitué par -C(=O)CH(Y)NH2, Y correspondant à la chaine latérale de l'un des aminoacides protéogèniques, CH₃SeCH₂CH₂CH(NH₂)C(=O), CH₃SeCH₂CH₂CH(OH)C(=O);
X = OH, O**R³**, NH₂, N**R⁴R⁵,** α-amino-acide constitué par -NHCH(Y)COOH, Y correspondant à la chaine latérale de l'un des aminoacides protéogèniques, CH₃SeCH₂CH₂CH(COOH)NH-, CH₃ScCH₂CH₂CH(COOH)O-;
**R³** = alkyle constitué par un radical carboné saturé de 1 à 26 atomes de carbone linéaire, ramifié ou cyclique;
**R⁴** = alkyle constitué par un radical carboné saturé de 1 à 26 atomes de carbone linéaire, ramifié ou cyclique, aryle; pyridinyle; ou imidazole ;
**R⁵** = H, alkyle constitué par un radical carboné saturé de 1 à 26 atomes de carbone linéaire, ramifié ou cyclique, aryle ; pyridinyle; ou imidazole ;
**R⁴** et **R⁵** pouvant former ensemble un radical cyclo-alkyle à 5 ou 6 chainons pouvant comporter un hétéroatome;
étant entendu que lorsque **X** = NHterbutyle alors **R²** ≠ C(=O)CH₃
ainsi que tous les stéréoisomères, diastéréoisomères et énantiomères notamment au niveau de l'atome de carbone portant le groupement O**R²**, ainsi qu'au niveau des radicaux **R¹** à **R⁵**, ainsi que les oligomères constitués par l'enchaînement de 2 à 15 monomères obtenus entre deux ou plusieurs molécules de dérivés de formule (I) par réaction d'estérification entre les fonctions alcool et acide carboxylique présentes le cas échéant, pris isolément ou en mélange ;
ainsi que tous les sels d'acides ou de bases pharmaceutiquement acceptables desdits composés de formule générale (I) ;
ainsi que les sels de sodium, de calcium, de zinc et de magnésium.

2. Composé sélénié selon la revendication 1, **caractérisé en ce que** R¹ représente un groupe méthyle, éthyle.

3. Composé sélénié selon la revendication 1 ou 2, **caractérisé en ce que** R² est choisi dans le groupe constitué par H, α-amino-acyle constitué par-C(=O)CH(Y)NH2, Y correspondant à la chaine latérale de l'un des aminoacides protéogèniques, R⁴(C=O), R⁴O(C=O), CH₃SeCH₂CH₂CH(OH)C(=O).

4. Composé sélénié selon l'une des revendications 1 à 3, **caractérisé en ce que** X est choisi dans le groupe OH, α-amino-acide constitué par-NHCH(Y)COOH, Y correspondant à la chaine latérale de l'un des aminoacides protéogèniques, CH₃SeCH₂CH₂CH(COOH)NH-, CH₃SeCH₂CH₂CH(COOH)O- .

5. Composé sélénié selon l'une des revendications 1 à 4, **caractérisé en ce que** R¹ représente un groupe méthyle, éthyle ; R² représente R⁴(C=O), R⁴O(C=O) et X représente OH ou OR³.

6. Composé sélénié selon l'une des revendications 1 à 5, **caractérisé en ce que** les acides pharmaceutiquement acceptables, sont choisis parmi les acides minéraux tels que chlorhydrique, bromhydrique, iodhydrique, sulfurique, tartrique, phosphorique ; ou choisis parmi les acides organiques tels que les acides formique, acétique, trifluoro-acétique, propionique, benzoïque, maléique, fumarique, succinique, citrique, oxalique, glyoxylique, aspartique, alcanesulfoniques tels que les acides méthane-sulfonique, trifluorométhanesulfonique, éthane-sulfonique, aryl-sulfoniques tels que les acides benzène- et paratoluènesulfonique.

7. Composé sélénié selon l'une des revendications 1 à 5, **caractérisé en ce que** les bases pharmaceutiquement acceptables sont choisies parmi les bases minérales telles que les hydroxydes de sodium, de lithium, de calcium, de potassium, de magnésium, d'ammonium ou de zinc, les carbonates de métaux alcalins ou alcalino-terreux tels que les carbonates et bicarbonates de sodium, de lithium, de calcium, de potassium, de magnésium, d'ammonium ou de zinc ou des bases organiques comme la méthylamine, la propylamine, la triméthylamine, la diéthylamine, la triéthylamine, la N,N-diméthyléthanolamine, le tris(hydroxy-méthyl)aminométhane, l'éthanolamine, la pyridine, la picoline, la dicyclohexylamine, la morpholine, la proceïne, la lysine, l'arginine, l'histidine, la N-méthylglucamine ou encore les sels de phosphonium tels que les sels d'alkyl-phosphonium, les sels d'aryl-phosphonium, les sels d'alkyl-aryl-phosphonium, les alkènyl-aryl-phosphonium ou les sels d'ammonium quaternaires tels que les sels de tétra-n-butyl-ammonium.

8. Composé sélénié selon l'une des revendications 1 à 7, **caractérisé en ce que** ledit composé est choisi parmi le groupe consistant de :
l'ester méthylique de l'acide 2-hydroxy-3-(méthylséléno)propanoïque ;
l'ester méthylique de l'acide (*R*)-2-hydroxy-3-(méthylséléno)propanoïque ;
l'ester méthylique de l'acide (*S*)-2-hydroxy-3-(méthylséléno)propanoïque ;
l'ester éthylique de l'acide 2-hydroxy-3-(méthylséléno)propanoïque ;
l'ester tert-butylique de l'acide 2-hydroxy-3-(méthylséléno)propanoïque ;
l'ester méthylique de l'acide 3-(éthylséléno)-2-hydroxypropanoïque ;
l'ester méthylique de l'acide 2-hydroxy-3-(isobutylséléno)propanoïque ;
l'ester isopropyle de l'acide 2-hydroxy-3-(méthylséléno)propanoïque ;
l'acide 2-hydroxy-3-(méthylséléno)propanoïque ;
l'acide (*R*)-2-hydroxy-3-(méthylséléno)propanoïque ;
l'acide (*S*)-2-hydroxy-3-(méthylséléno)propanoïque ;
l'acide 3-éthylséléno-2-hydroxypropanoïque ;
l'acide 2-hydroxy-3-(isobutylséléno)propanoïque ;
le sel dicyclohexylammonium 2-hydroxy-3-(méthylséléno)propanoate ;
le sel de sodium 2-hydroxy-3-(méthylséléno)propanoate ;
le sel de magnesium bis(2-hydroxy-3-(méthylséléno)propanoate ;
le sel du zinc bis(2-hydroxy-3-(méthylséléno)propanoate ;
le sel du calcium bis(2-hydroxy-3-(méthylséléno)propanoate ;
le 2-hydroxy-3-(méthylséléno)propanamide ;
le N-cyclopropyl-2-hydroxy-3-(méthylséléno)propanamide ;
la 2-hydroxy-3-(méthylséléno)-1-(pyrrolidin-1-yl)propan-1-one ;
la 2-hydroxy-3-(méthylséléno)-1-(pipéridin-1-yl)propan-1-one ;
la 2-hydroxy-3-(méthylséléno)-1-(morpholin-4-yl)propan-1-one ;
le N,N-diéthyl-2-hydroxy-3-(méthylséléno)-propanamide ;
la [(2*RS*)-2-hydroxy-3-méthylsélénopropanoyl]-(*S*)-alanine ;
la [(2*RS*)-2-hydroxy-3-méthylsélénopropanoyl]-(*S*)-méthionine ;
la [(2*R*)-2-hydroxy-3-méthylsélénopropanoyl]-(*S*)-méthionine ;
la [(2*S*)-2-hydroxy-3-méthylsélénopropanoyl]-(*S*)-méthionine ;
la [(2*RS*)-2-hydroxy-3-méthylsélénopropanoyl]-(*S*)-sélénométhionine ;
la [(2*R*)-2-hydroxy-3-méthylsélénopropanoyl]-(*S*)-sélénométhionine ;
la [(2*S*)-2-hydroxy-3-méthylsélénopropanoyl]-(*S*)-sélénométhionine ;
l'acide 2-(acétyloxy)-3-(méthylséléno)propanoïque ;
l'acide 2-(dodécanoyloxy)-3-(méthylséléno)propanoïque ;
l'ester méthylique de l'acide 2-(benzoyloxy)-3-(méthylséléno)propanoïque ;
l'acide 2-(benzoyloxy)-3-(méthylséléno)propanoïque ;
l'ester méthylique de l'acide 3-(méthylséléno)-2-[(3'-pyridine)oxycarbonyl]propanoïque ;
l'ester méthylique de l'acide 2-[(*tert*-butoxycarbonyl)oxyl-3-(méthylséléno)propanoïque ;
l'acide 2-[(*tert*-butoxycarbonyl)oxyl-3-(méthylséléno)propanoïque ;
l'ester méthylique de l'acide 4-méthylséléno-2-(2'-acétyloxy-3'-méthylsélénopropanoyl)butyrique ;
l'acide 4-méthylséléno-2-(2'-acétyloxy-3'-méthylsélénopropanoyl)butyrique ;
l'acide 2-(pentanoyloxy)-3-(méthylséléno)-propanoïque ;
l'acide 2-(nonanoyloxy)-3-(méthylséléno)-propanoïque ;
l'acide 2-(linoleoyloxy)-3-(méthylséléno)-propanoïque ;
l'acide 2-(pivaloyloxy)-3-(méthylséléno)-propanoïque ;
l'acide 2-{(1H-imidazoyl-4-ylcarbonyl)oxy)}-3-(méthylséléno)-propanoïque ;
l'ester méthylique de l'acide 2-(pivaloyloxy)-3-(méthylséléno)-propanoïque ;

9. Composé sélénié choisi parmi le groupe consistant de :
l'ester benzylique de l'acide (*S*)-(2-hydroxy-3-(méthylséléno)propanoïque ;
le N-[2-(diméthylamino)éthyl]-2-hydroxy-3-(méthylséléno)propanamide ;
le 2-hydroxy-N-(2-hydroxyéthyl)-3-(méthylséléno)-propanamide ;
l'ester tert-butylique du [(2*RS*)-2-hydroxy-3-méthylsélénopropanoyl]-(*S*)-alanine ;
l'ester méthylique du [(2*RS*)-2-hydroxy-3-méthylsélénopropanoyl]-(*S*)-méthionine ;
l'ester méthylique de la [(2*R*)-2-hydroxy-3-méthylsélénopropanoyl]-(*S*)-méthionine ;
l'ester méthylique de la [(2*S*)-2-hydroxy-3-méthylsélénopropanoyl]-(*S*)-méthionine ;
l'ester méthylique de la N(α)-[(2*RS*)-2-hydroxy-3-méthylsélénopropanoyll-N(ω)-*tert-*butoxycarbonyl-(*S*)-lysine ;
l'ester méthylique de la N(α)-[(2*RS*)-2-hydroxy-3-méthylsélénopropanoyl]-N(ω)-fluorenylméthyloxycarbonyl-(*S*)-lysine ;
l'ester méthylique de la N(α)-[(2*RS*)-2-hydroxy-3-méthylsélénopropanoyl]-N(ω)-benzyloxycarbonyl-(*S*)-lysine ;
l'ester méthylique de la [(2*RS*)-2-hydroxy-3-méthylsélénopropanoyl]-(*S*)-sélénométhionine ;
l'ester méthylique de la [(2*R*)-2-hydroxy-3-méthylsélénopropanoyl]-(*S*)-sélénométhionine ;
l'ester méthylique de la [(2*S*)-2-hydroxy-3-méthylsélénopropanoyl]-(*S*)-sélénométhionine ;
l'ester méthylique de l'acide (2*RS*)-[N-(*tert*-butoxycarbonyl)-*S*-methionyl]-3-(méthylséléno)propanoïque ;
l'ester méthylique de l'acide 3-méthylséléno-2-(2'-acétyloxy-4'-méthylsélénobutanoyl)propanoïque ;
l'acide 3-méthylséléno-2-(2'-hydroxy-4'-méthylsélénobutanoyl)-propanoïque ;
l'ester méthylique de l'acide 2-(linoleoyloxy)-3-(méthylséléno)-propanoïque ;
l'acide 2-(3-chloropropanoyloxy)-3-(méthylséléno)-propanoïque ;

10. Procédé de préparation des composés séléniés, en particulier de formule générale (I), définis à l'une quelconque des revendications 1 à 9, **caractérisé en ce qu'**il comprend les étapes suivantes :
1) la réaction d'un ester de l'acide oxirane-2-carboxylique racémique (DL) ou de l'un de ses énantiomères (D ou L) disponibles commercialement par exemple chez SAF France, avec
a/ soit un alkylsélénol R¹SeH, lui-même préparé *in situ* à partir d'un sel alcalin d'alkylsélénolate de formule R¹-Se-M₁ obtenu lui-même par réduction du dialkyle disélénure, dans laquelle M₁ représente un atome de métal alcalin, mis en réaction avec du chlorure d'ammonium;
b/ soit avec un dérivé dialkylaluminium alkylsélénolate de formule Al(R¹)₂SeR¹, lui-même généré *in situ* à partir du trialkylaluminium Al(R¹)₃ correspondant et du sélénium élémentaire Se°.
2) le cas échéant une ou plusieurs des réactions ou séries de réactions suivantes:
- hydrolyse de la fonction ester, puis
- acidification du milieu réactionnel pour obtenir les acides correspondants en particulier de formule (I) où X = OH; puis
- estérification des acides en particulier de formule (I) ou de leurs sels alcalins avec un alcool ou un halogénure d'alkyle pour obtenir les esters correspondants en particulier de formule générale (I) dans laquelle X = O**R³**, avec R³ tel que défini précédemment;
- amidification des acides en particulier de formule (I) avec une amine appropriée de formule R⁴R⁵NH, ou NH₃ dans laquelle R⁵ est tel que défini précédemment, pour obtenir le composé sélénié en particulier de formule générale (I) dans laquelle X = NH₂, NR⁴R⁵ ou α-amino-acide, CH₃SeCH₂CH₂CH(COOH)NH-,
- estérification, lorsque R² = H, de la fonction hydroxyle par un acide approprié pour obtenir le composé sélénié en particulier de formule générale (I) dans laquelle OR² est différent du groupe OH ;
- salification par un acide ou par une base.

11. Procédé selon la revendication 10, **caractérisé en ce que** le réactif sélénié est :
* soit un dialkylaluminium alkylsélénolate, et plus particulièrement le diméthyle aluminium méthylsélénolate généré *in situ* à partir de sélénium métal Se° et du triméthyle aluminium Al(CH₃)₃ dans un solvant polaire aprotique.
* soit un alkylsélénol, généré *in situ* à partir de sélénium métal Se(0) et d'alkyle lithium, dans un solvant polaire aprotique, puis mis en présence de chlorure d'ammonium.

12. Procédé selon la revendication 10 ou 11, **caractérisé en ce que** la réaction 1) a lieu au sein d'un solvant polaire aprotique tel que par exemple le THF et **en ce que** les réactions subséquentes, conduisant aux différents composés séléniés en particulier de formule (I), comprennent au moins une acidification, ou une estérification, ou une amidification et salification, dans des conditions bien connues de l'homme du métier.

13. Composé sélénié en particulier de formule (I) tel que défini à l'une des revendications 1 à 9, pour son utilisation comme agent pharmaceutique, en particulier comme agent anti-tumoral, seul ou associé à au moins un autre agent pharmaceutique et notamment à au moins un autre agent anti-tumoral, dans une méthode de traitement pharmaceutique, en particulier pour réaliser la prévention et le traitement de tumeurs ou cancers, soit seul soit en association avec un ou plusieurs autres agents anti-cancéreux ou cytotoxiques connus, soit en pré-administration soit en co-administration, tels que ceux notamment de la prostate, du foie, du rein, du pancréas, du poumon, du colon et de la peau.

14. Composition pharmaceutique **caractérisée en ce qu'**elle comprend au moins un principe pharmaceutiquement actif comprenant au moins un composé sélénié en particulier de formule générale (I), tel que défini à l'une quelconque des revendications 1 à 9, seul ou associé avec au moins un autre principe pharmaceutiquement actif.

15. Composition pharmaceutique selon la revendication 14, **caractérisée en ce que** le composé sélénié en particulier de formule générale (I), constitue un principe pharmaceutiquement actif pour réaliser la prévention et le traitement de tumeurs ou cancers, soit seul soit en association avec un ou plusieurs autres agents anti-cancéreux ou cytotoxiques connus, soit en pré-administration soit en co-administration, tels que ceux notamment de la prostate, du foie, du rein, du pancréas, du poumon, du colon et de la peau.

16. Composition pharmaceutique selon la revendication 14 ou 15, **caractérisée en ce que** au moins un des autres agents anticancéreux soit choisi parmi les composés suivants : l'aminoglutéthimide, l'estramustine, le medroxyprogesteroneacetate, le leuprolide, le flutamide, le torémifène, le Zoladex, les inhibiteurs de métalloprotéinase de matrice; les inhibiteurs du VEGF, tels que les anticorps anti-VEGF (Avastin (R)) et les petites molécules comme le ZD6474 et le SU6668; le vatalanib, le BAY-43-9006, le SU11248, le CP-547632 et le CEP-7055; les SA-1 et SA-2 inhibiteurs y compris les anticorps anti-HER2 (Herceptin), les inhibiteurs de l'EGFR, y compris le gefitinib, l'erlotinib, l'ABX-EGF, l'EMD72000, le 11F8, et le cetuximab ; les inhibiteurs d'Eg5, tels que le SB-715992, le SB-743921, et le MKI-833 ; les inhibiteurs de PAN, tels que le canertinib, l'EKB-569, le CI-1033, l'AEE-788, le XL-647 ; les inhibiteurs de kinase, tels que le 2C4, et le GW-572016, le Gleevec (R) et le dasatinib (Sprycel (R)); le Casodex (R) (bicalutamide, Astra Zeneca), le tamoxifène; les inhibiteurs de MAPK kinase, les inhibiteurs de PI3 kinase, les inhibiteurs de PDGF, tels que l'imatinib; les anti-angiogéniques et les agents anti-vasculaires; les inhibiteurs de non-récepteurs et récepteurs tyrosine kinases, les inhibiteurs de la signalisation de l'intégrine; la tubuline ; les agents tels que la vinblastine, la vincristine, la vinorelbine, la vinflunine, le paclitaxel, le docétaxel, le 7-O-methylthiomethylpaclitaxel, le 4-désacétyl-4-methylcarbonatepaclitaxel, le C-4 méthyl carbonate paclitaxel, l'epothilone A, l'epothilone B, l'épothilone C, l'epothilone D, la désoxyépothilone A, la désoxyépothilone B, l'oxabicyclo [14.1.0] le heptadécane-5-9-dione (ixabepilone), la leucovorine, le tégafur et des dérivés de ceux-ci; les inhibiteurs de CDK, les inhibiteurs du cycle cellulaire anti-prolifératifs, l'epidophyllotoxin, l'étoposide, le VM-26; les enzymes antinéoplasiques, les inhibiteurs de topoisomérase I et II tels que la camptothécine, le topotécan, la SN-38, la procarbazine, la mitoxantrone ; les complexes de coordination du platine tels que le cisplatine, le carboplatine et oxaliplatine ; les antagonistes de la purine tels que la 6-thioguanine et la 6-mercaptopurine; les antagonistes de la glutamine.

17. Composition pharmaceutique selon la revendication 14, 15 ou 16, **caractérisée en ce que** au moins un des autres agents cytotoxiques soit choisi parmi au moins un composé suivant: le cyclophosphamide, la doxorubicine, la daunorubicine, la mitoxantrone, le melphalan, l'hexaméthyle mélamine, le thiotépa, la cytarabine, l'idatrexate, le trimétrexate, la dacarbazine, la L-asparaginase, le bicalutamide, le leuprolide, les dérivés de pyridobenzoindole, les interférons, les interleukines.

18. Composition pharmaceutique selon l'une des revendications 14, 15, 16, ou 17, **caractérisée en ce qu'**elle comprend au moins un composé sélénié en particulier de formule (I) à une concentration comprise entre 0,02% et 0,15% en poids équivalent sélénium.

19. Composition pharmaceutique selon l'une des revendications 14 à 18, **caractérisée en ce qu'**elle comprend au moins un support pharmaceutiquement acceptable et une quantité thérapeutiquement efficace d'un ou plusieurs des composés sélénies ou d'un stéréoisomère, d'un tautomère, ou d'un sel pharmaceutiquement acceptable; ces milieux peuvent notamment consister en :
* une solution injectable ou buvable,
* un milieu solide composé d'un ou plusieurs excipients pouvant être sélectionnés parmi des vitamines, des antioxydants naturels tel que la L-ergothionéine, des sels minéraux, des mono-, di- ou polysaccharides, notamment l'acide folique, les vitamines B₆, E ou C, du lactose, de l'amidon.

20. Composition pharmaceutique selon l'une des revendications 14 à 19, **caractérisée en ce qu'**elle est formulée pour une voie d'administration choisie parmi la voie orale, intraveineuse, inhalation et en particulier se présenter sous la forme d'une gélule, d'un gel, d'un comprimé ou d'une poudre.

21. Composition pharmaceutique selon l'une des revendications 14 à 20, **caractérisée en ce que** le composé de formule (I) est choisi parmi :
a) le composé 4 de formule : 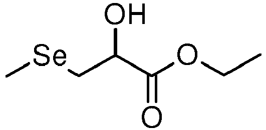 ou
b) le composé 10 de formule : 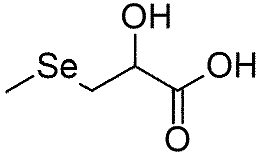 ou
c) le composé 38 de formule :

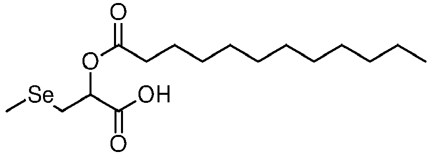

## Patentansprüche

1. Selenverbindung, die die folgende allgemeine Formel (I) aufweist: 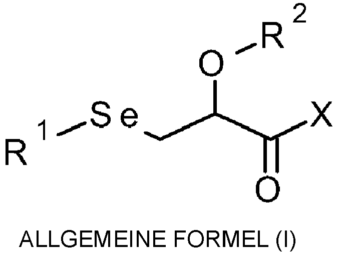 wobei
R¹ = Alkyl, das durch einen linearen, verzweigten oder cyclischen gesättigten Kohlenstoffrest dargestellt ist, der von 1 bis 26 Kohlenstoffatome aufweist;
R² = H, R⁴C(=O), R⁴OC(=O), α-Aminoacyl, das durch -C(=O)CH(Y)NH2 dargestellt ist, wobei Y der Seitenkette einer der proteogenen Aminosäuren, CH₃SeCH₂CH₂CH(NH₂)C(=O), CH₃SeCH₂CH₂CH(OH)C(=O), entspricht;
X = OH, OR³, NH₂, NR⁴R⁵,α-Aminosäure, die durch -NHCH(Y)COOH dargestellt ist, wobei Y der Seitenkette einer der proteogenen Aminosäuren, CH₃SeCH₂CH₂CH(COOH)NH-, CH₃SeCH₂CH₂CH(COOH)O-, entspricht;
R³ = Alkyl, das durch einen linearen, verzweigten oder cyclischen gesättigten Kohlenstoffrest dargestellt ist, der von 1 bis 26 Kohlenstoffatome aufweist;
R⁴ = Alkyl, das durch einen linearen, verzweigten oder cyclischen gesättigten Kohlenstoffrest dargestellt ist, der von 1 bis 26 Kohlenstoffatome aufweist, Aryl; Pyridinyl; oder Imidazol;
R⁵ = H, Alkyl, das durch einen linearen, verzweigten oder cyclischen gesättigten Kohlenstoffrest dargestellt ist, der von 1 bis 26 Kohlenstoffatome aufweist, Aryl; Pyridinyl; oder Imidazol;
R⁴ und R⁵ in der Lage sind, zusammen einen 5- oder 6-gliedrigen Cycloalkylrest auszubilden, der ein Heteroatom umfassen kann;
vorausgesetzt, dass, wenn X = NH-tert-Butyl, R²≠ C(=O)CH₃,
sowie alle Stereoisomere, Diastereoisomere und Enantiomere insbesondere in Bezug auf das Kohlenstoffatom, das die Gruppe OR² trägt, sowie in Bezug auf die Radikale R¹ bis R⁵ sowie alle Oligomere, die durch die Kette von 2 bis 15 Monomeren dargestellt sind, die zwischen zwei oder mehr Molekülen von Derivaten der Formel (I) durch Veresterungsumsetzung zwischen den gegebenenfalls vorhandenen Alkohol- und Carbonsäurefunktionen erhalten wird, allein oder in einer Mischung betrachtet;
sowie die gesamten Salze von pharmazeutisch unbedenklichen Säuren oder Basen der Verbindungen der allgemeinen Formel (I);
sowie die Salze von Natrium, Calcium, Zink und Magnesium.

2. Selenverbindung nach Anspruch 1, **dadurch gekennzeichnet, dass** R¹ für eine Methyl-, Ethylgruppe steht.

3. Selenverbindung nach Anspruch 1 oder 2, **dadurch gekennzeichnet, dass** R² aus der Gruppe ausgewählt ist, die aus H, α-Aminoacyl, das durch -C(=O)CH(Y)NH2 dargestellt ist, wobei Y der Seitenkette einer der proteogenen Aminosäuren, R⁴(C=O), R⁴O(C=O), CH₃SeCH₂CH₂CH(OH)C(=O) entspricht, besteht.

4. Selenverbindung nach einem der Ansprüche 1 bis 3, **dadurch gekennzeichnet, dass** X aus der Gruppe OH, α-Aminosäure, die durch -NHCH(Y)COOH dargestellt ist, wobei Y der Seitenkette einer der proteogenen Aminosäuren, CH₃SeCH₂CH₂CH(COOH)NH-, CH₃SeCH₂CH₂CH(COOH)O-, entspricht, ausgewählt ist.

5. Selenverbindung nach einem der Ansprüche 1 bis 4, **dadurch gekennzeichnet, dass** R¹ für eine Methyl-, Ethylgruppe steht; R² für R⁴(C=O), R⁴O(C=O) steht und X für OH oder OR³ steht.

6. Selenverbindung nach einem der Ansprüche 1 bis 5, **dadurch gekennzeichnet, dass** die pharmazeutisch unbedenklichen Säuren aus den Mineralsäuren wie etwa Chlorwasserstoff-, Bromwasserstoff-, lodwasserstoff-, Schwefel-, Wein-, Phosphorsäuren ausgewählt sind; oder aus den organischen Säuren wie Ameisen-, Essig-, Trifluoressig-, Propion-, Benzoe-, Malein-, Fumar-, Bernstein-, Zitronen-, Oxal-, Glyoxyl-, Asparaginsäuren, Alkansulfonsäuren wie etwa Methansulfon-, Trifluormethansulfon-, Ethansulfon-, Arylsulfonsäuren wie etwa Benzol- und para-Toluolsulfonsäuren ausgewählt sind.

7. Selenverbindung nach einem der Ansprüche 1 bis 5, **dadurch gekennzeichnet, dass** die pharmazeutisch unbedenklichen Basen aus den Mineralbasen wie etwa Natrium-, Lithium-, Calcium-, Kalium-, Magnesium-, Ammonium- oder Zinkhydroxiden, den Carbonaten von Alkali- oder Erdalkalimetallen wie etwa Natrium-, Lithium-, Calcium-, Kalium-, Magnesium-, Ammonium- oder Zinkcarbonaten und -bicarbonaten oder organischen Basen wie etwa Methylamin, Propylamin, Trimethylamin, Diethylamin, Triethylamin, N,N-Dimethylethanolamin, Tris(hydroxymethyl)aminomethan, Ethanolamin, Pyridin, Picolin, Dicyclohexylamin, Morpholin, Procein, Lysin, Arginin, Histidin, N-Methylglucamin oder aber den Phosphoniumsalzen wie etwa den Alkylphosphoniumsalzen, den Arylphosphoniumsalzen, den Alkylarylphosphoniumsalzen, den Alkenylarylphosphoniumsalze oder den quaternären Ammoniumsalzen wie etwa den Tetran-butylammoniumsalzen ausgewählt sind.

8. Selenverbindung nach einem der Ansprüche 1 bis 7, **dadurch gekennzeichnet, dass** die Verbindung aus der Gruppe ausgewählt ist, die aus Folgendem besteht:
2-Hydroxy-3-(methylseleno)propansäuremethylester;
(*R*)-2-Hydroxy-3-(methylseleno)propansäuremethylester;
(S)-2-Hydroxy-3-(methylseleno)propansäuremethylester;
2-Hydroxy-3-(methylseleno)propansäureethylester;
2-Hydroxy-3-(methylseleno)propansäure-*tert*-butylester;
3-(Ethylseleno)-2-hydroxypropansäuremethylester;
2-Hydroxy-3-(isobutylseleno)propansäuremethylester;
2-Hydroxy-3-(methylseleno)propansäureisopropylester;
2-Hydroxy-3-(methylseleno)propansäure;
(R)-2-Hydroxy-3-(methylseleno)propansäure;
(S)-2-Hydroxy-3-(methylseleno)propansäure;
3-Ethylseleno-2-hydroxypropansäure;
2-Hydroxy-3-(isobutylseleno)propansäure;
Dicyclohexylammonium-2-hydroxy-3-(methylseleno)propanoatsalz;
Natrium-2-hydroxy-3-(methylseleno)propanoatsalz;
Magnesium-bis(2-hydroxy-3-(methylseleno)propanoatsalz;
Zink-bis(2-hydroxy-3-(methylseleno)propanoatsalz;
Calcium-bis(2-hydroxy-3-(methylseleno)propanoatsalz;
2-Hydroxy-3-(methylseleno)propanamid;
N-Cyclopropyl-2-hydroxy-3-(methylseleno)propanamid;
2-Hydroxy-3-(methylseleno)-1-(pyrrolidin-1-yl)propan-1-on;
2-Hydroxy-3-(methylseleno)-1-(piperidin-1-yl)propan-1-on;
2-Hydroxy-3-(methylseleno)-1-(morpholin-4-yl)propan-1-on;
N, N-Diethyl-2-hydroxy-3-(methylseleno)propanamid;
[(2*RS*)-2-Hydroxy-3-methylselenopropanoyl]-(*S*)-alanin;
[(2RS)-2-Hydroxy-3-methylselenopropanoyl]-(*S*)-methionin;
[(2*R*)-2-Hydroxy-3-methylselenopropanoyl]-(*S*)-methionin;
[(2*S*)-2-Hydroxy-3-methylselenopropanoyl]-(*S*)-methionin;
[(2RS)-2-Hydroxy-3-methylselenopropanoyl]-(*S*)-selenomethionin;
[(2*R*)-2-Hydroxy-3-methylselenopropanoyl]-(*S*)-selenomethionin;
[(2*S*)-2-Hydroxy-3-methylselenopropanoyl]-(*S*)-selenomethionin;
2-(Acetyloxy)-3-(methylseleno)propansäure;
2-(Dodecanoyloxy)-3-(methylseleno)propansäure;
2-(Benzoyloxy)-3-(methylseleno)propansäuremethylester;
2-(Benzoyloxy)-3-(methylseleno)propansäure;
3-(Methylseleno)-2-[(3'-pyridin)oxycarbonyl]propansäuremethylester;
2-[(*tert*-Butoxycarbonyl)oxy]-3-(methylseleno)propansäuremethylester;
2-[(*tert*-Butoxycarbonyl)oxy]-3-(methylseleno)propansäure;
4-Methylseleno-2-(2'-acetyloxy-3'-methylselenopropanoyl)buttersäuremethylester;
4-Methylseleno-2-(2'-acetyloxy-3'-methylselenopropanoyl)buttersäure;
2-(Pentanoyloxy)-3-(methylseleno)propansäure;
2-(Nonanoyloxy)-3-(methylseleno)propansäure;
2-(Linoleoyloxy)-3-(methylseleno)propansäure;
2-(Pivaloyloxy)-3-(methylseleno)propansäure;
2-{(1H-Imidazoyl-4-ylcarbonyl)oxy)}-3-(methylseleno)propansäure;
2-(Pivaloyloxy)-3-(methylseleno)propansäuremethylester;

9. Selenverbindung, die aus der Gruppe ausgewählt ist, die aus Folgendem besteht:
(*S*)-(2-Hydroxy-3-(methylseleno)propansäurebenzylester;
N-[2-(Dimethylamino)ethyl]-2-hydroxy-3-(methylseleno)propanamid;
2-Hydroxy-N-(2-hydroxyethyl)-3-(methylseleno)propanamid;
[(2*RS*)-2-Hydroxy-3-methylselenopropanoyl]-(*S*)-alanin-*tert*-butylester;
[(2*RS*)-2-Hydroxy-3-methylselenopropanoyl]-(*S*)-methioninmethylester;
[(2*R*)-2-Hydroxy-3-methylselenopropanoyl]-(*S*)-methioninmethylester;
[(2*S*)-2-Hydroxy-3-methylselenopropanoyl]-(*S*)-methioninmethylester;
N(α)-[(2*RS*)-2-Hydroxy-3-methylselenopropanoyl]-N(ω)-*tert*-butoxycarbonyl-(*S*)-lysinmethylester;
N(α)-[(2*RS*)-2-Hydroxy-3-methylselenopropanoyl]-N(ω)-Fluorenylmethyloxycarbonyl-(S)-lysinmethylester;
N(α)-[(2*RS*)-2-Hydroxy-3-methylselenopropanoyl]-N(ω)benzyloxycarbonyl-(*S*)-lysinmethylester;
[(2RS)-2-Hydroxy-3-methylselenopropanoyl]-(S)-selenomethioninmethylester;
[(2*R*)-2-Hydroxy-3-methylselenopropanoyl]-(*S*)-selenomethioninmethylester;
[(2S)-2-Hydroxy-3-methylselenopropanoyl]-(S)-selenomethioninmethylester;
(2*RS*)-[N-(tert-Butoxycarbonyl)-*S*-methionyl]-3-(methylseleno)propansäuremethylester;
3-Methylseleno-2-(2'-acetoxy-4'-methylselenobutanoyl)propansäuremethylester;
3-Methylseleno-2-(2'-hydroxy-4'-methylselenobutanoyl)propansäure;
2-(Linoleoyloxy)-3-(methylseleno)propansäuremethylester;
2-(3-Chlorpropanoyloxy)-3-(methylseleno)propansäure;

10. Verfahren zum Herstellen der Selenverbindungen, insbesondere der allgemeinen Formel (I), nach einem der Ansprüche 1 bis 9, **dadurch gekennzeichnet, dass** es die folgenden Schritte umfasst:
1) die Umsetzung eines racemischen (DL)-Oxiran-2-carbonsäureesters oder eines der Enantiomere davon (D oder L), die im Handel beispielsweise von SAF France erhältlich sind, mit
a/ entweder einem Alkylselenol R¹SeH, das selbst *in situ* aus einem Alkalimetallsalz von Alkylselenolat der Formel R¹-Se-M₁ hergestellt wird, das selbst durch Reduktion von Dialkyldiselenid erhalten wird, wobei M₁ für ein Alkalimetallatom steht, das mit Ammoniumchlorid umgesetzt ist;
b/ oder einem Dialkylaluminiumalkylselenolat-Derivat der Formel Al(R¹)₂SeR¹, das selbst *in situ* aus dem entsprechenden Trialkylaluminium Al(R¹)₃ und elementarem Selen Se(0) erzeugt wird,
2) gegebenenfalls eine oder mehrere der folgenden Umsetzungen oder Umsetzungsreihen:
- Hydrolyse der Esterfunktion, dann
- Ansäuerung des Umsetzungsmediums, um die entsprechenden Säuren der Formel (I) zu erhalten, wobei X = OH; dann
- Veresterung der Säuren der Formel (I) oder der Alkalimetallsalze davon mit einem Alkohol oder einem Alkylhalogenid, um die entsprechenden Ester insbesondere der allgemeinen Formel (I) zu erhalten, wobei X = O**R³**, wobei R³ wie oben definiert ist;
- Amidifizierung der Säuren insbesondere der Formel (I) mit einem geeigneten Amin der Formel R⁴R⁵NH oder NH₃, wobei R⁵ wie oben definiert ist, um die Verbindung insbesondere der allgemeinen Formel (I) zu erhalten, wobei X = NH₂, NR⁴R⁵ oder α-Aminosäure, CH₃SeCH₂CH₂CH(COOH)NH-,
- Veresterung, wenn R² = H, der Hydroxylfunktion durch eine geeignete Säure, um die Verbindung insbesondere der allgemeinen Formel (I) zu erhalten, wobei sich OR² von der OH-Gruppe unterscheidet;
- Salzbildung durch eine Säure oder durch eine Base.

11. Verfahren nach Anspruch 10, **dadurch gekennzeichnet, dass** das Selenreagens Folgendes ist:
* entweder ein Dialkylaluminiumalkylselenolat und genauer gesagt Dimethylaluminiummethylselenolat, das *in situ* aus Metallselen Se(0) und Trimethylaluminium (Al(CH₃)₃ in einem aprotischen polaren Lösungsmittel erzeugt wird.
* oder ein Alkylselenol, das *in situ* aus Metallselen Se(0) und Alkyllithium in einem aprotischen polaren Lösungsmittel erzeugt und dann in die Gegenwart von Ammoniumchlorid gegeben wird.

12. Verfahren nach Anspruch 10 oder 11, **dadurch gekennzeichnet, dass** Umsetzung 1) in einem aprotischen polaren Lösungsmittel wie etwa beispielsweise THF stattfindet, und dadurch dass die nachfolgenden Umsetzungen, die zu den unterschiedlichen Verbindungen der Formel (I) führen, wenigstens eine Ansäuerung oder eine Veresterung oder eine Amidifizierung und eine Salzbildung unter Bedingungen beinhalten, die dem Fachmann gut bekannt sind.

13. Selenverbindung, insbesondere der Formel (I), nach einem der Ansprüche 1 bis 9 für ihre Verwendung als pharmazeutisches Mittel, insbesondere als Antitumormittel, allein oder kombiniert mit wenigstens einem anderen pharmazeutischen Mittel und insbesondere mit wenigstens einem anderen Antitumormittel.

14. Pharmazeutische Zusammensetzung, **dadurch gekennzeichnet, dass** sie wenigstens einen pharmazeutischen Wirkstoff beinhaltet, der wenigstens eine Selenverbindung, insbesondere der allgemeinen Formel (I), nach einem der Ansprüche 1 bis 9 umfasst, allein oder in Kombination mit wenigstens einem anderen pharmazeutischen Wirkstoff.

15. Pharmazeutische Zusammensetzung nach Anspruch 14, **dadurch gekennzeichnet, dass** die Selenverbindung, insbesondere der allgemeinen Formel (I), einen pharmazeutischen Wirkstoff zum Durchführen der Vorbeugung und der Behandlung von Tumoren oder Krebsgeschwüren, entweder allein oder in Kombination mit einem oder mehreren anderen bekannten Antikrebs- oder cytotoxischen Mitteln, entweder durch Vorverabreichung oder durch gleichzeitige Verabreichung, wie etwa besonders von Tumoren oder Krebsgeschwüren der Prostata, der Leber, der Nieren, der Bauchspeicheldrüse, der Lunge, des Dickdarms und der Haut darstellt.

16. Pharmazeutische Zusammensetzung nach Anspruch 14 oder 15, **dadurch gekennzeichnet, dass** wenigstens eines der anderen Antikrebsmittel aus den folgenden Verbindungen ausgewählt ist: Aminoglutethimid, Estramustin, Medroxyprogesteronacetat, Leuprolid, Flutamid, Toremifen, Zoladex, den Matrix-Metalloproteinase-Inhibitoren; den VEGF-Inhibitoren wie etwa den Anti-VEGF-Antikörpern (Avastin (R)) und den niedermolekularen Verbindungen wie etwa ZD6474 und SU6668; Vatalanib, BAY-43-9006, SU11248, CP-547632 und CEP-7055; den SA-1- und SA-2-Inhibitoren, die die Anti-HER2-Antikörper (Herceptin) beinhalten, den EGFR-Inhibitoren, die Gefitinib, Erlotinib, ABX-EGF, EMD72000, 11F8 und Cetuximab beinhalten; den Eg5-Inhibitoren wie etwa SB-715992, SB-743921 und MKI-833; den PAN-Inhibitoren wie etwa Canertinib, EKB-569, Cl-1033, AEE-788, XL-647; den Kinaseinhibitoren wie etwa 2C4 und GW-572016, Gleevec (R) und Dasatinib (Sprycel (R)); Casodex (R) (Bicalutamid, Astra Zeneca), Tamoxifen; den MAPK-Kinase-Inhibitoren, den PI3-Kinase-Inhibitoren, den PDGF-Inhibitoren wie etwa Imatinib; den anti-angiogenen Mitteln und den antivaskulären Mitteln; den Rezeptor- und den Nichtrezeptor-Tyrosinkinase-Inhibitoren, den Inhibitoren einer Integrin-Signalübertragung; Tubulin; den Mitteln, wie etwa Vinblastin, Vincristin, Vinorelbin, Vinflunin, Paclitaxel, Docetaxel, 7-O-Methylthiomethylpaclitaxel, 4-Desacetyl-4-methylcarbonatepaclitaxel, C-4-Methylcarbonatpaclitaxel, Epothilon A, Epothilon B, Epothilon C, Epothilon D, Desoxyepothilon A, Desoxyepothilon B, Oxabicyclo[14.1.0]heptadecan-5-9-dion (Ixabepilon), Leucovorin, Tegafur und Derivaten davon; den CDK-Inhibitoren, den antiproliferativen Zellcyclusinhibitoren, Epidophyllotoxin, Etoposid, VM-26; den antineoplastischen Enzymen, den Topoisomerase-I- oder-II-Inhibitoren wie etwa Camptothecin, Topotecan, SN-38, Procarbazin, Mitoxantron; den Platinkoordinationskomplexen wie etwa Cisplatin, Carboplatin und Oxaliplatin; den Purinantagonisten wie etwa 6-Thioguanin und 6-Mercaptopurin; den Glutaminantagonisten.

17. Pharmazeutische Zusammensetzung nach Anspruch 14, 15 oder 16, **dadurch gekennzeichnet, dass** wenigstens eines der anderen cytotoxischen Mittel aus wenigstens einer folgenden Verbindung ausgewählt ist: Cyclophosphamid, Doxorubicin, Daunorubicin, Mitoxantron, Melphalan, Hexamethylmelamin, Thiotepa, Cytarabin, Idatrexat, Trimetrexat, Dacarbazin, L-Asparaginase, Bicalutamid, Leuprolid, den Pyridobenzoindolderivate, den Interferonen.

18. Pharmazeutische Zusammensetzung nach einem der Ansprüche 14, 15, 16 oder 17, **dadurch gekennzeichnet, dass** sie wenigstens eine Selenverbindung, insbesondere der Formel (I), in einer Konzentration zwischen 0,02 Gew.-% und 0,15 Gew.-% in Selenäquivalent umfasst.

19. Pharmazeutische Zusammensetzung nach einem der Ansprüche 14 bis 18, **dadurch gekennzeichnet, dass** sie wenigstens eine pharmazeutisch unbedenkliche Trägersubstanz und eine therapeutisch wirksame Menge einer oder mehrerer der Selenverbindungen oder eines Stereoisomers, eines Tautomers oder eines pharmazeutisch unbedenklichen Salzes umfasst. Diese Medien können insbesondere aus Folgendem bestehen:
* einer injizierbaren oder trinkbaren Lösung,
* einem festen Medium, das sich aus einem oder mehreren Arzneistoffträgern zusammensetzt, die aus Vitaminen, natürlichen Antioxidantien wie etwa L-Ergothionein, Mineralsalzen, Mono-, Di- oder Polysacchariden, insbesondere Folsäure, Vitaminen B₆, E oder C, Lactose, Stärke ausgewählt sein können.

20. Pharmazeutische Zusammensetzung nach einem der Ansprüche 14 bis 19, **dadurch gekennzeichnet, dass** sie für einen Verabreichungsweg formuliert ist, der aus dem oralen Weg, dem intravenösen Weg, dem parenteralen Weg, dem topischen Weg, der den transdermalen Weg oder den nasalen Weg oder den okularen Weg beinhaltet, oder durch Inhalation ausgewählt ist und insbesondere in der Form einer Kapsel, eines Gels, einer Tablette oder eines Pulvers vorliegt.

21. Pharmazeutische Zusammensetzung nach einem der Ansprüche 14 bis 20, **dadurch gekennzeichnet, dass** die Verbindung der Formel (I) aus Folgendem ausgewählt ist:
a) Verbindung 4 der Formel: 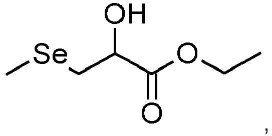 oder
b) Verbindung 10 der Formel: 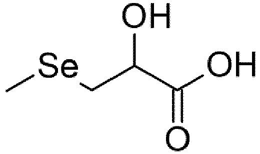 oder
c) Verbindung 38 der Formel:

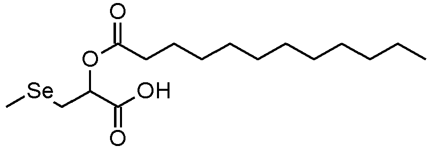

## Claims

1. A selenium compound having the following general formula (I): 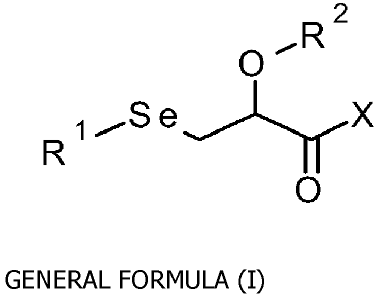 where
R¹ = alkyl constituted by a linear, branched or cyclic saturated carbon radical having from 1 to 26 carbon atoms;
R² = H, R⁴C(=O), R⁴OC(=O), α-aminoacyl constituted by - C(=O)CH(Y)NH2, Y corresponding to the lateral chain of one of the proteogenic aminoacids, CH₃SeCH₂CH₂CH(NH₂)C(=O), CH₃SeCH₂CH₂CH(OH)C(=O);
X = OH, OR³, NH₂, NR⁴R⁵, α-amino acid constituted by-NHCH(Y)COOH, Y corresponding to the lateral chain of one of the proteogenic aminoacids, CH₃SeCH₂CH₂CH(COOH)NH-, CH₃SeCH₂CH₂CH(COOH)O-;
R³ = alkyl constituted by a linear, branched or cyclic saturated carbon radical having from 1 to 26 carbon atoms;
R⁴ = alkyl constituted by a linear, branched or cyclic saturated carbon radical having from 1 to 26 carbon atoms, aryl; pyridinyl; or imidazole;
R⁵ = H, alkyl constituted by a linear, branched or cyclic saturated carbon radical having from 1 to 26 carbon atoms, aryl; pyridinyl; or imidazole;
R⁴ and R⁵ being capable of forming together a 5- or 6-membered cycloalkyl radical which can comprise a heteroatom;
provided that, when X = NH-tert-butyl, R² ≠ C(=O)CH₃,
as well as all the stereoisomers, diastereoisomers and enantiomers in particular with respect to the carbon atom bearing the group OR², as well as with respect to the radicals R¹ to R⁵, as well as all oligomers constituted by the chain of 2 to15 monomers obtained between two or more molecules of derivatives of formula (I) by esterification reaction between the alcohol and carboxylic acid functions present if applicable, considered alone or in a mixture;
as well as all the salts of pharmaceutically acceptable acids or bases of said compounds of general formula (I);
as well as the salts of sodium, calcium, zinc and magnesium.

2. The selenium compound according to Claim 1, **characterized in that** R¹ represents a methyl, ethyl group.

3. The selenium compound according to Claim 1 or 2, **characterized in that** R² is selected from the group consisting of H, α-aminoacyl constituted by -C(=O)CH(Y)NH2, Y corresponding to the lateral chain of one of the proteogenic aminoacids, R⁴(C=O), R⁴O(C=O), CH₃SeCH₂CH₂CH(OH)C(=O).

4. The selenium compound according to any one of Claims 1 to 3, **characterized in that** X is selected from the group OH, α-amino acid constituted by-NHCH(Y)COOH, Y corresponding to the lateral chain of one of the proteogenic aminoacids, CH₃SeCH₂CH₂CH(COOH)NH-, CH₃SeCH₂CH₂CH(COOH)O-.

5. The selenium compound according to any one of Claims 1 to 4, **characterized in that** R¹ represents a methyl, ethyl group; R² represents R⁴(C=O), R⁴O(C=O), and X represents OH or OR³.

6. The selenium compound according to any one of Claims 1 to 5, **characterized in that** the pharmaceutically acceptable acids are selected from the mineral acids such as hydrochloric, hydrobromic, hydroiodic, sulfuric, tartaric, phosphoric acids; or selected from the organic acids such as formic, acetic, trifluoroacetic, propionic, benzoic, maleic, fumaric, succinic, citric, oxalic, glyoxylic, aspartic acids, alkanesulfonic acids such as methanesulfonic, trifluoromethanesulfonic, ethanesulfonic, arylsulfonic acids such as benzene- and paratoluenesulfonic acids.

7. The selenium compound according to any one of Claims 1 to 5, **characterized in that** the pharmaceutically acceptable bases are selected from the mineral bases such as sodium, lithium, calcium, potassium, magnesium, ammonium or zinc hydroxides, the carbonates of alkali or alkaline earth metals such as sodium, lithium, calcium, potassium, magnesium, ammonium or zinc carbonates and bicarbonates, or organic bases such as methylamine, propylamine, trimethylamine, diethylamine, triethylamine, N,N-dimethylethanolamine, tris(hydroxymethyl)aminomethane, ethanolamine, pyridine, picoline, dicyclohexylamine, morpholine, proceine, lysine, arginine, histidine, N-methylglucamine, or else the phosphonium salts such as the alkyl-phosphonium salts, the aryl-phosphonium salts, the alkyl-aryl-phosphonium salts, the alkenyl-aryl-phosphonium salts, or the quaternary ammonium salts such as the tetra-n-butyl-ammonium salts.

8. The selenium compound according to any one of Claims 1 to 7, **characterized in that** said compound is selected from the group consisting of:
2-hydroxy-3-(methylseleno)propanoic acid methyl ester;
(R)-2-hydroxy-3-(methylseleno)propanoic acid methyl ester;
(S)-2-hydroxy-3-(methylseleno)propanoic acid methyl ester;
2-hydroxy-3-(methylseleno)propanoic acid ethyl ester;
2-hydroxy-3-(methylseleno)propanoic acid tert-butyl ester;
3-(ethylseleno)-2-hydroxypropanoic acid methyl ester;
2-hydroxy-3-(isobutylseleno)propanoic acid methyl ester;
2-hydroxy-3-(methylseleno)propanoic acid isopropyl ester;
2-hydroxy-3-(methylseleno)propanoic acid;
(R)-2-hydroxy-3-(methylseleno)propanoic acid;
(S)-2-hydroxy-3-(methylseleno)propanoic acid;
3-ethylseleno-2-hydroxypropanoic acid;
2-hydroxy-3-(isobutylseleno)propanoic acid;
dicyclohexylammonium 2-hydroxy-3-(methylseleno)propanoate salt;
sodium 2-hydroxy-3-(methylseleno)propanoate salt;
magnesium bis(2-hydroxy-3-(methylseleno)propanoate salt;
zinc bis(2-hydroxy-3-(methylseleno)propanoate salt;
calcium bis(2-hydroxy-3-(methylseleno)propanoate salt;
2-hydroxy-3-(methylseleno)propanamide;
N-cyclopropyl-2-hydroxy-3-(methylseleno)propanamide;
2-hydroxy-3-(methylseleno)-1-(pyrrolidin-1-yl)propan-1-one;
2-hydroxy-3-(methylseleno)-1-(piperidin-1-yl)propan-1-one;
2-hydroxy-3-(methylseleno)-1-(morpholin-4-yl)propan-1-one;
N,N-diethyl-2-hydroxy-3-(methylseleno)propanamide;
[(2RS)-2-hydroxy-3-methylselenopropanoyl]-(S)-alanine;
[(2RS)-2-hydroxy-3-methylselenopropanoyl]-(S)-methionine;
[(2R)-2-hydroxy-3-methylselenopropanoyl]-(S)-methionine;
[(2S)-2-hydroxy-3-methylselenopropanoyl]-(S)-methionine;
[(2RS)-2-hydroxy-3-methylselenopropanoyl]-(S)-selenomethionine;
[(2R)-2-hydroxy-3-methylselenopropanoyl]-(S)-selenomethionine;
[(2S)-2-hydroxy-3-methylselenopropanoyl]-(S)-selenomethionine;
2-(acetyloxy)-3-(methylseleno)propanoic acid;
2-(dodecanoyloxy)-3-(methylseleno)propanoic acid;
2-(benzoyloxy)-3-(methylseleno)propanoic acid methyl ester;
2-(benzoyloxy)-3-(methylseleno)propanoic acid;
3-(methylseleno)-2-[(3'-pyridine)oxycarbonyl]propanoic acid methyl ester;
2-[(tert-butoxycarbonyl)oxy]-3-(methylseleno)propanoic acid methyl ester;
2-[(tert-butoxycarbonyl)oxy]-3-(methylseleno)propanoic acid;
4-methylseleno-2-(2'-acetyloxy-3'-methylselenopropanoyl)butyric acid methyl ester;
4-methylseleno-2-(2'-acetyloxy-3'-methylselenopropanoyl)butyric acid;
2-(pentanoyloxy)-3-(methylseleno)propanoic acid;
2-(nonanoyloxy)-3-(methylseleno)propanoic acid;
2-(linoleoyloxy)-3-(methylseleno)propanoic acid;
2-(pivaloyloxy)-3-(methylseleno)propanoic acid;
2-{(1H-imidazoyl-4-ylcarbonyl)oxy)}-3-(methylseleno)propanoic acid;
2-(pivaloyloxy)-3-(methylseleno)propanoic acid methyl ester;

9. A selenium compound selected from the group consisting of:
(S)-(2-hydroxy-3-(methylseleno)propanoic acid benzyl ester;
N-[2-(dimethylamino)ethyl]-2-hydroxy-3-(methylseleno)propanamide;
2-hydroxy-N-(2-hydroxyethyl)-3-(methylseleno)propanamide;
[(2RS)-2-hydroxy-3-methylselenopropanoyl]-(S)-alanine tert-butyl ester;
[(2RS)-2-hydroxy-3-methylselenopropanoyl]-(S)-methionine methyl ester;
[(2R)-2-hydroxy-3-methylselenopropanoyl]-(S)-methionine methyl ester;
[(2S)-2-hydroxy-3-methylselenopropanoyl]-(S)-methionine methyl ester;
N(a)-[(2RS)-2-hydroxy-3-methylselenopropanoyl]-N(ω)-tert-butoxycarbonyl-(S)-lysine methyl ester;
N(α)-[(2RS)-2-hydroxy-3-methylselenopropanoyl]-N((ω)-fluorenylmethyloxycarbonyl-(S)-lysine methyl ester;
N(α)-[(2RS)-2-hydroxy-3-methylselenopropanoyl]-N((ω)-benzyloxycarbonyl-(S)-lysine methyl ester;
[(2RS)-2-hydroxy-3-methylselenopropanoyl]-(S)-selenomethionine methyl ester;
[(2R)-2-hydroxy-3-methylselenopropanoyl]-(S)-selenomethionine methyl ester;
[(2S)-2-hydroxy-3-methylselenopropanoyl]-(S)-selenomethionine methyl ester;
(2RS)-[N-(tert-butoxycarbonyl)-S-methionyl]-3-(methylseleno)propanoic acid methyl ester;
3-methylseleno-2-(2'-acetoxy-4'-methylselenobutanoyl)propanoic acid methyl ester;
3-methylseleno-2-(2'-hydroxy-4'-methylselenobutanoyl)propanoic acid;
2-(linoleoyloxy)-3-(methylseleno)propanoic acid methyl ester;
2-(3-chloropropanoyloxy)-3-(methylseleno)propanoic acid;

10. A method for preparing the selenium compounds in particular of general formula (I), defined in any one of Claims 1 to 9, **characterized in that** it comprises the following steps:
1) the reaction of a racemic (DL) oxirane-2-carboxylic acid ester or of one of the enantiomers thereof (D or L) which are commercially available, for example, from SAF France, with
a/ either an alkylselenol R¹SeH, which is itself prepared in situ from an alkali metal salt of alkylselenolate of formula R¹-Se-M₁ which is itself obtained by reduction of dialkyl diselenide, wherein M₁ represents an alkali metal atom, which is reacted with ammonium chloride;
b/ or a dialkylaluminum alkylselenolate derivative of formula Al(R¹)₂SeR¹, which is itself generated in situ from the corresponding trialkylaluminum Al(R¹)₃ and elemental selenium Se(0),
2) if applicable, one or more of the following reactions or series of reactions:
- hydrolysis of the ester function, then
- acidification of the reaction medium in order to obtain the corresponding acids of formula (I) where X = OH; then
- esterification of the acids of formula (I) or of the alkali metal salts thereof with an alcohol or an alkyl halide in order to obtain the corresponding esters in particular of general formula (I) where X = OR³, with R³ as defined above;
- amidification of the acids in particular of formula (I) with an appropriate amine of formula R⁴R⁵NH or NH₃ wherein R⁵ is as defined above, in order to obtain the compound in particular of general formula (I) wherein X = NH₂, NR⁴R⁵ or α-amino acid, CH₃SeCH₂CH₂CH(COOH)NH-,
- esterification, when R² = H, of the hydroxyl function by an appropriate acid in order to obtain the compound in particular of general formula (I) wherein OR² is different from the OH group;
- salification by an acid or by a base.

11. The method according to Claim 10, **characterized in that** the selenium reagent is:
* either a dialkylaluminum alkylselenolate, and more particularly dimethylaluminum methylselenolate generated in situ from metal selenium Se(0) and trimethylaluminum (Al(CH₃)₃ in an aprotic polar solvent.
* or an alkylselenol, generated in situ from metal selenium Se(0) and alkyl lithium, in an aprotic polar solvent, and then put in the presence of ammonium chloride.

12. The method according to Claim 10 or 11, **characterized in that** reaction 1) takes place in an aprotic polar solvent such as THF, for example, and **in that** the subsequent reactions leading to the different compounds of formula (I) include at least one acidification, or esterification, or amidification and salification, under conditions well known to the person skilled in the art.

13. A selenium compound in particular of formula (I) as defined in any one of Claims 1 to 9 for its use as pharmaceutical agent, in particular as antitumor agent, alone or combined with at least one other pharmaceutical agent and in particular with at least one other antitumor agent.

14. A pharmaceutical composition, **characterized in that** it includes at least one pharmaceutically active ingredient comprising at least one selenium compound in particular of general formula (I) as defined in anyone of claims 1 to 9, alone or combined with at least one other pharmaceutically active ingredient.

15. The pharmaceutical composition according to Claim 14, **characterized in that** the selenium compound in particular of general formula (I) constitutes a pharmaceutically active ingredient for carrying out the prevention and treatment of tumors or cancers, either alone or in combination with one or more other known anticancer or cytotoxic agents, either by pre-administration or by co-administration, such as notably tumors or cancers of the prostate, of the liver, of the kidneys, of the pancreas, of the lung, of the colon and of the skin.

16. The pharmaceutical composition according to Claim 14 or 15, **characterized in that** at least one of the other anticancer agents is selected from the following compounds: aminoglutethimide, estramustine, medroxyprogesterone acetate, leuprolide, flutamide, toremifene, Zoladex, the matrix metalloproteinase inhibitors; the VEGF inhibitors, such as the anti-VEGF antibodies (Avastin (R)) and the small molecules such as ZD6474 and SU6668; vatalanib, BAY-43-9006, SU11248, CP-547632 and CEP-7055; the SA-1 and SA-2 inhibitors including the anti-HER2 antibodies (Herceptin), the EGFR inhibitors, including gefitinib, erlotinib, ABX-EGF, EMD72000, 11F8, and cetuximab; the Eg5 inhibitors, such as SB-715992, SB-743921, and MKI-833; the PAN inhibitors, such as canertinib, EKB-569, CI-1033, AEE-788, XL-647; the kinase inhibitors, such as 2C4, and GW-572016, Gleevec (R) and dasatinib (Sprycel (R)); Casodex (R) (bicalutamide, Astra Zeneca), tamoxifen; the MAPK kinase inhibitors, the PI3 kinase inhibitors, the PDGF inhibitors, such as imatinib; the anti-angiogenic agents and the antivascular agents; the receptor and non-receptor tyrosine kinase inhibitors, the inhibitors of integrin signaling; tubulin; the agents such as vinblastine, vincristine, vinorelbine, vinflunine, paclitaxel, docetaxel, 7-O-methylthiomethylpaclitaxel, 4-deacetyl-4-methylcarbonatepaclitaxel, C-4 methyl carbonate paclitaxel, epothilone A, epothilone B, epothilone C, epothilone D, deoxyepothilone A, deoxyepothilone B, oxabicyclo[14.1.0]heptadecane-5-9-dione (ixabepilone), leucovorin, tegafur and derivatives thereof; the CDK inhibitors, the antiproliferative cell cycle inhibitors, epidophyllotoxin, etoposide, VM-26; the anti-neoplastic enzymes, the topoisomerase I or II inhibitors such as camptothecin, topotecan, SN-38, procarbazine, mitoxantrone; the platinum coordination complexes such as cisplatin, carboplatin and oxaliplatin; the purine antagonists such as 6-thioguanine and 6-mercaptopurine; the glutamine antagonists.

17. The pharmaceutical composition according to Claim 14, 15 or 16, **characterized in that** at least one of the other cytotoxic agents is selected from at least one compound that follows: cyclophosphamide, doxorubicin, daunorubicin, mitoxantrone, melphalan, hexamethyl melamine, thiotepa, cytarabine, idatrexate, trimetrexate, dacarbazine, L-asparaginase, bicalutamide, leuprolide, the pyridobenzoindole derivatives, the interferons, the interleukins.

18. The pharmaceutical composition according to any one of Claims 14, 15, 16 or 17, **characterized in that** it comprises at least one selenium compound in particular of formula (I) at a concentration between 0.02% and 0.15% by weight in selenium equivalent.

19. The pharmaceutical composition according to any one of Claims 14 to 18, **characterized in that** it comprises at least one pharmaceutically acceptable carrier and a therapeutically effective quantity of one or more of the selenium compounds or of a stereoisomer, of a tautomer, or of a pharmaceutically acceptable salt. These media can in particular consist of:
* an injectable or drinkable solution,
* a solid medium composed of one or more excipients which can be selected from vitamins, natural antioxidants such as L-ergothioneine, mineral salts, mono-, di- or polysaccharides, in particular folic acid, vitamins B₆, E or C, lactose, starch.

20. The pharmaceutical composition according to any one of Claims 14 to 19, **characterized in that** it is formulated for a route of administration selected from the oral route, intravenous route, parenteral route, topical route including the transdermal route or the nasal route or the ocular route, or by inhalation, and in particular is present in the form of a capsule, a gel, a tablet or a powder.

21. A pharmaceutical composition according to anyone of claims 14 to 20, **characterized in that** the compound of formula (I) is selected from:
a) compound 4 of formula : 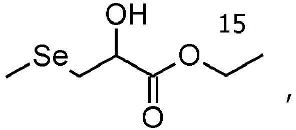 or
b) compound 10 of formula : 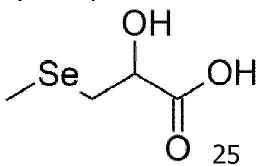 or
c) compound 38 of formula :
